(19) Europäisches Patentamt
European Patent Office
Office européen des brevets

(11) EP 4 775 580 A1

# (12) EUROPEAN PATENT APPLICATION

published in accordance with Art. 153(4) EPC

(43) Date of publication:
**15.07.2026 Bulletin 2026/29**

(21) Application number: **24861691.4**

(22) Date of filing: **17.07.2024**

(51) International Patent Classification (IPC):
***C07D 471/04** (2006.01)* ***C07D 475/00** (2006.01)*
***A61K 31/551** (2006.01)* ***A61K 31/519** (2006.01)*
***A61K 31/5377** (2006.01)* ***A61K 45/06** (2006.01)*
***A61P 35/00** (2006.01)* ***A61P 3/10** (2006.01)*
***A61P 35/02** (2006.01)* ***A61P 37/06** (2006.01)*
***A61P 1/16** (2006.01)*

(52) Cooperative Patent Classification (CPC):
**A61K 31/519; A61K 31/53; A61K 31/5377; A61K 31/551; A61K 45/06; A61P 1/16; A61P 3/10; A61P 29/00; A61P 35/00; A61P 35/02; A61P 37/00; A61P 37/06; C07D 471/04; C07D 475/00; C07D 487/04;** (Cont.)

(86) International application number:
**PCT/CN2024/106034**

(87) International publication number:
**WO 2025/050846 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.09.2023 CN 202311151302**

(71) Applicant: **Euregen Biopharma Co., Ltd.**
**Zhongshan, Guangdong 528449 (CN)**

(72) Inventors:
- **LI, Jing**
  **Guangdong 528449 (CN)**
- **CHEN, Yanhong**
  **Guangdong 528449 (CN)**
- **DENG, Wenjia**
  **Guangdong 528449 (CN)**
- **CHEN, Xuxing**
  **Guangdong 528449 (CN)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

## (54) COMPOUND, PHARMACEUTICAL COMPOSITION COMPRISING SAME, AND USE THEREOF

(57) Provided are a compound, a pharmaceutical composition comprising the same, and a use thereof. The compound has a structure as shown in Formula I, wherein substituents are as described in the specification. The compound can interfere with the interaction between Menin protein and MLL1, MLL2, or MLL-fusion oncoproteins, and is expected to become a drug for treating tumors, diabetes, and other diseases dependent on the activity of MLL1, MLL2, MLL fusion proteins, and/or Menin proteins.

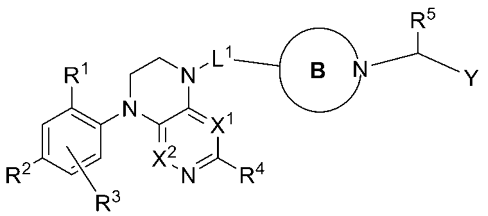

I

EP 4 775 580 A1

(52) Cooperative Patent Classification (CPC): (Cont.)
**C07D 519/00**

## Description

### TECHNICAL FIELD

The present invention pertains to the field of medicinal chemistry, and more particularly to a class of compounds, pharmaceutical compositions comprising said compounds, and therapeutic applications thereof.

### BACKGROUND ART

Mixed-lineage leukemia (MLL) protein is a histone methyltransferase that plays a critical role in transcriptional regulation. In the majority of acute leukemias, including acute myeloid leukemia (AML), acute lymphoblastic leukemia (ALL), and mixed-lineage leukemia, the MLL gene located at chromosome 11q23 frequently undergoes chromosomal translocation, resulting in fusion with one of approximately 80 partner proteins (e.g., AF4, AF9, ENL, AF10, ELL, AF6, AF1p, GAS7, among others) to generate MLL-rearranged (MLL-r) fusion proteins.

MLL-r fusion proteins retain approximately 1,400 amino acids from the N-terminus of wild-type MLL protein while lacking the C-terminal SET domain responsible for methyltransferase activity. These fusion proteins aberrantly regulate the transcription of multiple oncogenes, including HOX and MEIS1, thereby promoting cellular proliferation and ultimately driving leukemogenesis. Patients harboring MLL chromosomal translocations typically exhibit poor prognosis, with 5-year survival rate below 40%.

Menin protein, encoded by the Multiple Endocrine Neoplasia type 1 (MEN1) gene, is a ubiquitously expressed nuclear protein that interacts with DNA replication and repair machinery, chromatin-modifying complexes, and various transcription factors. Menin binds to the N-terminus of MLL family proteins, including MLL1, MLL2, and MLL-r fusion proteins. This protein-protein interaction is essential for the oncogenic activity of MLL proteins. Disruption of the Menin-MLL interaction selectively inhibits proliferation of MLL-r leukemia cells both in vitro and in vivo.

Certain hematological malignancies harbor specific genetic abnormalities or mutations, such as nucleoporin 98 (NUP98) gene fusions, nucleophosmin (NPM1) mutations, DNA methyltransferase 3A (DNMT3A) mutations, and MLL gene amplification. These genetic alterations are frequently associated with elevated HOX gene expression. In Ewing sarcoma, posterior HOXD genes, particularly HOXD13, are aberrantly overexpressed in conjunction with elevated Menin and MLL1 protein levels, wherein HOXD13 represents a downstream target gene regulated by the Menin-MLL1 complex.

Furthermore, the human ether-à-go-go-related gene (hERG) potassium channel is essential for normal cardiac electrical activity. Inhibition of hERG leads to QT interval prolongation, a potentially fatal cardiac arrhythmia. This cardiotoxicity represents a common cause of drug attrition in preclinical safety assessment, rendering compounds with minimal hERG inhibitory activity highly desirable in drug design. Accordingly, evaluation of hERG channel inhibition potential is critical in drug development; however, this property cannot be readily predicted from molecular structure alone, as structurally similar compounds may exhibit substantially different hERG blocking potencies. There remains an urgent unmet need for the development of efficacious compounds with minimal hERG channel blockade.

WO2023078426 disclosed a class of pyrimido-pyridazine-based inhibitors that disrupt the Menin-MLL protein-protein interaction, exemplified by Example 3 compound. However, this compound demonstrated unfavorable in vivo metabolic properties, suboptimal in vitro and in vivo efficacy, and pronounced hERG ion channel inhibition.

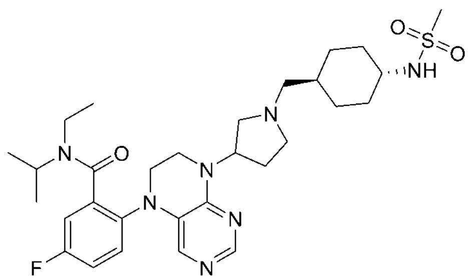

Example 3

The clinical-stage compound SNDX-5613 (revumenib) induces point mutation-mediated resistance in approximately 40% of patients following two treatment cycles, with most Menin inhibitors exhibiting markedly diminished activity against resistant mutant variants (Perner et al., Nature, March 2023).

Accordingly, there exists a pressing need for the development of small molecule compounds capable of disrupting the Menin-MLL protein-protein interaction with enhanced in vitro and in vivo potency, retained efficacy against resistant mutant strains, and minimal hERG ion channel inhibition, for therapeutic application in tumors, diabetes, and

other disorders associated with MLL1, MLL2, MLL fusion protein, and/or Menin activity.

## SUMMARY OF THE INVENTION

It is an object of the present invention to provide compounds capable of disrupting the Menin-MLL protein-protein interaction.

In a first aspect, the present invention provides a compound, or a pharmaceutically acceptable salt, enantiomer, diastereomer, tautomer, geometric isomer, solvate, polymorph, deuterated derivative, or combination thereof, wherein said compound has the structure represented by Formula I:

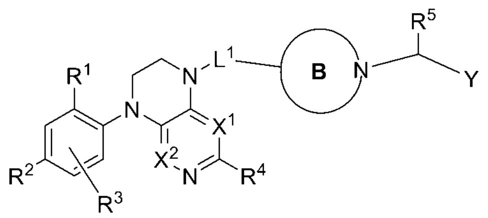

I ;

wherein:

$R^1$ is selected from the group consisting of: -C(O)($NR^aR^b$), phenyl, and 5- to 6-membered heteroaryl; wherein said phenyl or heteroaryl is optionally substituted with $R^{1a}$;

$R^a$ and $R^b$ are each independently selected from the group consisting of: H, optionally substituted C1-C6 alkyl, optionally substituted 3- to 8-membered cycloalkyl, or optionally substituted 4- to 8-membered heterocyclyl; alternatively, $R^a$ and $R^b$ together with the nitrogen atom to which they are attached form an optionally substituted 4- to 8-membered heterocyclic ring; wherein said heterocyclic ring contains, in addition to the attached nitrogen atom, 0 to 2 heteroatoms selected from N, O, S, and P; wherein "substituted" refers to replacement of one or more hydrogen atoms by R;

Each $R^{1a}$ is independently selected from the group consisting of: H, cyano, halogen, C1-C3 alkyl, halo-C1-C3 alkyl, C2-C4 alkenyl, C2-C4 alkynyl, C1-C3 alkoxy, halo-C1-C3 alkoxy, and C3-C5 cycloalkyl;

$R^2$ is selected from the group consisting of: H, halogen, methyl, and trifluoromethyl;

$R^3$ represents 0, 1, 2, or 3 substituents each independently selected from the group consisting of: H, halogen, C1-C3 alkyl, and halo-C1-C3 alkyl;

$R^4$ is selected from the group consisting of: H, optionally substituted C1-C6 alkyl, optionally substituted C1-C4 alkoxy, optionally substituted C1-C4 alkylamino, optionally substituted di(C1-C4 alkyl)amino, halogen, -NH2, -NO2, -COOH, -CN, -OH, optionally substituted C1-C6 alkylsulfonyl, optionally substituted C1-C6 alkylsulfinyl, optionally substituted C1-C6 alkylthio, $-NHCOCR^{4a}=CH_2$, $-NHCOCHR^{4a}R^{4b}$, $-SO_2C(R^{4a})=CH_2$, $-NHSO_2CR^{4a}-CH_2$, or $-NHSO_2CHR^{4a}R^{46}$; wherein each $R^{4a}$ is independently selected from: H, methyl, and fluorine; each $R^{4b}$ is independently chlorine or bromine; wherein "substituted" refers to replacement of one or more (e.g., 1, 2, or 3) hydrogen atoms by R;

$X^1$ and $X^2$ are each independently N or $CR^X$; wherein $R^X$ is selected from the group consisting of: H, halogen, -CN, -OH, $-NH_2$, optionally substituted C1-C4 alkyl, optionally substituted C1-C4 alkoxy, optionally substituted C1-C4 alkylamino, and optionally substituted $(C1\text{-}C4\ alkyl)_2$ amino; wherein "substituted" refers to replacement of one or more hydrogen atoms by R;

Ring B is a 4- to 12-membered saturated or partially saturated nitrogen-containing heterocyclic ring; wherein said nitrogen-containing heterocyclic ring is optionally substituted with $R^B$, and comprises at least one nitrogen atom and 0 to 2 additional heteroatoms selected from N, O, and S;

Each $R^B$ is independently selected from the group consisting of: deuterium, halogen, oxo, C1-C3 alkyl, halo-C1-C3

alkyl, or cyano;

$L^1$ is selected from: absent (single bond) , -($CR^{La}R^{Lb}$)-, or -($CR^{La}R^{Lb}$)$_2$-; wherein $R^{La}$ and $R^{Lb}$ are each independently selected from: H, deuterium, optionally substituted C1-C4 alkyl, and halogen; alternatively, $R^{La}$ nd $R^{Lb}$ together with the carbon atom to which they are attached form an optionally substituted 3- to 8-membered saturated or unsaturated carbocyclic ring, or an optionally substituted 4- to 8-membered saturated or unsaturated heterocyclic ring; wherein said heterocyclic ring contains 1 to 3 heteroatoms selected from N, O, S, and P;

$R^5$ is selected from the group consisting of: optionally substituted C1-C4 alkyl, optionally substituted 3- to 8-membered saturated or unsaturated carbocyclic ring, optionally substituted 4-to 8-membered saturated or unsaturated heterocyclic ring; wherein said heterocyclic ring contains 1 to 3 heteroatoms selected from N, O, S, and P; wherein "substituted" refers to substitution by one or more R;

Y is selected from: -C1-C6 alkylene-$NR^{Y1}R^{Y2}$, 3- to 6-membered cycloalkylene-$NR^{Y1}R^{Y2}$, optionally substituted 4- to 8-membered heterocyclyl; wherein "substituted" refers to substitution by one or more R;

$R^{Y1}$ is selected from the group consisting of: H and C1-C4 alkyl; wherein said alkyl is optionally further substituted with one or more substituents selected from: halogen, hydroxyl, C1-C4 alkoxy, and cyano;

$R^{Y2}$ is -$L^2$-$R^{Y3}$ ; wherein $L^2$ is selected from: absent and C1-C6 alkylene; $R^{Y3}$ is selected from the group consisting of: hydrogen, C1-C6 alkyl, C1-C6 alkoxy, optionally substituted saturated or unsaturated 3- to 8-membered carbocyclyl, or optionally substituted saturated or unsaturated 4- to 12-membered heterocyclyl; wherein "substituted" refers to substitution by one or more R;

alternatively, $R^{Y1}$ and $R^{Y2}$ together with the nitrogen atom to which they are attached form an optionally substituted 4- to 10-membered nitrogen-containing heterocyclic ring; wherein said nitrogen-containing heterocyclic ring comprises at least one nitrogen atom and 0 to 2 additional heteroatoms selected from N, O, and S; wherein "substituted" refers to replacement of one or more hydrogen atoms by R;

Each R is independently selected from the group consisting of: deuterium, halogen, -OH, oxo, mercapto, cyano, -CD3, -C1-C6 alkyl, methylene, C2-C6 alkenyl, C2-C6 alkynyl, C3-C8 cycloalkyl, 6- to 10-membered aryl, 4- to 12-membered heterocyclyl, 5- to 10-membered heteroaryl, 6- to 10-membered aryl-C1-C6 alkylene-, 5- to 10-membered heteroaryl-C1-C6 alkylene-, C3-C8 cycloalkyl-C1-C6 alkylene-, 4- to 12-membered heterocyclyl-C1-C6 alkylene-, C1-C6 haloalkyl-, -OC1-C6 alkyl, -OC2-C6 alkenyl, C3-C8 cycloalkyl-O-, 4- to 12-membered heterocyclyl-O-, 6- to 10-membered aryl-O-, 5- to 10-membered heteroaryl-O-, -OC1-C6 alkylphenyl, -C1-C6 alkyl-OH, -C1-C6 alkyl-SH, -C1-C6 alkyl-O-C1-C6 alkyl, -OC1-C6 haloalkyl, -$NH_2$, -C1-C6 alkyl-$NH_2$, -N(C1-C6 alkyl)$_2$, -NH(C1-C6 alkyl), -N(C1-C6 alkyl)(C1-C6 alkylphenyl), -NH(C1-C6 alkylphenyl), -N(C1-C6 alkyl)(6- to 10-membered aryl), -NH(6- to 10-membered aryl), nitro, -C(O)-OH, -C(O)OC1-C6 alkyl, -$CONR^iR^{ii}$, -NHC(O)(C1-C6 alkyl), -NHC(O)(phenyl), -N(C1-C6 alkyl)C(O)(C1-C6 alkyl), -N(C1-C6 alkyl)C(O)(phenyl), - C(O)C1-C6 alkyl, 5- to 10-membered heteroaryl C(O)-, -C(O) C1-C6 alkylphenyl, -C(O)C1-C6 haloalkyl, -OC(O)C1-C6 alkyl, -S(O)$_2$-C1-C6 alkyl, -S(O)-C1-C6 alkyl, -S(O)$_2$-phenyl, -S(O)$_2$-C1-C6 haloalkyl, -S(O)$_2$$NH_2$, -S(O)$_2$NH(C1-C6 alkyl), -S(O)$_2$NH(phenyl), -NHS(O)$_2$(C1-C6 alkyl), -NHS(O)$_2$(phenyl), and -NHS(O)$_2$(C1-C6 haloalkyl); wherein each hydrogen in said alkyl, alkenyl, alkynyl, cycloalkyl, phenyl, aryl, heterocyclyl, and heteroaryl is optionally further substituted with one or more substituents selected from: halogen, -OH, oxo (=O), -$NH_2$, C3-C8 cycloalkyl, 3- to 8-membered heterocyclyl, C1-C4 alkyl, C1-C4 haloalkyl-, -OC1-C4 alkyl, -C1-C4 alkyl-OH, -C1-C4 alkyl-O-C1-C4 alkyl, -OC1-C4 haloalkyl, cyano, nitro, -C(O)-OH, - C(O) OC1-C6 alkyl, -CON(C1-C6 alkyl)$_2$, -CONH(C1-C6 alkyl), -CONH2, -NHC(O)(C1-C6 alkyl), -NH(C1-C6 alkyl)C(O) (C1-C6 alkyl), -$SO_2$(C1-C6 alkyl), -$SO_2$(phenyl), -$SO_2$(C1-C6 haloalkyl), -$SO_2NH_2$, -$SO_2$NH(C1-C6 alkyl), -$SO_2$NH(phenyl), -$NHSO_2$(C1-C6 alkyl), - $NHSO_2$(phenyl), and -$NHSO_2$(C1-C6 haloalkyl); $R^i$ and $R^{ii}$ are each independently H, deuterium, or C1-C6 alkyl.

In certain embodiments,

$R^1$ is selected from the group consisting of: -C(O)($NR^aR^b$) and 5- to 6-membered heteroaryl; wherein said heteroaryl is optionally substituted with $R^{1a}$;

$R^a$ and $R^b$ are each independently selected from: H, optionally substituted C1-C6 alkyl, optionally substituted 3- to 8-membered cycloalkyl;

Each $R^{1a}$ is independently selected from: H, cyano, halogen, C1-C3 alkyl, halo-C1-C3 alkyl, C2-C4 alkenyl, C2-C4 alkynyl, C1-C3 alkoxy, and C3-C5 cycloalkyl.

In certain embodiments,

$R^1$ is selected from the group consisting of: -C(O)($NR^aR^b$) and 5- to 6-membered heteroaryl; wherein said heteroaryl is optionally substituted with $R^{1a}$;

$R^a$ and $R^b$ are each independently selected from: H, C1-C6 alkyl, 3- to 6-membered cycloalkyl;

Each $R^{1a}$ is independently selected from: H, cyano, C1-C3 alkyl, halo-C1-C3 alkyl, C1-C3 alkoxy, and C3-C5 cycloalkyl.

In certain embodiments,

$R^1$ is selected from the group consisting of: -C(O)($NR^aR^b$) and

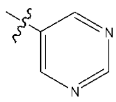;

wherein said

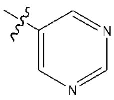

is optionally substituted with $R^{1a}$;
$R^a$ and $R^b$ are each independently selected from: H, ethyl, isopropyl, and cyclopropyl;
each $R^{1a}$ is independently selected from: H, cyano, methyl, and cyclopropyl.

In certain embodiments, $R^2$ is selected from: H, halogen, methyl, and trifluoromethyl; preferably, $R^2$ is H or F.
In certain embodiments, $R^3$ is H.
In certain embodiments, $R^4$ is selected from: H, C1-C6 alkyl, C1-C4 alkoxy, halogen, $-NH_2$, $-NO_2$, -COOH, -CN, and -OH.
In certain embodiments, $R^4$ is H.
In certain embodiments, Ring B is a 4- to 11-membered saturated or partially saturated nitrogen-containing heterocyclic ring comprising at least one nitrogen atom and 0 to 2 additional heteroatoms selected from N, O, and S; wherein said nitrogen-containing heterocyclic ring is optionally substituted with $R^B$; each $R^B$ is independently selected from: deuterium, halogen, oxo, C1-C3 alkyl, halo-C1-C3 alkyl, or cyano.
In certain embodiments, Ring B is selected from the group consisting of:

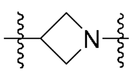 、 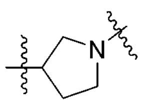 、 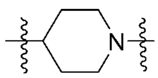 、 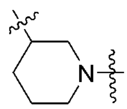 、 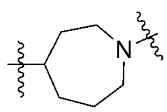 、 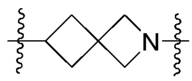 、

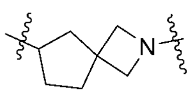 、 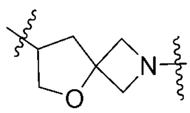 、 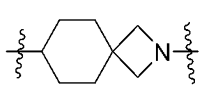 、 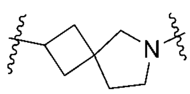 、 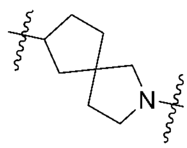 、

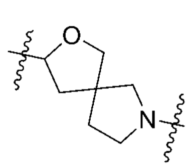 、 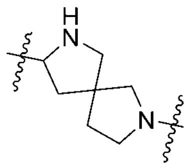 、 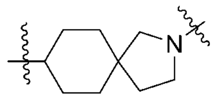 、 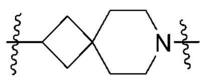 、 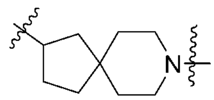 、

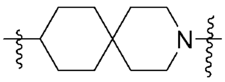 、 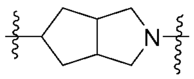 、 and 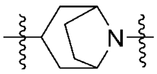 ;

wherein Ring B is optionally substituted with $R^B$; each $R^B$ is independently deuterium, halogen, oxo, C1-C3 alkyl, halo-C1-C3 alkyl, or cyano.

In certain embodiments, Ring B is a 4- to 7-membered saturated nitrogen-containing heterocyclic ring comprising at least one nitrogen atom and 0 to 2 additional heteroatoms selected from N and O; wherein said nitrogen-containing heterocyclic ring is optionally substituted with $R^B$ ; each $R^B$ is independently selected from the group consisting of: deuterium, halogen, oxo, C1-C3 alkyl, halo-CC1-C3 alkyl, or cyano.

In certain embodiments, Ring B is selected from the group consisting of

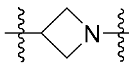 、 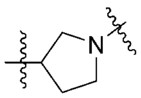 、 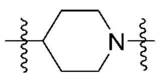 、 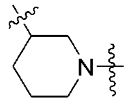 and 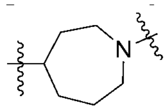 ,

wherein , Ring B is optionally substituted with $R^B$; each $R^B$ is independently deuterium, halogen, oxo, C1-C3 alkyl, halo-C1-C3 alkyl, or cyano.

In certain embodiments, Ring B is a 5- to 6-membered saturated nitrogen-containing heterocyclic ring; wherein said nitrogen-containing heterocyclic ring is optionally substituted with $R^B$; each $R^B$ is independently selected from: H, deuterium, halogen, and C1-C3 alkyl.

In certain embodiments, Ring B is a 5-membered saturated nitrogen-containing heterocyclic ring; wherein said nitrogen-containing heterocyclic ring is optionally substituted with $R^B$; each $R^B$ is independently selected from: H, deuterium, halogen, and C1-C3 alkyl.

In certain embodiments, Ring B is unsubstituted.

In certain embodiments, Ring B is selected from

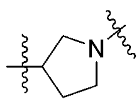 .

In certain embodiments, $L^1$ is selected from: absent (single bond) or -($CR^{La}R^{Lb}$)-; wherein $R^{La}$ and $R^{Lb}$ are each independently selected from: H, deuterium, optionally substituted C1-C4 alkyl, and halogen.

In certain embodiments, $L^1$ is absent.

In certain embodiments, $R^5$ is selected from: C1-C4 alkyl.

In certain embodiments, $R^5$ is isopropyl.

In certain embodiments, $X^1$ and $X^2$ are each independently N or $CR^X$; wherein $R^X$ is selected from: H, halogen, -CN, -OH, $-NH_2$, optionally substituted C1-C4 alkyl, optionally substituted C1-C4 alkoxy; wherein "substituted" refers to replacement of one or more hydrogen atoms by R.

In certain embodiments, $X^1$ and $X^2$ are each independently N.

In certain embodiments, $X^1$ is N; $X^2$ is N or CH.

In certain embodiments, Y is selected from: -C1-C6 alkylene-$NR^{Y1}R^{Y2}$, 3- to 6-membered cycloalkylene-$NR^{Y1}R^{Y2}$;

$R^{Y1}$ is selected from the group consisting of: H and C1-C4 alkyl; wherein said alkyl is optionally further substituted with one or more substituents selected from: halogen, hydroxyl, C1-C4 alkoxy, and cyano;

$R^{Y2}$ is -$L^2$-$R^{Y3}$; wherein $L^2$ is selected from: absent and C1-C6 alkylene; $R^{Y3}$ is selected from the group consisting of: hydrogen, C1-C6 alkyl, C1-C6 alkoxy, optionally substituted saturated or unsaturated 3- to 6-membered carbocyclyl, or optionally substituted saturated or unsaturated 4- to 6-membered heterocyclyl; wherein "substituted" refers to

substitution by one or more R;

alternatively, $R^{Y1}$ and $R^{Y2}$ together with the nitrogen atom to which they are attached form an optionally substituted 4- to 9-membered nitrogen-containing heterocyclic ring; wherein said nitrogen-containing heterocyclic ring comprises at least one nitrogen atom and 0 to 2 additional heteroatoms selected from N and O; wherein "substituted" refers to replacement of one or more hydrogen atoms in the group by R; R is as defined in the first aspect of the present invention.

In certain embodiments, Y is selected from: -C1-C4 alkylene-$NR^{Y1}R^{Y2}$, 3- to 6-membered cycloalkylene-$NR^{Y1}R^{Y2}$;

$R^{Y1}$ is selected from the group consisting of: H and C1-C4 alkyl; wherein said alkyl is optionally further substituted with one or more substituents selected from: halogen, hydroxyl, C1-C4 alkoxy, and cyano;

$R^{Y2}$ is $-L^2-R^{Y3}$; wherein $L^2$ is selected from: a bond and C1-C4 alkylene; $R^{Y3}$ is selected from the group consisting of: hydrogen, C1-C4 alkyl, C1-C4 alkoxy;

alternatively, $R^{Y1}$ and $R^{Y2}$ together with the nitrogen atom to which they are attached form an optionally substituted 4- to 10-membered nitrogen-containing heterocyclic ring; wherein said nitrogen-containing heterocyclic ring comprises at least one nitrogen atom and 0 to 2 additional heteroatoms selected from N and O; wherein "substituted" refers to replacement of one or more hydrogen atoms in the group by R; R is as defined in the first aspect of the present invention.

In certain embodiments, the nitrogen-containing heterocyclic ring is a monocyclic, fused, bridged, or spiro ring system.

In certain embodiments, said nitrogen-containing heterocyclic ring is unsubstituted.

In certain embodiments, $R^{Y1}$ is methyl.

In certain embodiments, $L^2$ is selected from: absent and C1-C6 alkylene; preferably a bond or ethylene.

In certain embodiments, $R^{Y3}$ is selected from the group consisting of: hydrogen, C1-C6 alkyl, C1-C6 alkoxy; preferably, $R^{Y3}$ is selected from: hydrogen, methyl, and methoxy.

In certain embodiments, $L^1$ is absent $R^5$ is

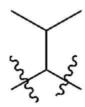;

Y is

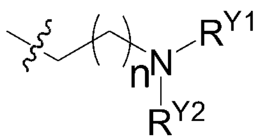 or 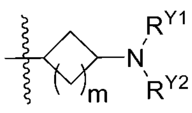;

wherein n is selected from: 0, 1, 2; m is selected from 1, 2;

$R^{Y1}$ and $R^{Y2}$ are as defined in the first aspect of the present invention.

In certain embodiments, when $R^{Y1}$ and $R^{Y2}$ together with the nitrogen atom to which they are attached form an optionally substituted 4- to 10-membered nitrogen-containing heterocyclic ring, the 4- to 10-membered heterocyclic ring is selected from the group consisting of:

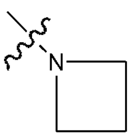、

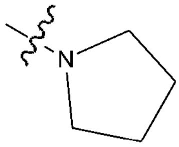、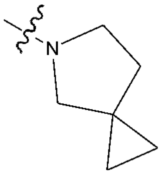、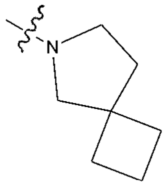、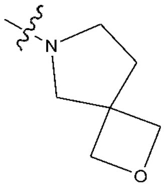、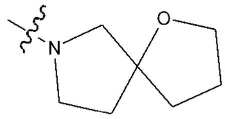、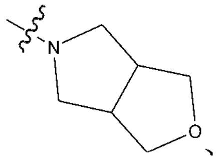、

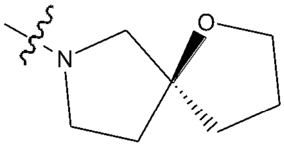、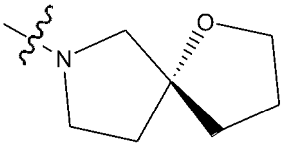、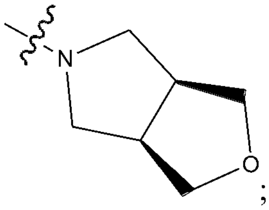;

wherein "substituted" refers to replacement of one or more hydrogen atoms in the group by R; R is as defined in the first aspect of the present invention.

In certain embodiments, said compound is selected from Table A:

Table A

| | | |
|---|---|---|
| 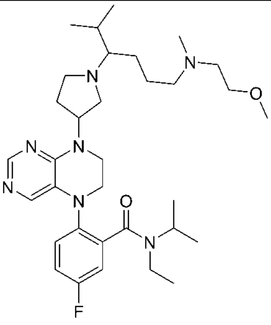 、 | 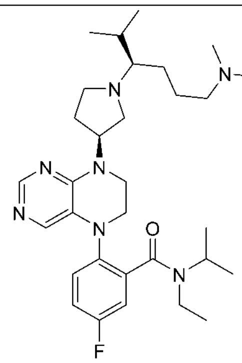 、 | 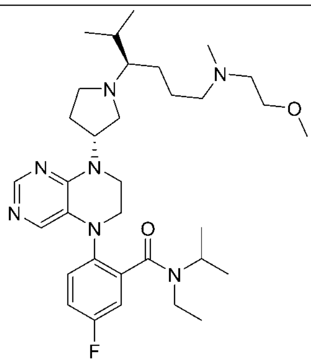 、 |
| 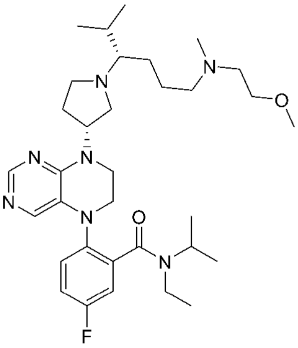 、 | 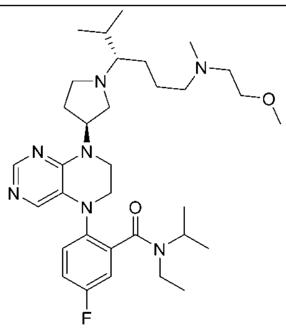 、 | 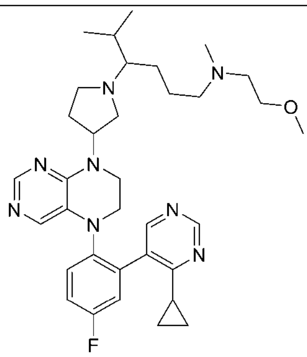 、 |
| 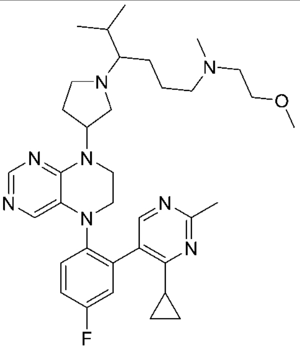 、 | 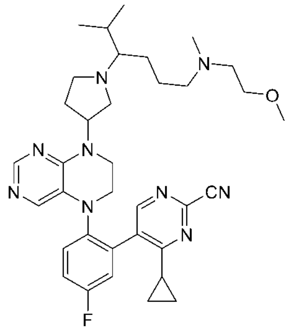 、 | 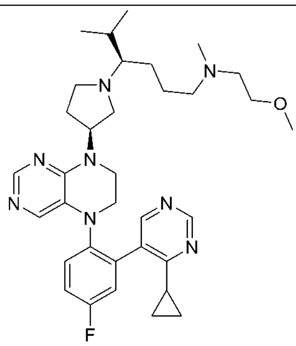 、 |
| 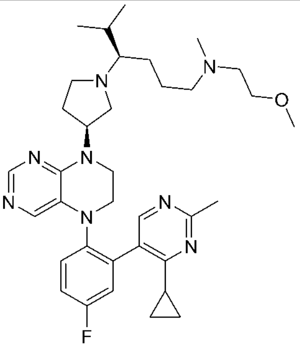 、 | 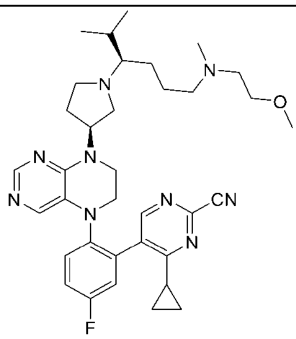 、 | 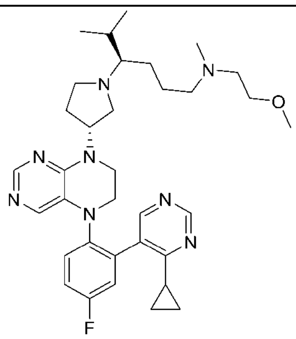 、 |
| 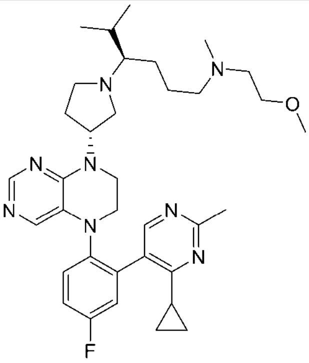 、 | 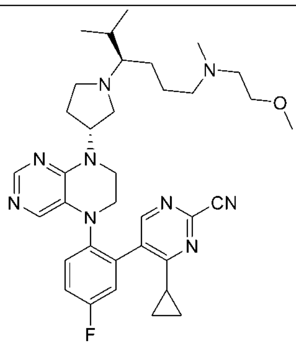 、 | 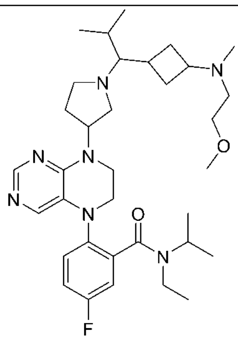 、 |

| | | |
|---|---|---|
| 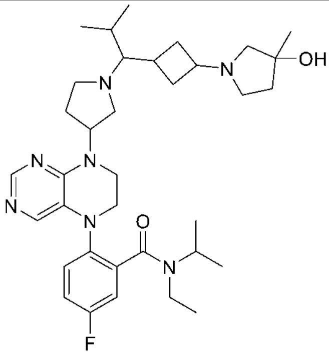 、 | 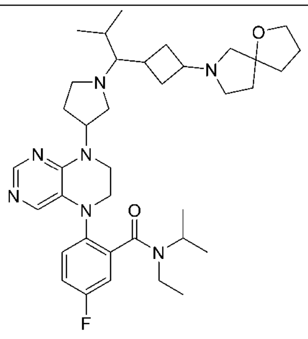 、 | 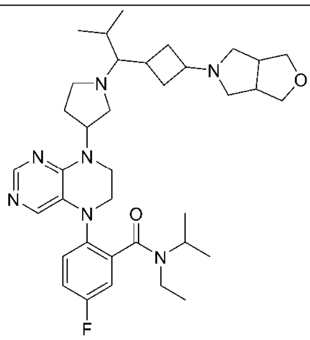 、 |
| 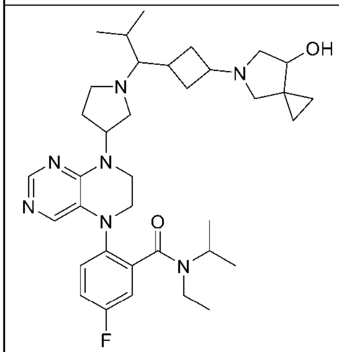 、 | 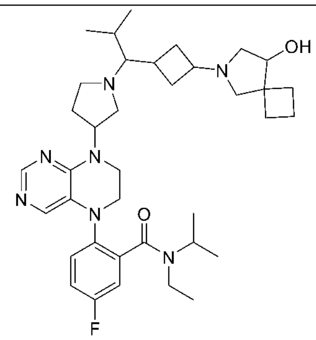 、 | 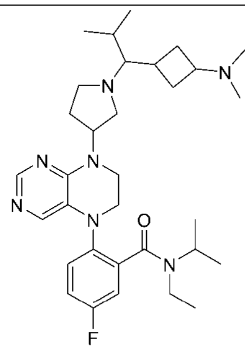 、 |
| 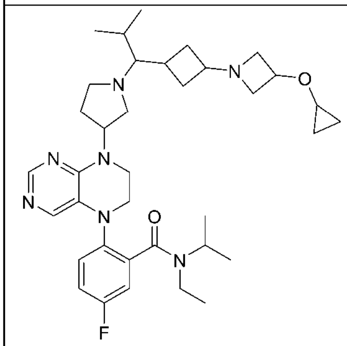 、 | 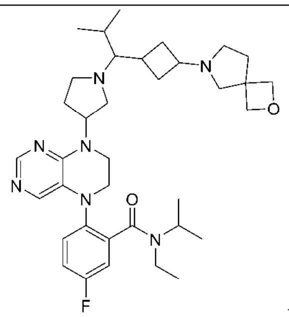 、 | 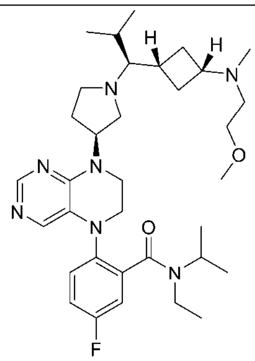 、 |
| 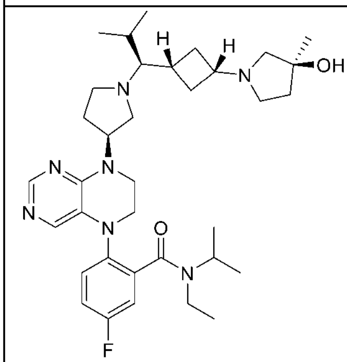 、 | 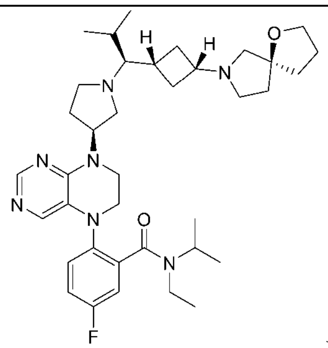 、 | 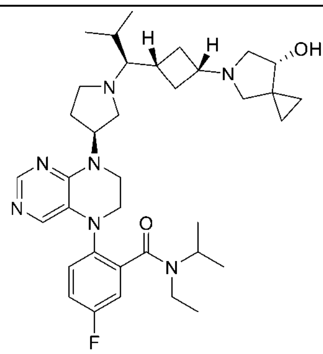 、 |
| 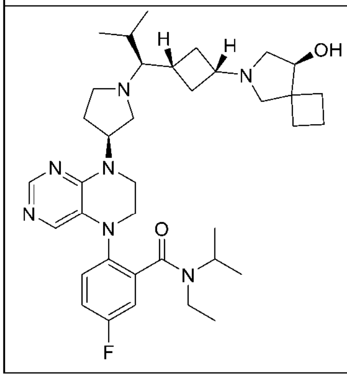 、 | 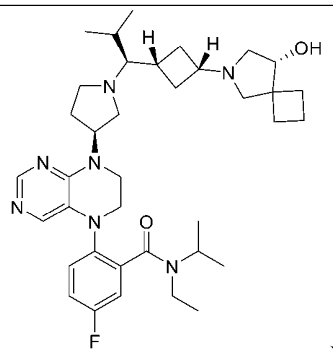 、 | 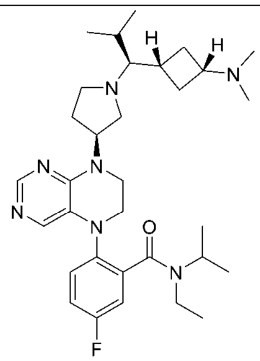 、 |

| | | |
|---|---|---|
| 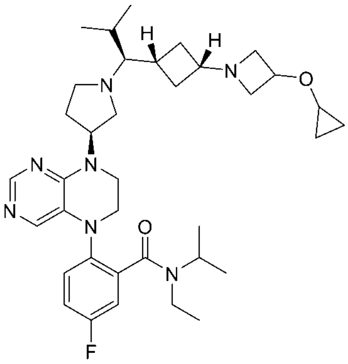 、 | 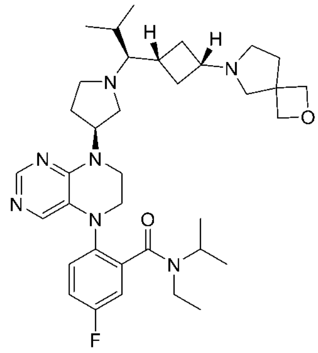 、 | 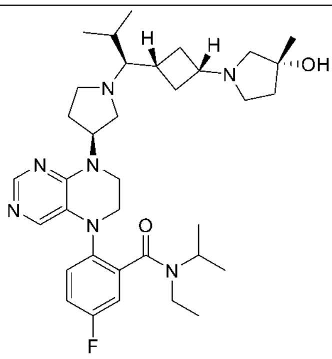 、 |
| 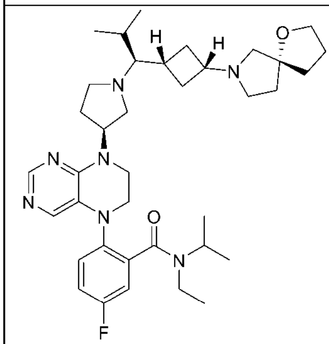 、 | 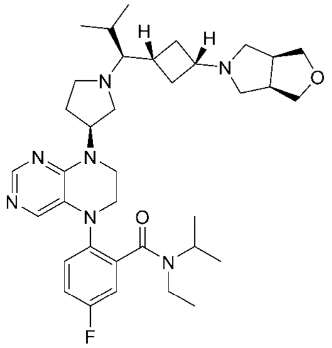 、 | 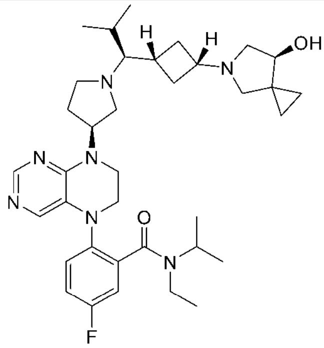 、 |
| 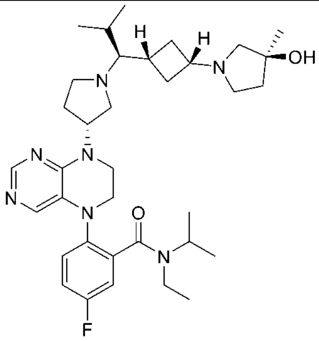 、 | 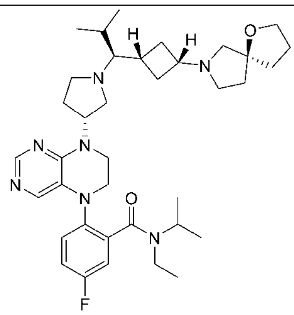 、 | 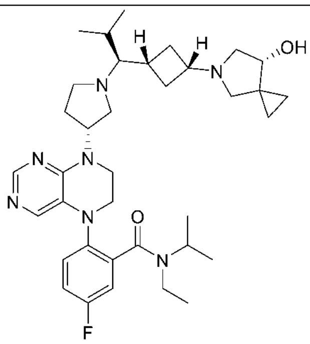 、 |
| 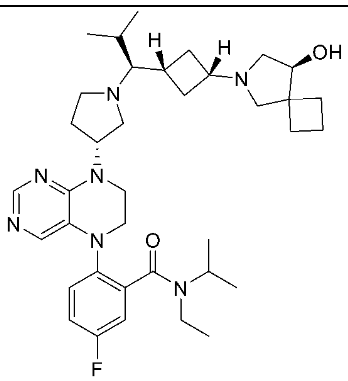 、 | 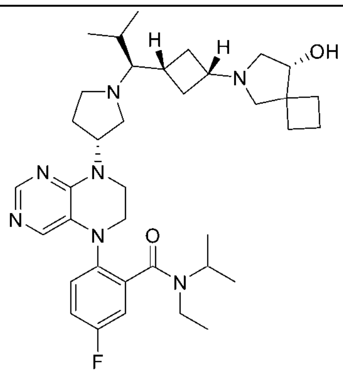 、 | 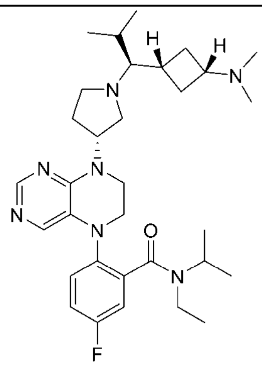 、 |
| 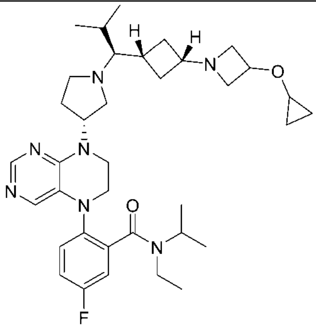 、 | 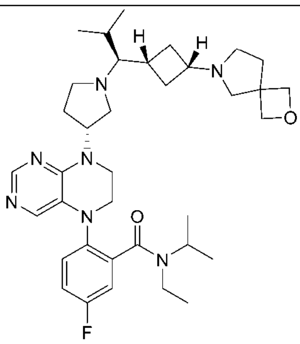 、 | 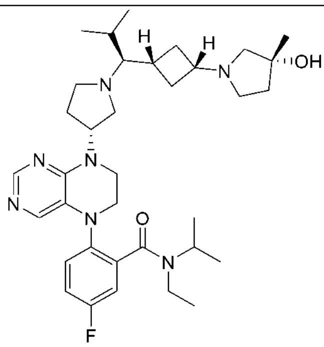 、 |

| | | |
|---|---|---|
| 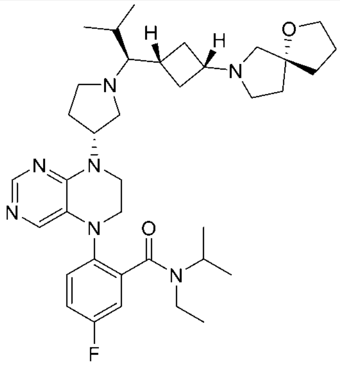 , | 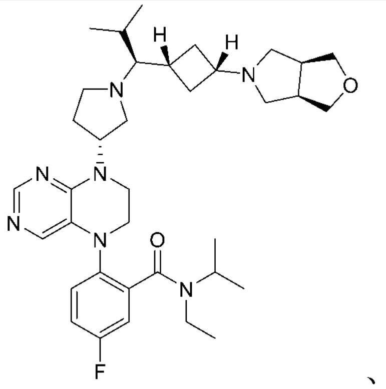 , | 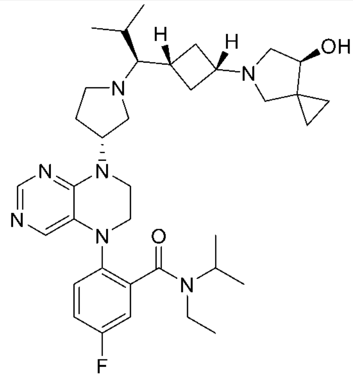 , |
| 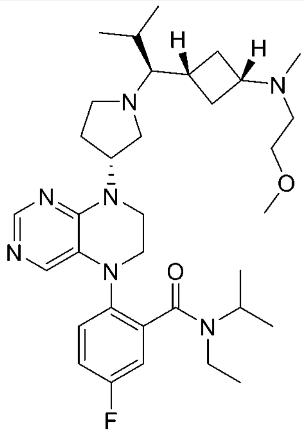 , | 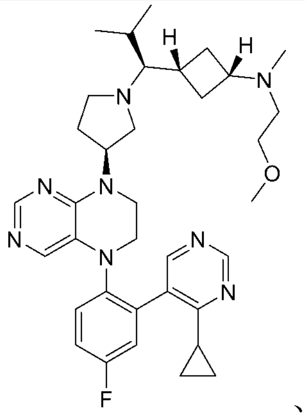 , | 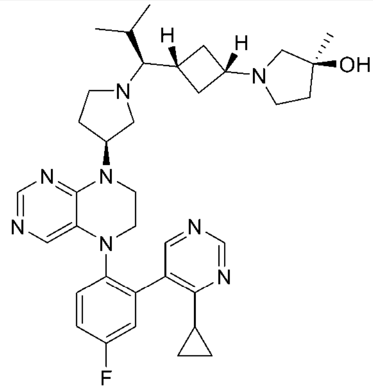 , |
| 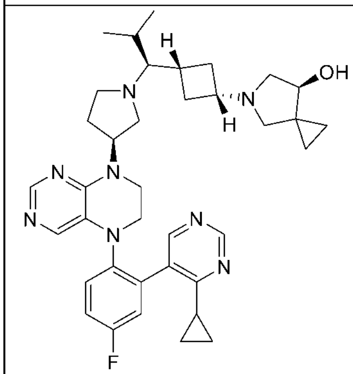 , | 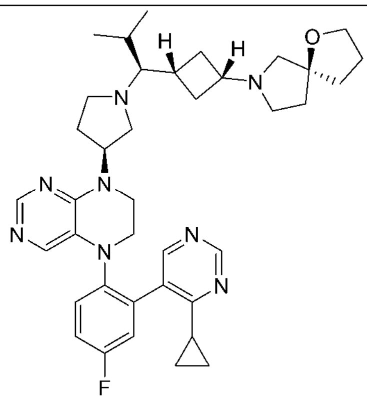 , | 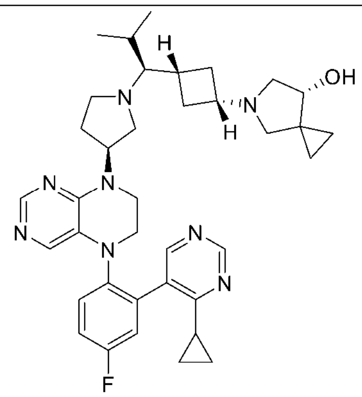 , |
| 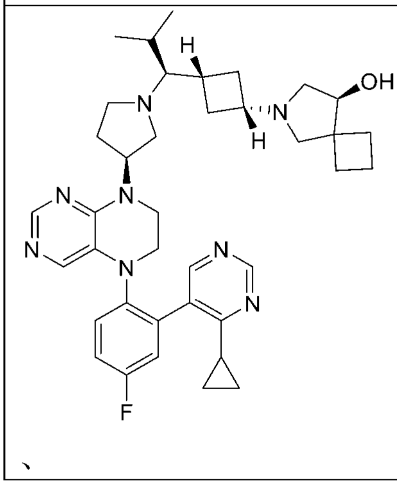 , | 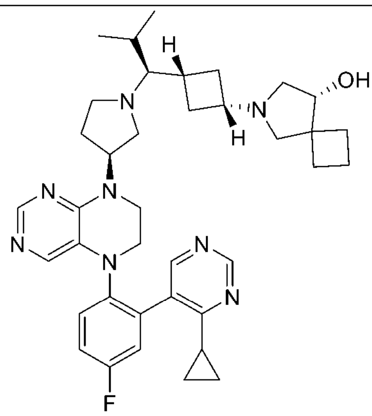 , | 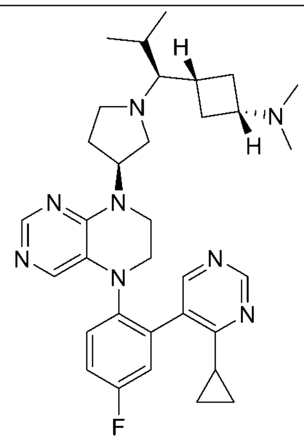 , |

| | | |
|---|---|---|
| 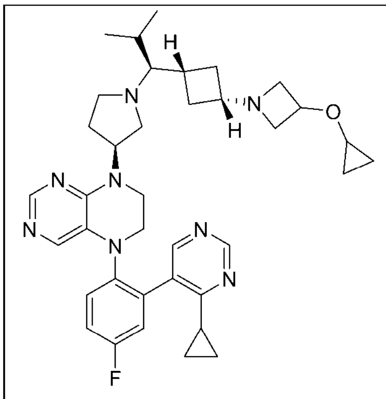 , | 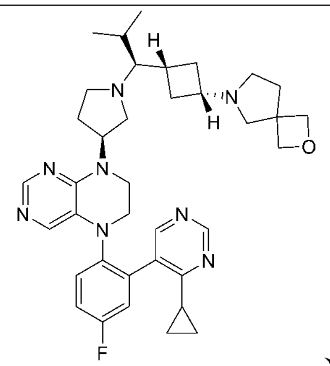 , | 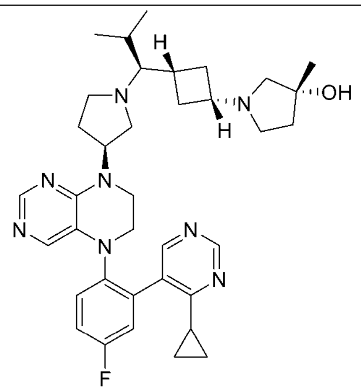 , |
| 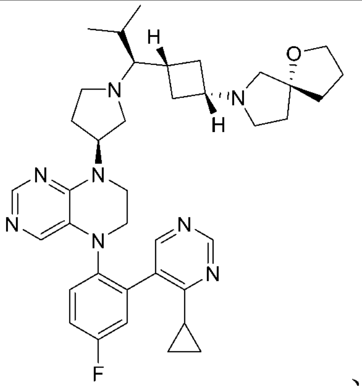 , | 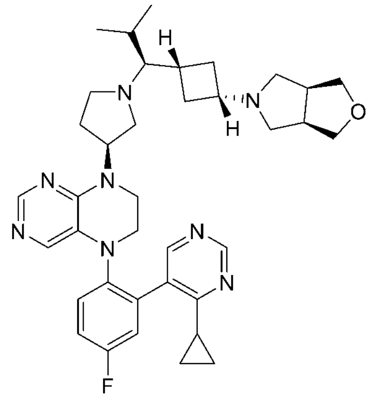 , | 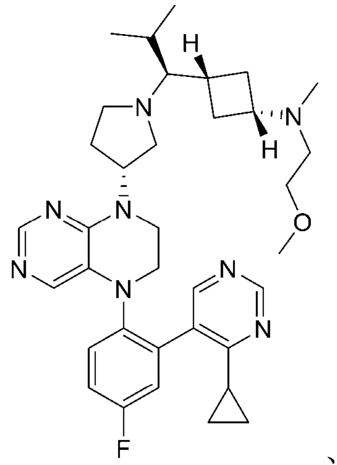 , |
| 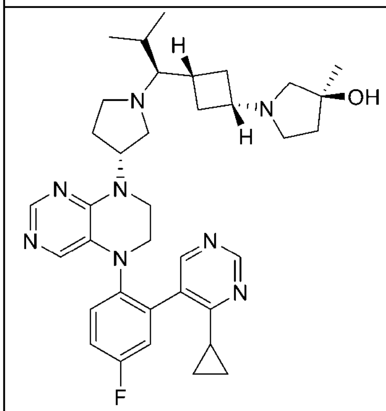 , | 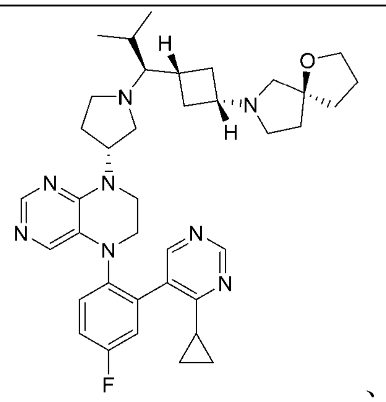 , | 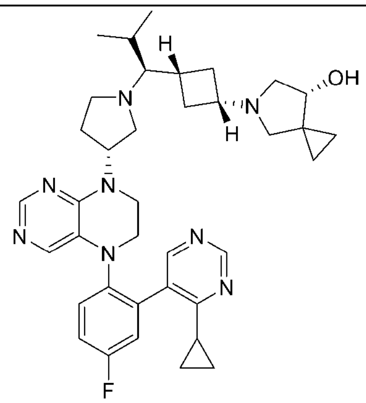 , |
| 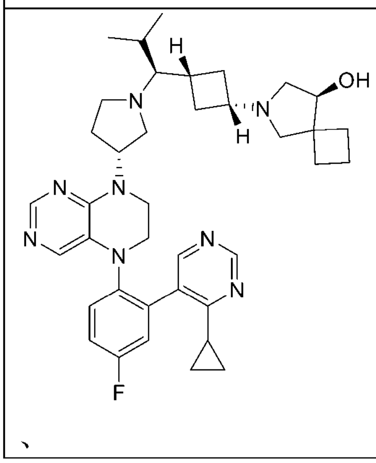 , | 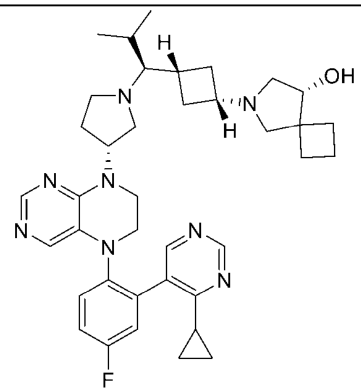 , | 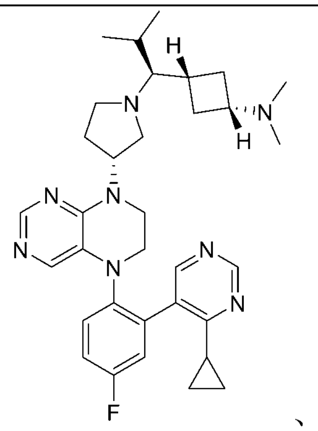 , |

| | | |
|---|---|---|
| 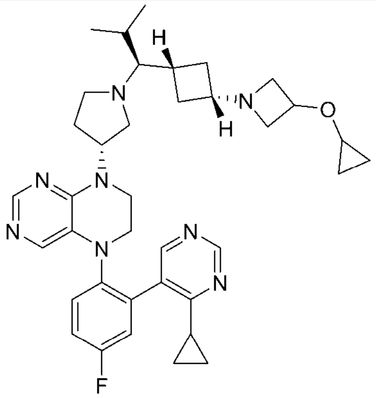 , | 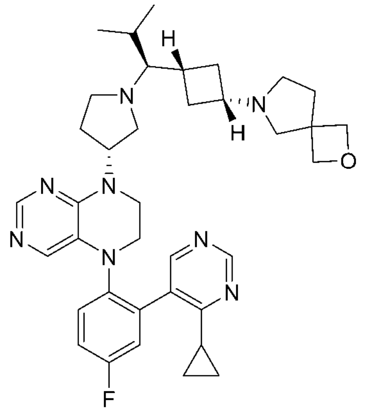 , | 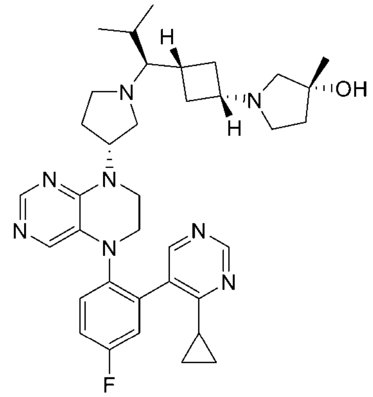 , |
| 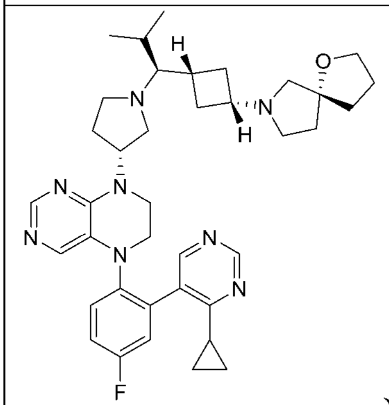 , | 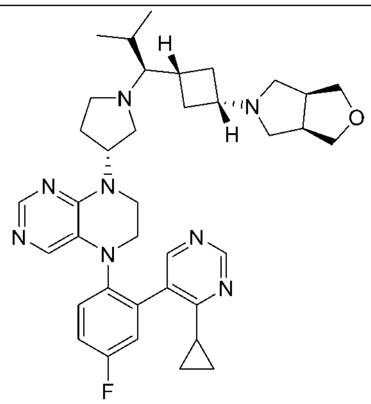 , | 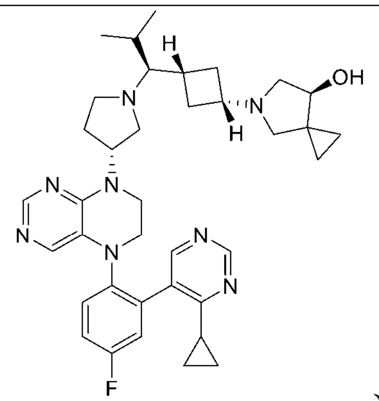 , |
| 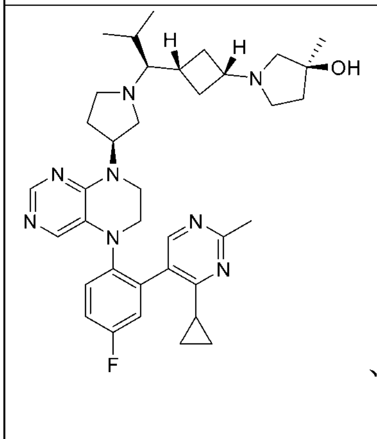 , | 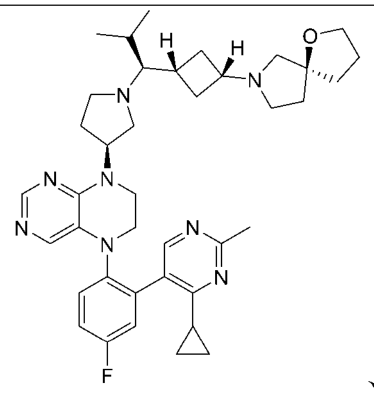 , | 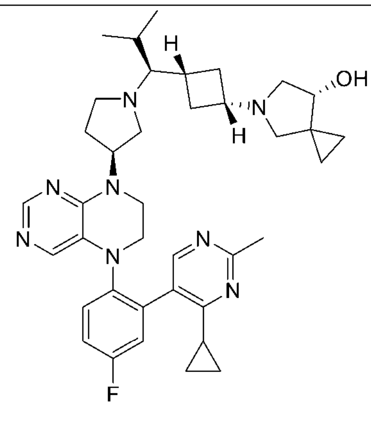 , |
| 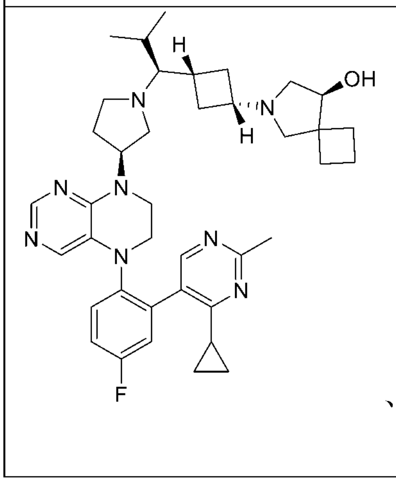 , | 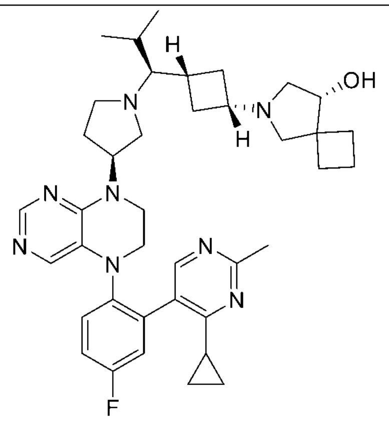 , | 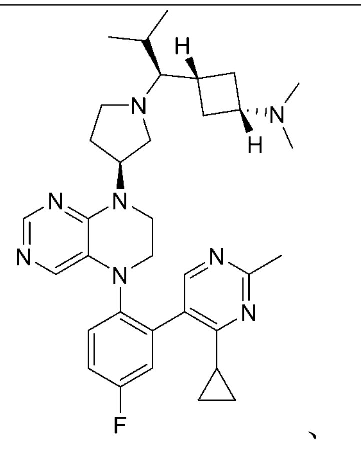 , |

| | | |
|---|---|---|
| 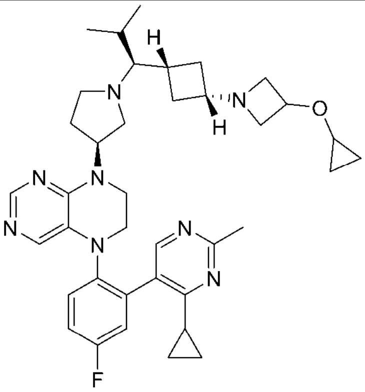 , | 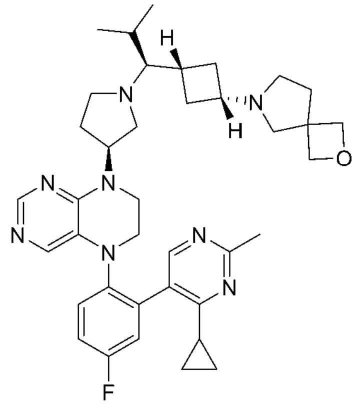 , | 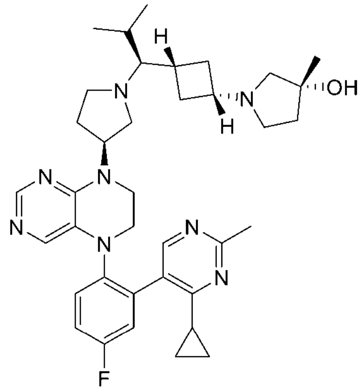 , |
| 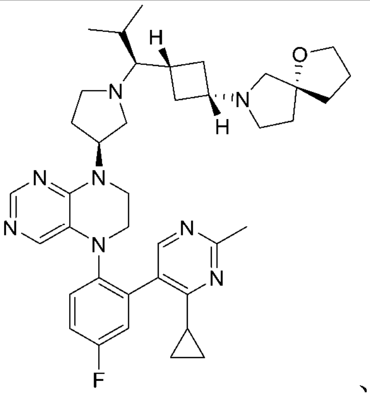 , | 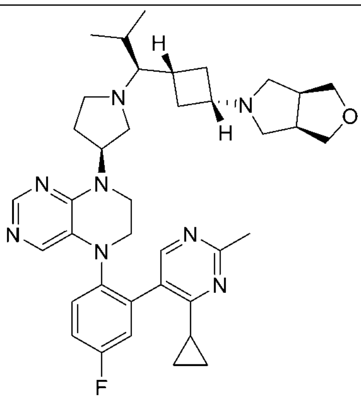 , | 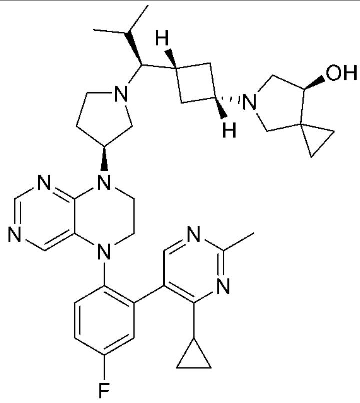 , |
| 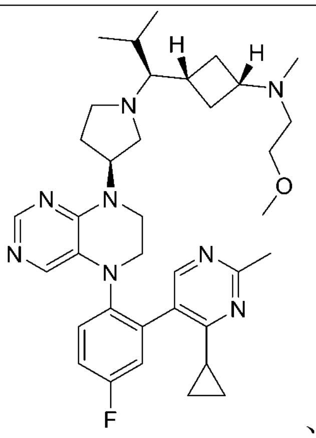 , | 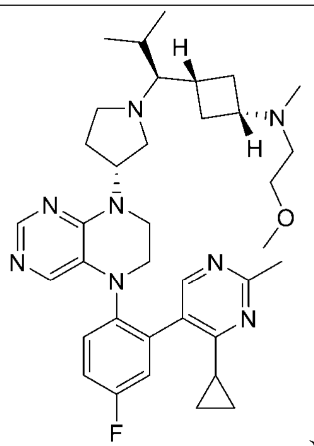 , | 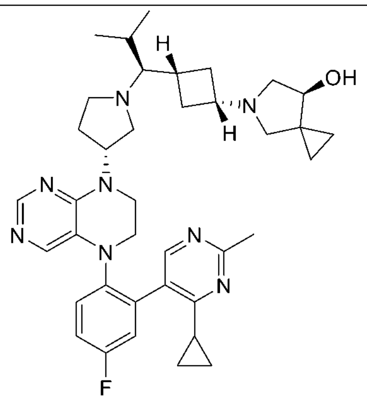 , |
| 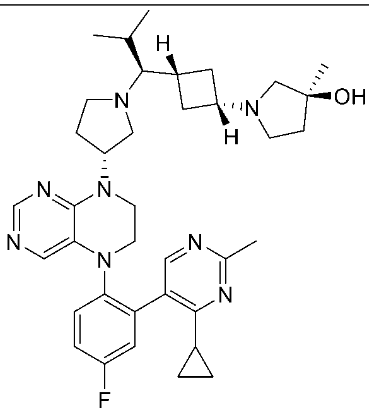 , | 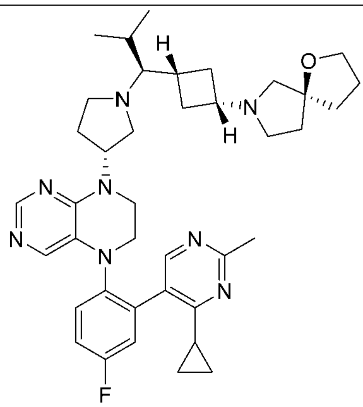 , | 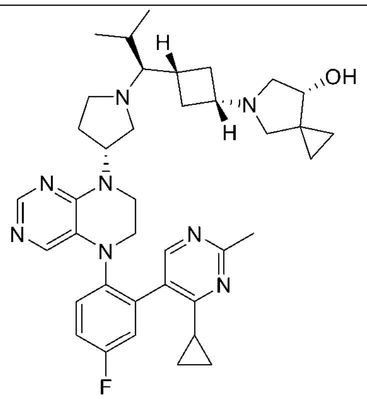 , |

| | | |
|---|---|---|
| 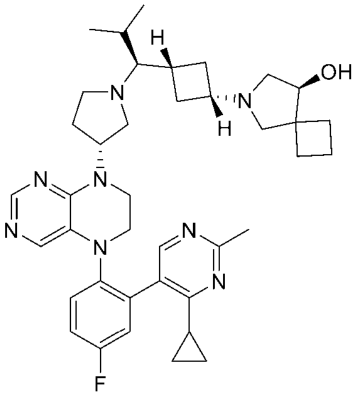 、 | 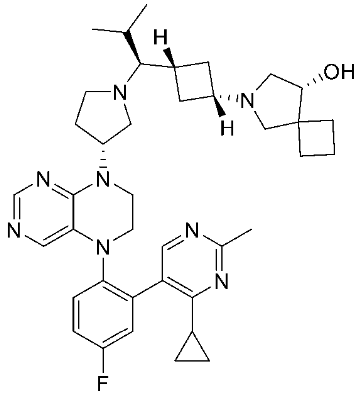 、 | 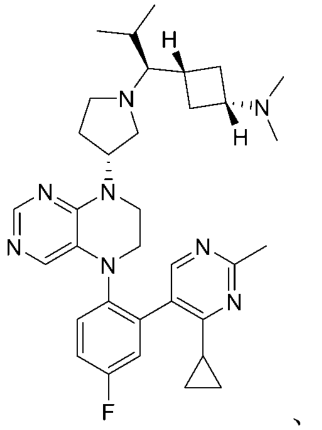 、 |
| 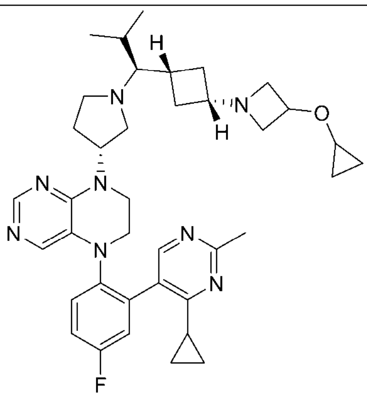 、 | 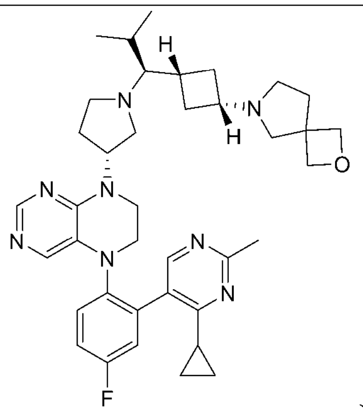 、 | 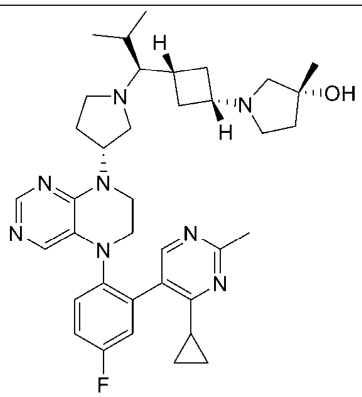 、 |
| 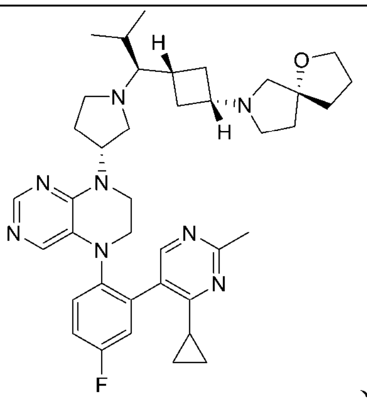 、 | 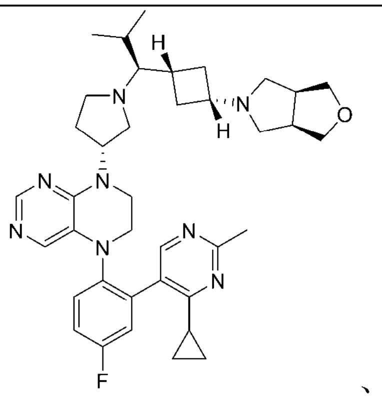 、 | 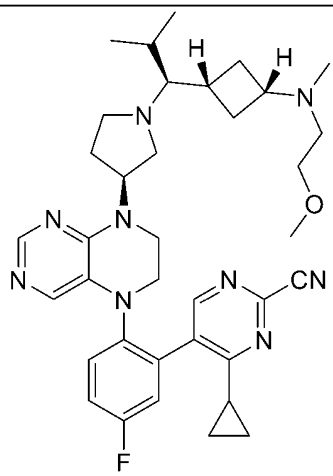 、 |
| 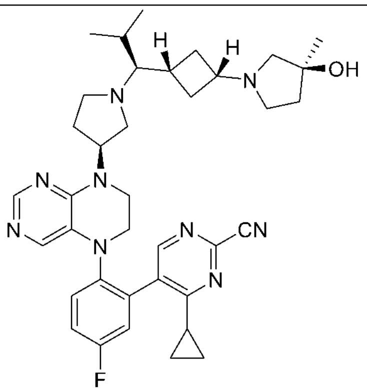 、 | 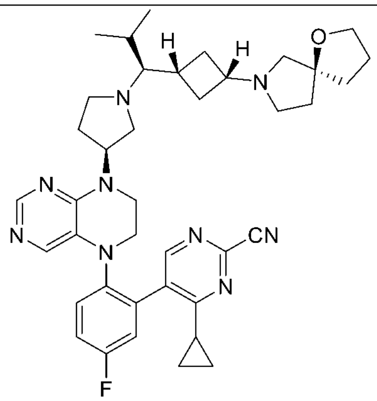 、 | 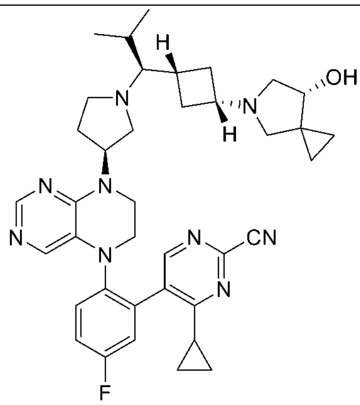 、 |

| | | |
|---|---|---|
| 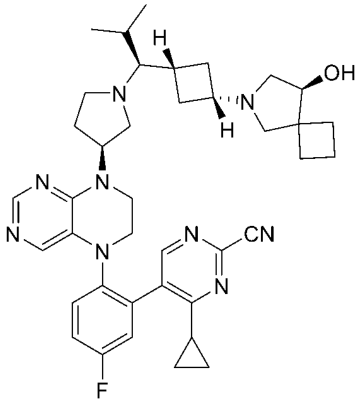 、 | 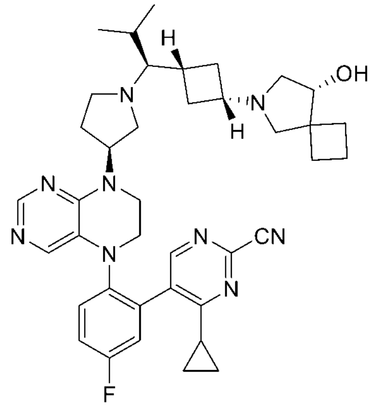 、 | 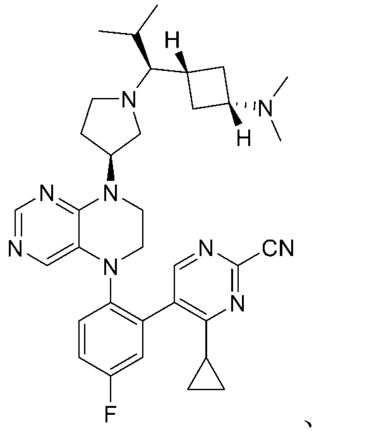 、 |
| 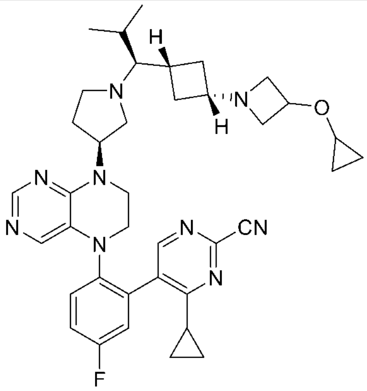 、 | 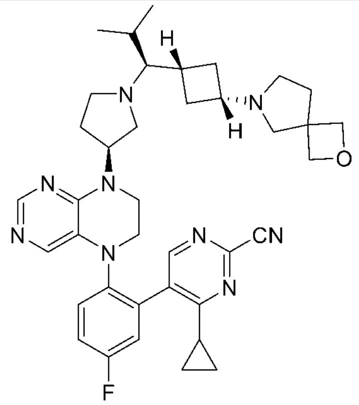 、 | 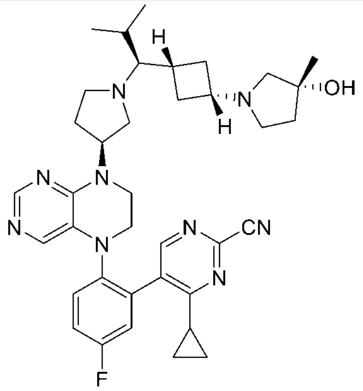 、 |
| 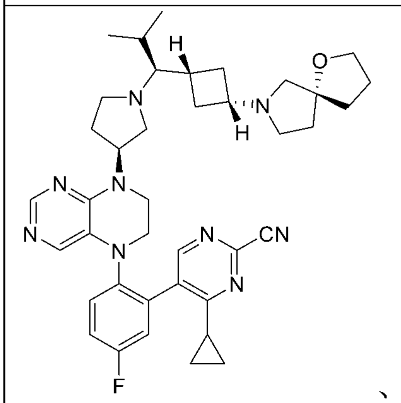 、 | 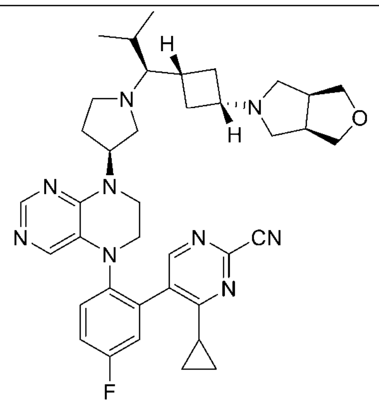 、 | 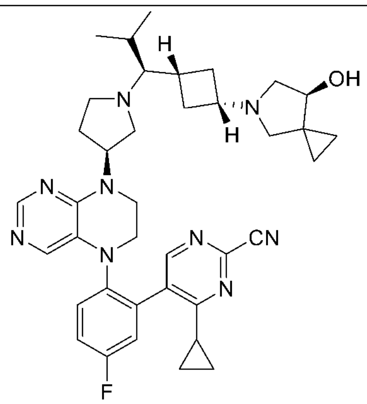 、 |
| 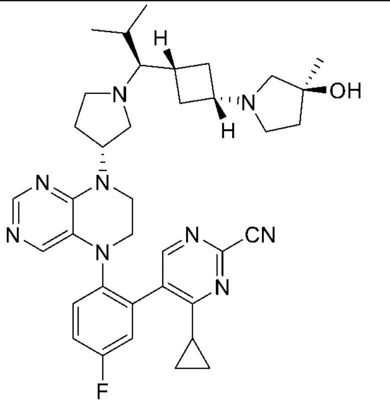 、 | 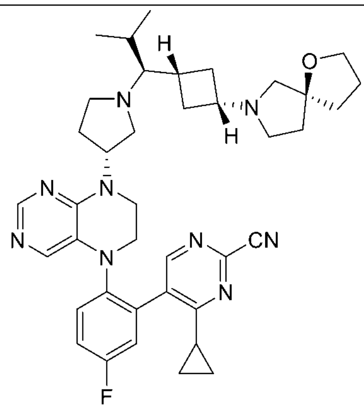 、 | 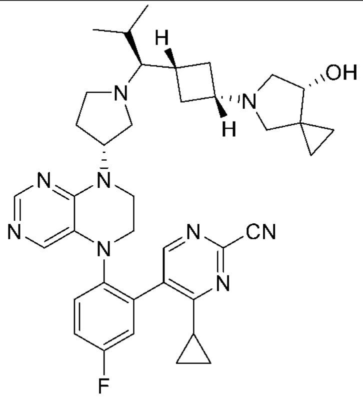 、 |

| | | |
|---|---|---|
| 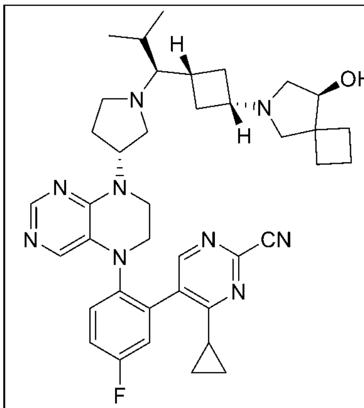 、 | 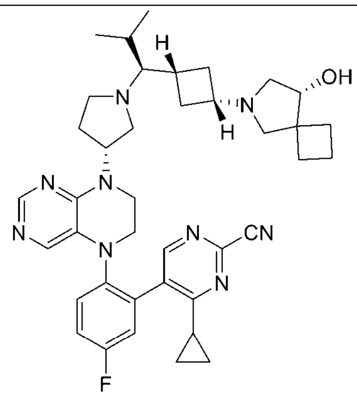 、 | 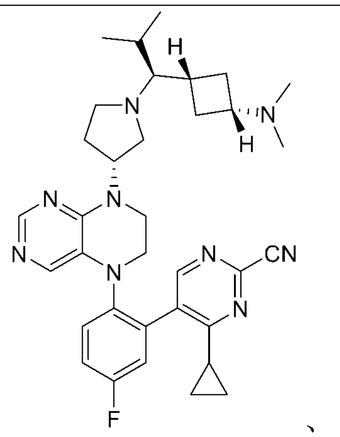 、 |
| 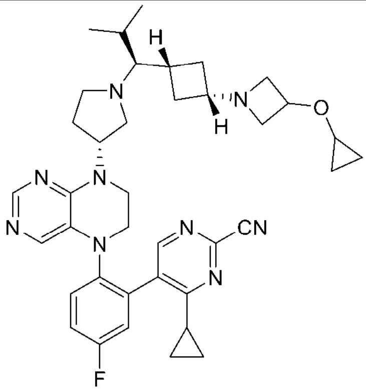 、 | 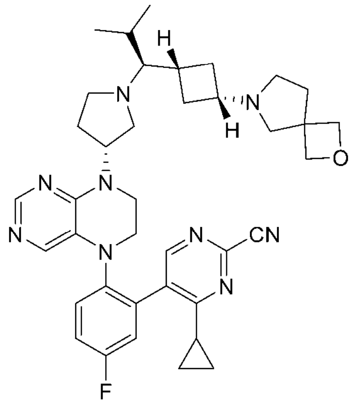 、 | 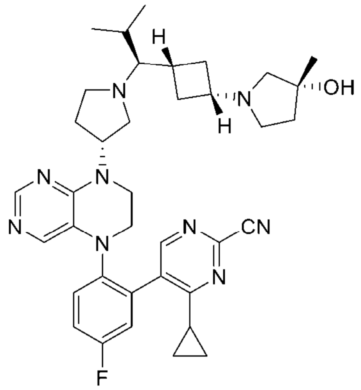 、 |
| 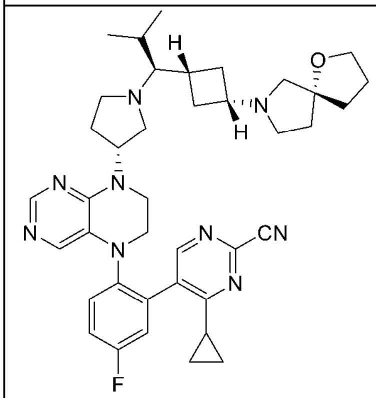 、 | 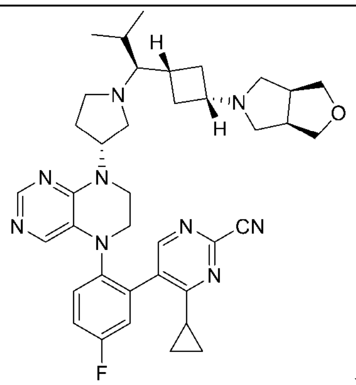 、 | 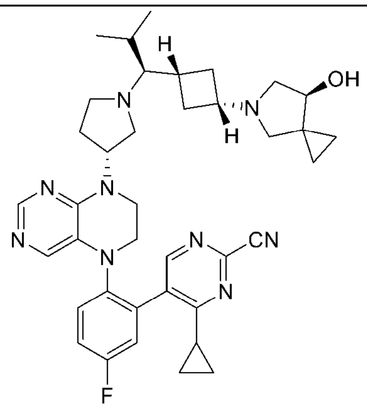 、 |
| 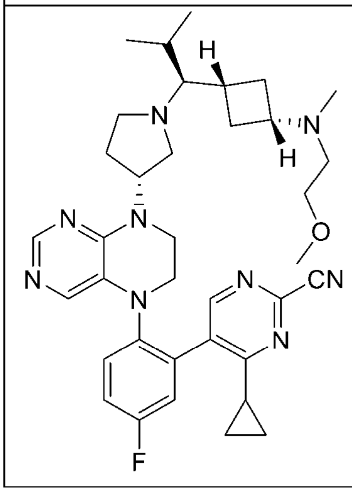 、 | 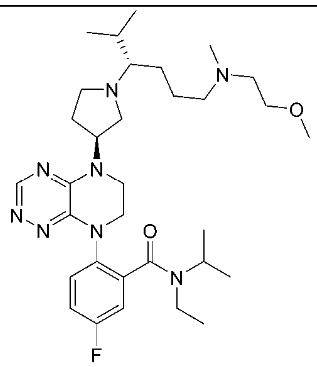 、 | 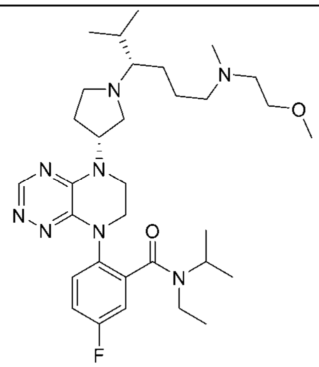 、 |

| | | |
|---|---|---|
| 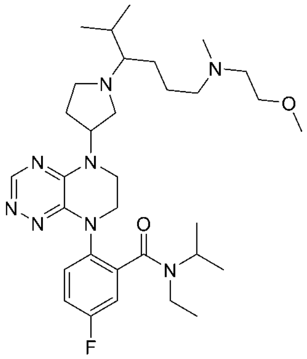 、 | 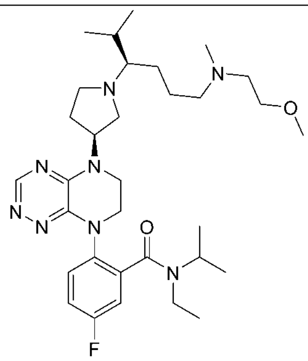 、 | 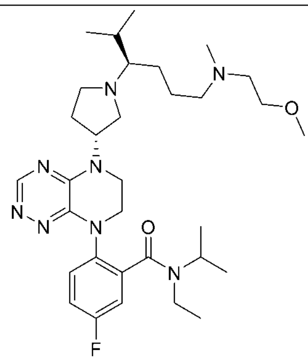 、 |
| 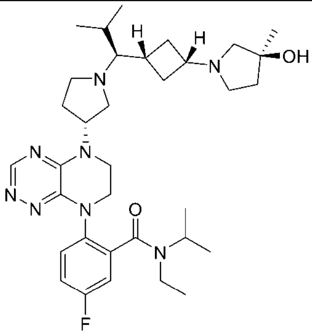 、 | 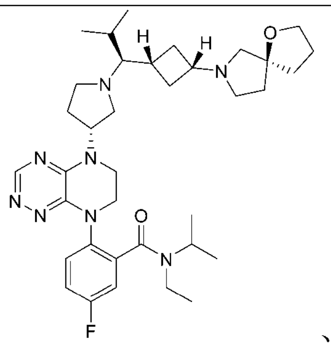 、 | 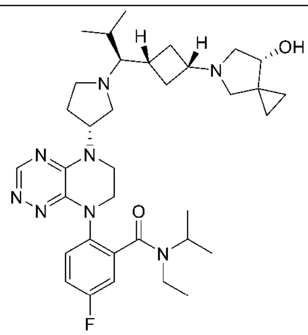 、 |
| 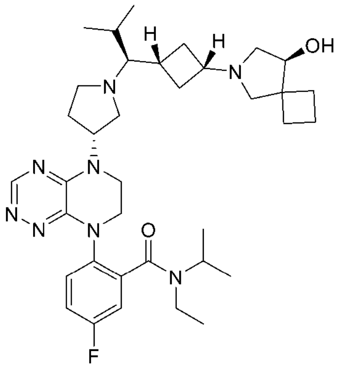 、 | 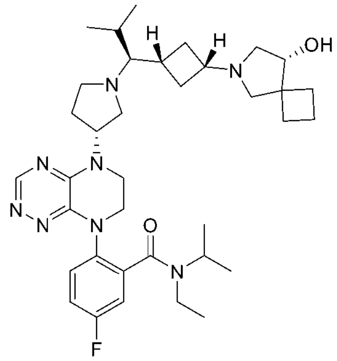 、 | 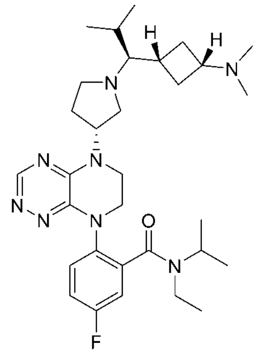 、 |
| 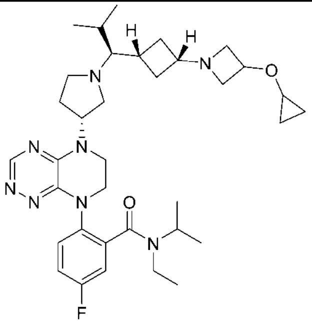 、 | 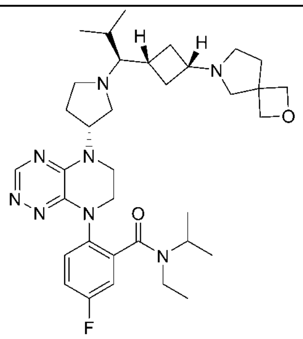 、 | 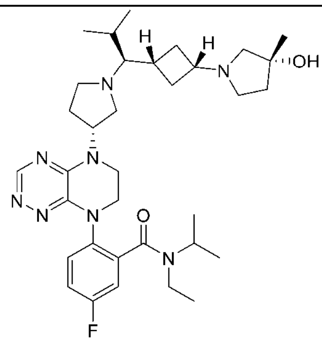 、 |
| 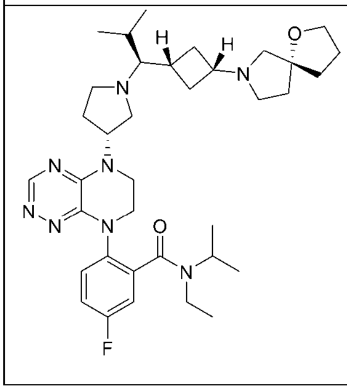 、 | 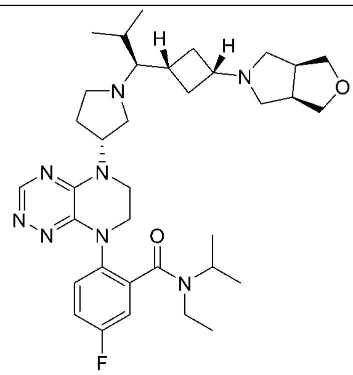 、 | 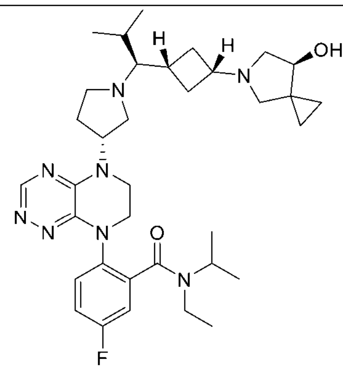 、 |

| | | |
|---|---|---|
| 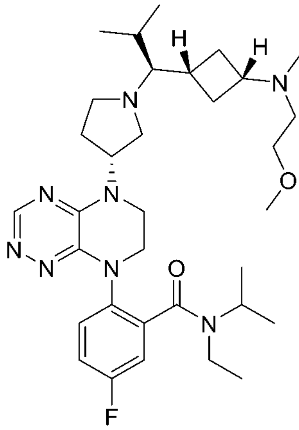 | | |

In certain embodiments, said compound is selected from Table B:

Table B

| | | |
|---|---|---|
| 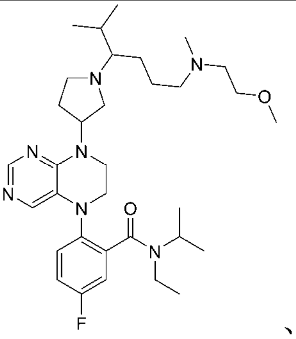 | 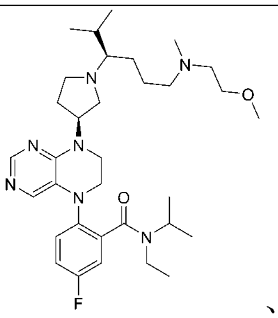 | 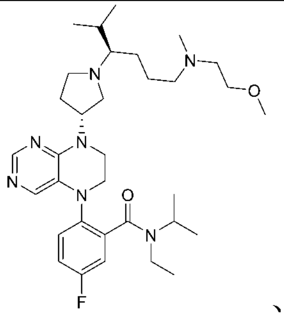 |
| 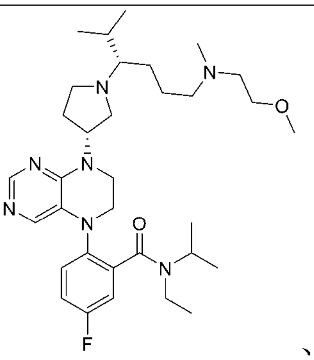 | 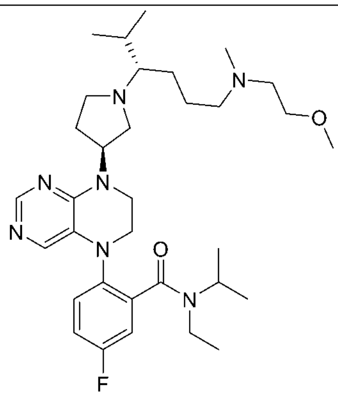 | |

In a second aspect, the present invention provides a pharmaceutical composition comprising a therapeutically effective amount of one or more compounds as described in the first aspect of the present invention, in combination with one or more pharmaceutically acceptable carriers.

In certain embodiments, said compound constitutes 0.001-99.999 wt% of the total composition weight; preferably 0.01-99.99 wt%; more preferably 0.1-90 wt%.

In certain embodiments, said pharmaceutical composition is formulated as an injectable, tablet, capsule, pill, suspension, or emulsion.

In a third aspect, the present invention provides uses of the compound as described in the first aspect or the pharmaceutical composition as described in the second aspect, wherein said uses are selected from one or more of the following (a) to (c):

(a) preparation of a medicament for the prevention or treatment of diseases associated with MLL1, MLL2, MLL fusion protein, and/or Menin activity;

(b) preparation of an inhibitor for in vitro non-therapeutic inhibition of MLL1, MLL2, MLL fusion protein, and/or Menin

activity;

(c) preparation of an inhibitor for in vitro non-therapeutic inhibition of tumor cell proliferation.

In certain embodiments, diseases associated with MLL1, MLL2, MLL fusion protein, and/or Menin activity are selected from: neoplastic diseases, diabetes mellitus, and other disorders associated with MLL1, MLL2, MLL fusion protein, and/or Menin activity.

In certain embodiments, said neoplastic diseases associated with MLL1, MLL2, MLL fusion protein, and/or Menin activity are selected from: leukemia, Ewing sarcoma, breast carcinoma, prostate carcinoma, T-cell lymphoma, B-cell lymphoma, malignant rhabdoid tumor, synovial sarcoma, colorectal carcinoma, endometrial carcinoma, gastric carcinoma, hepatocellular carcinoma, renal cell carcinoma, lung carcinoma, melanoma, ovarian carcinoma, pancreatic carcinoma, glioblastoma, cholangiocarcinoma, nasopharyngeal carcinoma, cervical carcinoma, head and neck squamous cell carcinoma, esophageal carcinoma, thyroid carcinoma, and urothelial carcinoma.

In certain embodiments, said other disorders associated with MLL1, MLL2, MLL fusion protein, and/or Menin activity are selected from: autoimmune disorders, non-alcoholic steatohepatitis.

In certain embodiments, said pharmaceutical composition further comprises one or more additional therapeutic agents.

In certain embodiments, said additional therapeutic agents are antineoplastic agents.

In certain embodiments, said additional therapeutic agents include: DNA-targeting antineoplastic agents such as cisplatin; antimetabolites affecting nucleic acid synthesis such as methotrexate (MTX) and 5-fluorouracil (5-FU); transcription-targeting antineoplastic agents such as Doxorubicin, Epirubicin, Aclarubicin, and Mithramycin; microtubule-targeting agents such as Paclitaxel and Vinorelbine; Aromatase inhibitors such as aminoglutethimide, formestane, letrozole, and anastrozole; and signal transduction inhibitors such as epidermal growth factor receptor inhibitors including Imatinib, Gefitinib, Erlotinib, and Lapatinib.

It is to be understood that, within the scope of the present invention, the above-described technical features and those specifically described hereinafter (e.g., in the Examples) may be combined with one another to constitute new or preferred technical embodiments. Due to space limitations, such combinations are not exhaustively enumerated herein.

## DETAILED DESCRIPTION OF THE INVENTION

Through rational design and comprehensive consideration of target-related activity, hERG channel inhibitory activity, and pharmacokinetic properties, the inventors of the present invention have developed a novel class of compounds that disrupt the Menin-MLL protein-protein interaction. These compounds exhibit favorable antiproliferative activity against MV4-11 cells, substantially reduced hERG inhibition, and excellent in vivo metabolic profiles. Compared with the compounds disclosed in WO2023078426, the compounds of the present invention demonstrate significant advantages and hold promise for development as novel therapeutic agents for the treatment of tumors, diabetes, and other disorders. Based on these findings, the inventors have completed the present invention.

## DEFINITIONS

Unless otherwise defined, all technical and scientific terms used herein have the same meaning as commonly understood by one of ordinary skill in the art to which this invention belongs.

As used herein, the terms "comprising", "including", or "containing" may be open-ended, semi-closed, or closed. In other words, these terms also encompass "consisting essentially of" or "consisting of".

The present invention is further illustrated with reference to specific examples hereinbelow. It should be understood that these examples are provided for illustrative purposes only and are not intended to limit the scope of the present invention. Unless otherwise specified, experimental methods in the following examples for which specific conditions are not indicated are generally performed under conventional conditions or according to the manufacturer's recommendations. Unless otherwise stated, percentages and parts are calculated by weight.

It should be understood that when a particular group is present at multiple different positions within a compound, its definition at each position is independent and may be the same or different. That is, the term "selected from the group consisting of" and the term "independently selected from the group consisting of" have the same meaning.

Unless otherwise specified, the structural formulae described in the present invention are intended to include all stereoisomeric forms (such as enantiomers, diastereomers, and geometric isomers or conformational isomers): for example, R and S configurations at asymmetric centers, (Z) and (E) isomers of double bonds, and the like. Accordingly, single stereochemical isomers of the compounds of the present invention, as well as mixtures of enantiomers, diastereomers, or geometric isomers (or conformational isomers) thereof, are all within the scope of the present invention.

The compounds of the present application may be prepared by various synthetic methods known to those skilled

in the art, including the specific embodiments enumerated below, embodiments formed by combining specific embodiments with other chemical synthesis methods, and equivalent substitution methods known to those skilled in the art. Preferred embodiments include, but are not limited to, the examples of the present application.

## GROUP DEFINITIONS

Definitions of standard chemical terms may be found in reference works, including Carey and Sundberg "ADVANCED ORGANIC CHEMISTRY 4TH ED." Vols. A (2000) and B (2001), Plenum Press, New York. Unless otherwise indicated, conventional methods within the skill of the art are employed, such as mass spectrometry, NMR, IR, and UV/VIS spectroscopy, and pharmacological methods. Unless specific definitions are provided, the terminology employed in the descriptions of analytical chemistry, organic synthetic chemistry, and medicinal and pharmaceutical chemistry herein is that known in the art. Standard techniques may be used in chemical synthesis, chemical analysis, pharmaceutical preparation, formulation, and delivery, as well as in the treatment of patients. For example, reactions may be carried out and purifications performed using manufacturer's instructions for reagent kits, or by methods well-known in the art, or as described in the present invention. Generally, the foregoing techniques and methods may be performed according to conventional procedures well-known in the art, as described in various general and more specific references cited and discussed throughout this specification. In this specification, groups and substituents thereof may be selected by those skilled in the art to provide stable moieties and compounds.

When a substituent is described by a conventional chemical formula written from left to right, the substituent equally includes the chemically equivalent substituent obtained when the structural formula is written from right to left. For example, $-CH_2O-$ is equivalent to $-OCH_2-$.

Section headings used herein are for organizational purposes only and should not be construed as limiting the subject matter described. All documents or portions of documents cited in this application, including but not limited to patents, patent applications, articles, books, manuals, and treatises, are hereby incorporated by reference in their entirety.

Certain chemical groups defined herein are prefixed with a shorthand notation indicating the total number of carbon atoms present in the group. For example, C1-C6 alkyl refers to an alkyl group as defined below having a total of 1 to 6 carbon atoms. The total number of carbon atoms in the shorthand notation does not include carbon atoms that may be present in any substituents of the group.

In addition to the foregoing, when used in the specification and claims of the present application, unless otherwise specifically indicated, the following terms have the meanings indicated below.

Unless otherwise specified, as used herein: the term "halogen" refers to fluorine, chlorine, bromine, or iodine; "hydroxyl" refers to the -OH group; "hydroxyalkyl" refers to an alkyl group as defined below substituted with hydroxyl (-OH); "carbonyl" refers to the -C(=O)- group; "nitro" refers to $-NO_2$; "cyano" refers to -CN; "amino" refers to $-NH_2$; "alkylamino" refers to an amino group in which one or two hydrogen atoms are replaced by alkyl groups as defined below (e.g., $-NH(CH_3)$ or $-N(CH_3)_2$); "alkylsulfonyl" refers to $-SO_2$-alkyl; "alkylsulfinyl" refers to -SO-alkyl; "carboxyl" refers to -COOH.

"Substituted amino" refers to an amino group substituted with one or two alkyl, alkylcarbonyl, arylalkyl, or heteroarylalkyl groups as defined below, such as monoalkylamino, dialkylamino, alkylacylamino, arylalkylamino, or heteroarylalkylamino.

As used herein, either alone or as part of another group (e.g., as used in halo-substituted alkyl and other groups), the term "alkyl" refers to a fully saturated straight-chain or branched hydrocarbon chain radical consisting solely of carbon and hydrogen atoms, having, for example, 1 to 12 (preferably 1 to 8, more preferably 1 to 6, such as 1, 2, 3, or 4) carbon atoms, and attached to the rest of the molecule by one or more single bonds. Examples include, but are not limited to, methyl, ethyl, n-propyl, isopropyl, n-butyl, isobutyl, sec-butyl, tert-butyl, n-pentyl, 2-methylbutyl, 2,2-dimethylpropyl, n-hexyl, heptyl, 2-methylhexyl, 3-methylhexyl, octyl, nonyl, decyl, and the like. For the purposes of the present invention, the term "alkyl" preferably refers to an alkyl group containing 1 to 6 carbon atoms.

As used herein, either alone or as part of another group, the term "alkenyl" refers to a straight-chain or branched hydrocarbon chain radical consisting solely of carbon and hydrogen atoms, containing at least one double bond, having, for example, 2 to 14 (preferably 2 to 10, more preferably 2 to 6, such as 2, 3, or 4) carbon atoms, and attached to the rest of the molecule by one or more single bonds. Examples include, but are not limited to, vinyl, propenyl, allyl, but-1-enyl, but-2-enyl, pent-1-enyl, penta-1,4-dienyl, and the like.

As used herein, either alone or as part of another group, the term "alkynyl" refers to a straight-chain or branched hydrocarbon chain radical consisting solely of carbon and hydrogen atoms, containing at least one carbon-carbon triple bond, having, for example, 2 to 14 (preferably 2 to 10, more preferably 2 to 6, such as 2, 3, or 4) carbon atoms, and attached to the rest of the molecule by one or more single bonds. Examples include, but are not limited to, ethynyl, 1-propynyl, 1-butynyl, heptynyl, octynyl, and the like.

As used herein, either alone or as part of another group, the term "carbocycle" or "carbocyclyl" refers to a stable non-aromatic monocyclic or polycyclic hydrocarbon radical consisting solely of carbon and hydrogen atoms, which may include fused ring systems, bridged ring systems, or spiro ring systems, having 3 to 15 carbon atoms (preferably 3 to 10,

more preferably 3 to 8, still more preferably 3 to 6 carbon atoms), which may be saturated or unsaturated (i.e., cycloalkyl, cycloalkenyl, etc.), and attached to the rest of the molecule via any suitable carbon atom through one or more single bonds. Unless otherwise specifically indicated in this specification, carbon atoms in a carbocyclyl group may optionally be oxidized. Examples of carbocyclyl groups include, but are not limited to, cyclopropyl, cyclobutyl, cyclopentyl, cyclohexyl, cycloheptyl, cyclooctyl, adamantyl, 2,3-dihydro-1H-indenyl, octahydro-4,7-methano-1H-indenyl, 1,2,3,4-tetrahydronaphthyl, 5,6,7,8-tetrahydronaphthyl, cyclopentenyl, cyclohexenyl, cyclohexadienyl, 1H-indenyl, 8,9-dihydro-7H-benzocyclohepten-6-yl, 6,7,8,9-tetrahydro-5H-benzocycloheptenyl, 5,6,7,8,9,10-hexahydrobenzocyclooctenyl, fluorenyl, bicyclo[1.1.1]pentane, bicyclo[2.2.1]heptyl, 7,7-dimethyl-bicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, octahydro-2,5-methano-pentalenyl, and the like.

As used herein, either alone or as part of another group, the term "heterocycle" or "heterocyclyl" refers to a stable 3- to 20-membered non-aromatic cyclic radical composed of 2 to 14 carbon atoms and 1 to 6 heteroatoms selected from nitrogen, phosphorus, oxygen, and sulfur. Unless otherwise specifically indicated in this specification, heterocyclyl groups may be monocyclic, bicyclic, tricyclic, or polycyclic ring systems, which may include fused ring systems, bridged ring systems, or spiro ring systems; nitrogen, carbon, or sulfur atoms in the heterocyclyl group may optionally be oxidized; nitrogen atoms may optionally be quaternized; and heterocyclyl groups may be partially or fully saturated. Heterocyclyl groups may be attached to the rest of the molecule via a carbon atom or a heteroatom through one or more single bonds. In fused heterocyclyl groups, one or more rings may be aryl or heteroaryl as defined below, provided that the point of attachment to the rest of the molecule is through a non-aromatic ring atom. For the purposes of the present invention, heterocyclyl groups are preferably stable 4- to 11-membered non-aromatic monocyclic, bicyclic, bridged, or spiro ring radicals containing 1 to 3 heteroatoms selected from nitrogen, oxygen, and sulfur, more preferably stable 4- to 8-membered non-aromatic monocyclic, bicyclic, bridged, or spiro ring radicals containing 1 to 3 heteroatoms selected from nitrogen, oxygen, and sulfur. Examples of heterocyclyl groups include, but are not limited to: pyrrolidinyl, morpholinyl, piperazinyl, homopiperazinyl, piperidinyl, thiomorpholinyl, 2-azabicyclo[2.2.2]octanyl, 2,7-diazaspiro[3.5]nonan-7-yl, 2-oxa-6-azaspiro[3.3]heptan-6-yl, 2,5-diazabicyclo[2.2.1]heptan-2-yl, azetidinyl, pyranyl, tetrahydropyranyl, thiopyranyl, tetrahydrofuranyl, oxazinyl, dioxolanyl, tetrahydroisoquinolinyl, decahydroisoquinolinyl, imidazolinyl, imidazolidinyl, quinolizinyl, thiazolidinyl, isothiazolidinyl, isoxazolidinyl, dihydroindolyl, octahydroindolyl, octahydroisoindolyl, pyrrolidinyl, pyrazolidinyl, phthalimidyl, and the like.

As used herein, either alone or as part of another group, the term "aryl" refers to a conjugated hydrocarbon ring system radical having 6 to 18 carbon atoms (preferably 6 to 10 carbon atoms). For the purposes of the present invention, aryl groups may be monocyclic, bicyclic, tricyclic, or polycyclic ring systems, and may also be fused with carbocyclyl or heterocyclyl groups as defined above, provided that the aryl group is attached to the rest of the molecule via an atom on the aromatic ring through one or more single bonds. Examples of aryl groups include, but are not limited to, phenyl, naphthyl, anthracenyl, phenanthrenyl, fluorenyl, 2,3-dihydro-1H-isoindolyl, 2-benzoxazolinonyl, 2H-1,4-benzoxazin-3(4H)-on-7-yl, and the like.

As used herein, the term "arylalkyl" refers to an alkyl group as defined above substituted with an aryl group as defined above.

As used herein, either alone or as part of another group, the term "heteroaryl" refers to a 5-to 16-membered conjugated ring system radical having 1 to 15 carbon atoms (preferably 1 to 10 carbon atoms) and 1 to 6 heteroatoms selected from nitrogen, oxygen, and sulfur within the ring. Unless otherwise specifically indicated in this specification, heteroaryl groups may be monocyclic, bicyclic, tricyclic, or polycyclic ring systems, and may also be fused with carbocyclyl or heterocyclyl groups as defined above, provided that the heteroaryl group is attached to the rest of the molecule via an atom on the heteroaromatic ring through one or more single bonds. Nitrogen, carbon, or sulfur atoms in the heteroaryl group may optionally be oxidized; nitrogen atoms may optionally be quaternized. For the purposes of the present invention, heteroaryl groups are preferably stable 5- to 12-membered aromatic radicals containing 1 to 5 heteroatoms selected from nitrogen, oxygen, and sulfur, more preferably stable 5- to 10-membered aromatic radicals containing 1 to 4 heteroatoms selected from nitrogen, oxygen, and sulfur, or 5- to 6-membered aromatic radicals containing 1 to 3 heteroatoms selected from nitrogen, oxygen, and sulfur. Examples of heteroaryl groups include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furyl, pyrrolyl, triazolyl, tetrazolyl, triazinyl, indolizinyl, isoindolyl, indazolyl, isoindazolyl, purinyl, quinolinyl, isoquinolinyl, cinnolinyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, thiophenyl, imidazo[1,2-a]pyridinyl, imidazo[1,2-b]pyridazinyl, imidazo[1,2-a]pyrazinyl, and the like.

As used herein, the term "heteroarylalkyl" refers to an alkyl group as defined above substituted with a heteroaryl group as defined above.

As used herein, the term "absent" or "a bond" means that the groups on either side of the defined moiety are directly connected through a chemical bond. For example, "B is absent in A-B-C" means "A-C".

As used herein, the term "$-[CH_2]_n-$" means that the groups on either side of the defined moiety are connected by n

methylene groups. When n is 0, it means that the groups on either side of the defined moiety are directly connected through a chemical bond.

As used herein, The symbol

“ 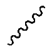 ”

in

“ 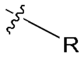 ”

indicates the point of attachment of group R.

As used herein, unless otherwise stated, the terms "optionally" or "optional" indicate that the subsequently described event or circumstance may or may not occur, and that the description includes both the occurrence and non-occurrence of said event or circumstance. For example, "optionally substituted aryl" indicates that the aryl group may or may not be substituted, and the description includes both substituted and unsubstituted aryl groups. For example, unless substituents are explicitly listed, the terms "optionally substituted", "substituted", or "substituted with" as used herein mean that one or more hydrogen atoms on a given atom or group are independently replaced by one or more (e.g., 1, 2, 3, or 4) substituents independently selected from: deuterium (D), halogen, -OH, oxo (=O), mercapto, cyano, $-CD_3$, -C1-C6 alkyl (preferably -C1-C3 alkyl), C2-C6 alkenyl, C2-C6 alkynyl, cycloalkyl (preferably C3-C8 cycloalkyl), aryl, heterocyclyl (preferably 3- to 8-membered heterocyclyl), heteroaryl, aryl-Cl-C6 alkyl-, heteroaryl-Cl-C6 alkyl-, C1-C6 haloalkyl-, -OC1-C6 alkyl (preferably -OC1-C3 alkyl), -OC2-C6 alkenyl, -O-cycloalkyl, -O-heterocyclyl, -O-aryl, -O-heteroaryl, -OC1-C6 alkylphenyl, -C1-C6 alkyl-OH (preferably -C1-C4 alkyl-OH), -C1-C6 alkyl-SH, -C1-C6 alkyl-O-C1-C6 alkyl, - OC1-C6 haloalkyl, $-NH_2$, -C1-C6 alkyl-$NH_2$ (preferably -C1-C3 alkyl-$NH_2$), -N(C1-C6 alkyl)$_2$ (preferably -N(C1-C3 alkyl)$_2$), -NH(C1-C6 alkyl) (preferably -NH(C1-C3 alkyl)), and the like. When an atom or group is substituted by multiple substituents, said substituents may be the same or different. As used herein, the terms "moiety", "structural moiety", "chemical moiety", "group", or "chemical group" refer to a specific fragment or functional group within a molecule. A chemical moiety is typically considered to be a chemical entity embedded in or appended to a molecule.

In the present invention, di(C1-C4 alkyl)amino represents an amine substituted with two identical or different C1-C4 alkyl groups, such as

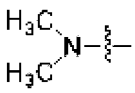 、 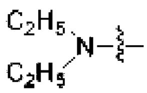 、 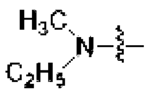 、

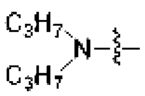 or 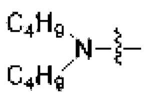

et.

Unless otherwise stated, as used herein, "plurality" or "multiple" refers to 2, 3, or 4.

## ACTIVE INGREDIENT

As used herein, "compound of the present invention" or "active ingredient" refers to a compound represented by Formula I, and also includes pharmaceutically acceptable salts, enantiomers, diastereomers, tautomers, geometric isomers, solvates, polymorphs, deuterated derivatives, or combinations thereof.

"Stereoisomers" refer to compounds composed of the same atoms bonded in the same manner but having different three-dimensional structures. The present invention encompasses various stereoisomers and mixtures thereof.

When a compound of the present invention contains an alkene double bond, unless otherwise stated, the compounds of the present invention are intended to include both E- and Z-geometric isomers.

"Tautomers" refer to isomers formed by the transfer of a proton from one atom of a molecule to another atom of the same molecule. All tautomeric forms of the compounds of the present invention are also included within the scope of the present invention.

The compounds of the present invention, or pharmaceutically acceptable salts thereof, may contain one or more chiral carbon atoms and thus may give rise to enantiomers, diastereomers, and other stereoisomeric forms. Each chiral carbon atom may be defined as (R)- or (S)- based on its stereochemistry. The present invention is intended to include all

possible isomers, as well as racemic and optically pure forms thereof. Preparation of the compounds of the present invention may employ racemates, diastereomers, or enantiomers as starting materials or intermediates. Optically active isomers may be prepared using chiral synthons or chiral reagents, or resolved using conventional techniques such as crystallization and chiral chromatography.

Conventional techniques for preparing/separating individual isomers include chiral synthesis from a suitable optically pure precursor, or resolution of racemates (or racemates of salts or derivatives) using, for example, chiral high-performance liquid chromatography. Reference may be made to Gerald Gübitz and Martin G. Schmid (Eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Furniss et al. (eds.), VOGEL'S ENCYCLOPEDIA OF PRACTICAL ORGANIC CHEMISTRY 5TH ED., Longman Scientific and Technical Ltd., Essex, 1991, 809-816; Heller, Acc. Chem. Res. 1990, 23, 128.

As used herein, the term "pharmaceutically acceptable salt" includes pharmaceutically acceptable acid addition salts and pharmaceutically acceptable base addition salts.

"Pharmaceutically acceptable acid addition salts" refer to salts formed with inorganic or organic acids that retain the biological effectiveness of the free base without other adverse effects. Inorganic acid salts include, but are not limited to, hydrochloride, hydrobromide, sulfate, nitrate, phosphate, and the like. Organic acid salts include, but are not limited to, formate, acetate, 2,2-dichloroacetate, trifluoroacetate, propionate, hexanoate, octanoate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalenedisulfonate, and the like. These salts may be prepared by methods known in the art.

"Pharmaceutically acceptable base addition salts" refer to salts formed with inorganic or organic bases that retain the biological effectiveness of the free acid without other adverse effects. Salts derived from inorganic bases include, but are not limited to, sodium, potassium, lithium, ammonium, calcium, magnesium, iron, zinc, copper, manganese, aluminum salts, and the like. Preferred inorganic salts are ammonium, sodium, potassium, calcium, and magnesium salts. Salts derived from organic bases include, but are not limited to, salts of primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resins, and the like. Preferred organic bases include isopropylamine, diethylamine, ethanolamine, trimethylamine, dicyclohexylamine, choline, and caffeine. These salts may be prepared by methods known in the art.

It should be understood that the specific methods for preparing the compounds of Formula (I) of the present invention do not constitute any limitation on the present invention. The compounds of the present invention may also be conveniently prepared by optionally combining various synthetic methods described in this specification or known in the art, and such combinations can be readily performed by those skilled in the art to which the present invention pertains.

Typically, the compounds of the present invention may be prepared according to the synthetic procedures illustrated in the Examples, wherein the starting materials and reagents used, unless otherwise specified, are commercially available.

**Pharmaceutical Compositions and Methods of Administration**

The present invention further provides a pharmaceutical composition comprising a pharmaceutically acceptable carrier and one or more compounds of the present invention, or a pharmaceutically acceptable salt, stereoisomer, or deuterated derivative thereof, in a safe and effective amount.

Given the excellent antitumor activity of the compounds of the present invention, the compounds and their various crystalline forms, pharmaceutically acceptable inorganic or organic salts, hydrates or solvates, as well as pharmaceutical compositions containing the compounds of the present invention as the primary active ingredient, may be used for the treatment, prevention, and alleviation of tumor-related diseases.

The pharmaceutical composition of the present invention comprises a compound of the present invention, or a pharmacologically acceptable salt thereof, in a safe and effective amount, together with a pharmacologically acceptable excipient or carrier. The term "safe and effective amount" refers to an amount of the compound sufficient to produce a significant improvement in the condition without causing serious adverse effects. Typically, the pharmaceutical composition contains 1-2000 mg of the compound of the present invention per dose, more preferably 10-1000 mg per dose. Preferably, one "dose" refers to one capsule or tablet.

As used herein, "pharmaceutical composition" refers to a formulation of a compound of the present invention with a medium commonly accepted in the art for delivering biologically active compounds to mammals (e.g., humans). Such

medium includes pharmaceutically acceptable carriers. The purpose of the pharmaceutical composition is to facilitate administration to an organism and to enable absorption of the active ingredient, thereby exerting biological activity.

As used herein, the term "pharmaceutically acceptable" refers to substances (such as carriers or diluents) that do not affect the biological activity or properties of the compounds of the present invention and are relatively non-toxic, i.e., substances that can be administered to an individual without causing adverse biological reactions or interacting in an undesirable manner with any component contained in the composition.

As used herein, "pharmaceutically acceptable carrier" includes, but is not limited to, any adjuvant, carrier, excipient, glidant, sweetening agent, diluent, preservative, dye/colorant, flavoring agent, surfactant, wetting agent, dispersing agent, suspending agent, stabilizer, isotonic agent, solvent, or emulsifier approved by the relevant governmental regulatory authorities for use in humans or livestock.

The term "tumor" as used in the present invention includes, but is not limited to, lung cancer, pancreatic cancer, colorectal cancer, leukemia, Ewing's sarcoma, breast cancer, prostate cancer, T-cell lymphoma, B-cell lymphoma, malignant rhabdoid tumor, synovial sarcoma, endometrial carcinoma, gastric cancer, hepatocellular carcinoma, renal cell carcinoma, melanoma, ovarian cancer, glioblastoma, cholangiocarcinoma, nasopharyngeal carcinoma, cervical cancer, head and neck cancer, esophageal cancer, thyroid cancer, and bladder cancer.

In a preferred embodiment, tumors associated with MLL1, MLL2, MLL fusion proteins, and/or menin protein activity are selected from the group consisting of: leukemia, Ewing's sarcoma, breast cancer, prostate cancer, T-cell lymphoma, B-cell lymphoma, malignant rhabdoid tumor, synovial sarcoma, colorectal cancer, endometrial carcinoma, gastric cancer, hepatocellular carcinoma, renal cell carcinoma, lung cancer, melanoma, ovarian cancer, pancreatic cancer, glioblastoma, cholangiocarcinoma, nasopharyngeal carcinoma, cervical cancer, head and neck cancer, esophageal cancer, thyroid cancer, and bladder cancer. Related "other diseases" include, but are not limited to, autoimmune diseases, nonalcoholic steatohepatitis, and the like.

As used herein, the terms "prophylactic," "prophylaxis," and "prevention" refer to reducing the likelihood of occurrence or worsening of a disease or condition in a patient.

As used herein, the term "treatment" and other similar synonyms include the following meanings:

(i) preventing a disease or condition from occurring in a mammal, particularly when such mammal is predisposed to having the disease or condition but has not yet been diagnosed as having it;
(ii) inhibiting the disease or condition, i.e., arresting its progression;
(iii) relieving the disease or condition, i.e., causing regression of the disease or condition state; or (iv) alleviating the symptoms caused by the disease or condition.

As used herein, the terms "effective amount," "therapeutically effective amount," or "pharmaceutically effective amount" refer to an amount of at least one agent or compound that, upon administration, is sufficient to relieve to some extent one or more symptoms of the disease or condition being treated. The result may be reduction and/or alleviation of signs, symptoms, or causes, or any other desired change in a biological system. For example, an "effective amount" for therapeutic use is the amount of a composition comprising a compound disclosed herein required to provide a clinically significant disease symptom relief. An appropriate effective amount for any individual case may be determined using techniques such as dose escalation studies.

As used herein, the terms "administering," "administration," "dosing," and the like refer to methods capable of delivering a compound or composition to the desired site of biological action. These methods include, but are not limited to, oral administration, duodenal administration, parenteral injection (including intravenous, subcutaneous, intraperitoneal, intramuscular, intraarterial injection, or infusion), topical administration, and rectal administration. Administration techniques that may be employed with the compounds and methods described herein are well known to those skilled in the art, for example, as discussed in Goodman and Gilman, The Pharmacological Basis of Therapeutics, current ed.; Pergamon; and Remington's, Pharmaceutical Sciences (current edition), Mack Publishing Co., Easton, Pa. In preferred embodiments, the compounds and compositions discussed herein are administered orally.

As used herein, the terms "drug combination," "pharmaceutical combination," "combination therapy," "administration of additional therapy," "administration of additional therapeutic agents," and the like refer to pharmaceutical treatments obtained by mixing or combining more than one active ingredient, including both fixed and non-fixed combinations of active ingredients. The term "fixed combination" means that at least one compound described herein and at least one co-agent are administered to a patient simultaneously in the form of a single entity or single dosage form. The term "non-fixed combination" means that at least one compound described herein and at least one co-agent are administered to a patient simultaneously, concomitantly, or sequentially at variable intervals as separate entities. These terms also apply to cocktail therapies, for example, the administration of three or more active ingredients.

Preferably, the pharmaceutical composition further comprises other pharmaceutically acceptable therapeutic agents, particularly other antitumor agents. Such therapeutic agents include, but are not limited to: antitumor agents that act on DNA chemical structure, such as cisplatin; antitumor agents that affect nucleic acid synthesis, such as methotrexate

(MTX) and 5-fluorouracil (5-FU); antitumor agents that affect nucleic acid transcription, such as Doxorubicin, Epirubicin, Aclarubicin, and Mithramycin; antitumor agents that act on tubulin synthesis, such as Paclitaxel and Vinorelbine; aromatase inhibitors, such as Aminoglutethimide, Formestane, Letrozole, and Exemestane; and cell signaling pathway inhibitors, such as epidermal growth factor receptor inhibitors including Imatinib, Gefitinib, Erlotinib, and Lapatinib.

"Pharmaceutically acceptable carrier" refers to one or more compatible solid or liquid fillers or gel substances that are suitable for human use and must have sufficient purity and sufficiently low toxicity. "Compatible" herein means that the components of the composition can be blended with the compounds of the present invention and with each other without significantly diminishing the pharmacological efficacy of the compounds. Examples of pharmaceutically acceptable carriers include cellulose and its derivatives (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agents (such as sodium lauryl sulfate), colorants, flavoring agents, stabilizers, antioxidants, preservatives, pyrogen-free water, and the like.

The pharmaceutical composition may be in the form of an injection, capsule, tablet, pill, powder, or granule.

There is no particular limitation on the mode of administration of the compound or pharmaceutical composition of the present invention. Representative modes of administration include, but are not limited to: oral, intratumoral, rectal, parenteral (intravenous, intramuscular, or subcutaneous), and topical administration.

Solid dosage forms for oral administration include capsules, tablets, pills, powders, and granules. In such solid dosage forms, the active compound is mixed with at least one conventional inert excipient (or carrier), such as sodium citrate or dicalcium phosphate, or with the following components: (a) fillers or extenders, for example, starch, lactose, sucrose, glucose, mannitol, and silicic acid; (b) binders, for example, hydroxymethyl cellulose, alginates, gelatin, polyvinyl pyrrolidone, sucrose, and acacia; (c) humectants, for example, glycerol; (d) disintegrants, for example, agar, calcium carbonate, potato starch or tapioca starch, alginic acid, certain complex silicates, and sodium carbonate; (e) solution retardants, for example, paraffin; (f) absorption accelerators, for example, quaternary ammonium compounds; (g) wetting agents, for example, cetyl alcohol and glyceryl monostearate; (h) adsorbents, for example, kaolin; and (i) lubricants, for example, talc, calcium stearate, magnesium stearate, solid polyethylene glycol, sodium lauryl sulfate, or mixtures thereof. In capsules, tablets, and pills, the dosage forms may also contain buffering agents.

Solid dosage forms such as tablets, dragees, capsules, pills, and granules may be prepared with coatings and shell materials, such as enteric coatings and other materials well known in the art. They may contain opacifying agents, and the release of the active compound or compounds in such compositions may be delayed in a certain part of the digestive tract. Examples of embedding components that may be employed are polymeric substances and waxes. When necessary, the active compound may also be formed into microcapsules with one or more of the above-mentioned excipients.

Liquid dosage forms for oral administration include pharmaceutically acceptable emulsions, solutions, suspensions, syrups, or tinctures. In addition to the active compound, liquid dosage forms may contain inert diluents commonly used in the art, such as water or other solvents, solubilizing agents and emulsifiers, for example, ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, and sesame oil, or mixtures of these substances.

In addition to these inert diluents, the composition may also contain adjuvants such as wetting agents, emulsifying agents and suspending agents, sweetening agents, flavoring agents, and perfuming agents.

In addition to the active compound, suspensions may contain suspending agents, for example, ethoxylated isostearyl alcohol, polyoxyethylene sorbitol and sorbitan esters, microcrystalline cellulose, aluminum methoxide, and agar, or mixtures of these substances.

Compositions for parenteral injection may comprise physiologically acceptable sterile aqueous or non-aqueous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

Dosage forms of compounds of the present invention for topical administration include ointments, powders, patches, sprays, and inhalants. The active ingredient is mixed under sterile conditions with a physiologically acceptable carrier and any preservatives, buffers, or propellants that may be required.

The compounds of the present invention may be administered alone or in combination with other pharmaceutically acceptable compounds (such as antitumor agents).

The therapeutic methods of the present invention may be administered alone or in combination with other therapeutic means or therapeutic agents.

When using the pharmaceutical composition, a safe and effective amount of the compound of the present invention is administered to a mammal (such as a human) in need of treatment, wherein the administered dose is a pharmaceutically recognized effective dose. For a person weighing 60 kg, the daily dosage is generally 1-2000 mg, preferably 50-1000 mg. Of course, the specific dosage should also take into account factors such as the route of administration and the patient's health status, which are within the skill of a competent physician.

Compared with the prior art, the main advantages of the present invention include:

(1) The present invention provides a compound of Formula I or a pharmaceutically acceptable salt thereof;
(2) The present invention provides a compound of Formula I for use in the preparation of a pharmaceutical composition for the prevention and treatment of diseases associated with MLL1, MLL2, MLL fusion proteins, and/or menin protein activity.

The present invention is further illustrated below with reference to specific examples. It should be understood that these examples are provided solely for illustrative purposes and are not intended to limit the scope of the present invention. Experimental methods for which specific conditions are not specified in the following examples are generally performed according to conventional conditions, such as those described in Sambrook et al., Molecular Cloning: A Laboratory Manual (New York: Cold Spring Harbor Laboratory Press, 1989), or according to the conditions recommended by the manufacturer. Unless otherwise specified, percentages and parts are calculated by weight.

Unless otherwise defined, all technical and scientific terms used herein have the same meaning as commonly understood by those skilled in the art. Furthermore, any methods and materials similar or equivalent to those described herein may be employed in the methods of the present invention. The preferred methods and materials described herein are provided for illustrative purposes only.

The experimental materials and reagents used in the following examples are commercially available unless otherwise specified.

In each example, $^{1}$H NMR spectra were recorded on a BRUKER AVANCE NEO 400 MHz nuclear magnetic resonance spectrometer, with chemical shifts expressed as δ (ppm); liquid chromatography-mass spectrometry (LC-MS) was performed using a Shimadzu LC-20AD, SIL-20A, CTO-20AC, SPD-M20A, CBM-20A, LCMS-2020 mass spectrometer; preparative HPLC purification was conducted using a Gilson-281 liquid chromatograph.

**Example 1**

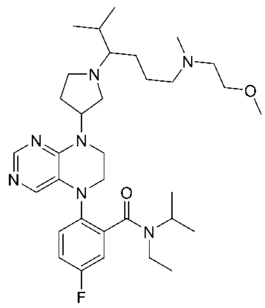

**1**

The synthetic route for Compound 1 is illustrated below:

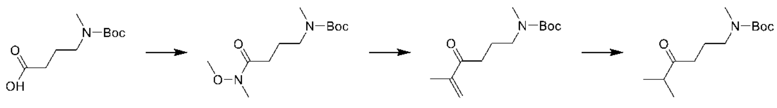

1-1 1-2 1-3 1-4

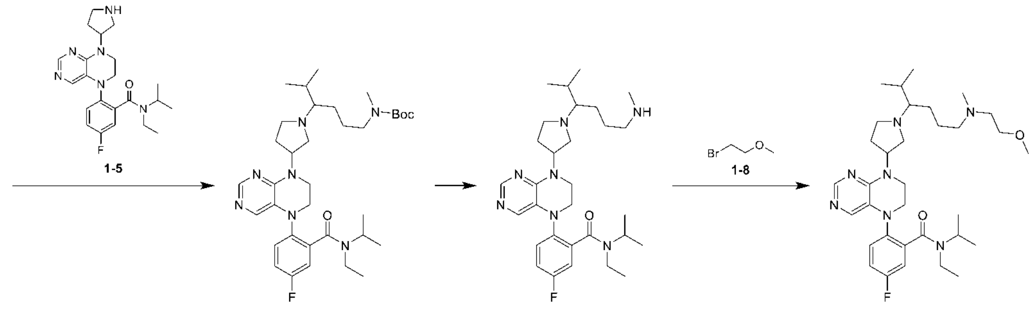


1-6 1-7 1

(1) To a solution of Compound **1-1** (5.00 g, 23.0 mmol) in dichloromethane (80.0 mL) were added N,N-diisopropylethylamine (8.92 g, 69.0 mmol, 12.0 mL), 1-hydroxybenzotriazole (3.73 g, 27.6 mmol), and 1-(3-dimethylaminopropyl)-3-ethylcarbodiimide hydrochloride (5.29 g, 27.6 mmol). The reaction mixture was stirred at 25°C for 1 hour. Subsequently, N,O-dimethylhydroxylamine (4.49 g, 46.0 mmol) was added, and the reaction mixture was stirred at 25°C for 5 hours. The mixture was washed with aqueous citric acid solution (50.0 mL), followed by washing of the organic phase with aqueous sodium bicarbonate solution (50.0 mL) and water (50.0 mL). The organic phase was dried over anhydrous sodium sulfate, filtered, and concentrated under reduced pressure to afford Compound **1-2.**

MS-ESI $[M+H]^+$, calculated 261, found 261.

$^1H$ NMR (400 MHz, $CDCl_3$) δ 3.67 (s, 3H), 3.27 (t, J = 7.2 Hz, 2H), 3.17 (s, 3H), 2.84 (s, 3H), 2.42 (br t, J = 7.2 Hz, 2H), 1.83 (m, 2H), 1.44 (s, 9H).

(2) To a solution of Compound **1-2** (2.00 g, 7.68 mmol) in tetrahydrofuran (60.0 mL) at 0°C was added isopropenylmagnesium bromide (1 mol/L, 19.2 mL). The reaction mixture was stirred at 0°C under nitrogen atmosphere for 1 hour, then stirred for an additional 12 hours at room temperature. The reaction was quenched with aqueous ammonium chloride solution (50.0 mL), and the mixture was extracted with ethyl acetate (50.0 mL). The organic phase was dried over anhydrous sodium sulfate, filtered, and concentrated under reduced pressure to afford Compound 1-3.

MS-ESI $[M-tBu+H]^+$, calculated 186, found 186.

$^1H$ NMR (400 MHz, $CDCl_3$) δ 5.95 (s, 1H), 5.76 (s, 1H), 3.24 (t, J = 6.8 Hz, 2H), 2.83 (s, 3H), 2.69 (t, J = 7.2 Hz, 2H), 1.87 (s, 3H), 1.80-1.85 (m, 2H), 1.43-1.45 (m, 9H).

(3) To a solution of Compound **1-3** (2.00 g, 8.29 mmol) in methanol (20.0 mL) was added palladium on carbon (10%, 200 mg). The reaction mixture was stirred under hydrogen atmosphere at 25°C for 2 hours. The mixture was filtered, concentrated under reduced pressure, and the crude product was purified by silica gel column chromatography (petroleum ether/ethyl acetate = 1:0 to 0:1) to afford Compound **1-4.**

MS-ESI $[M-Boc+H]^+$, calculated 144, found 144.

$^1H$ NMR (400 MHz, $CDCl_3$) δ 3.21 (t, J = 7.2 Hz, 2H), 2.82 (s, 3H), 2.58 (dt, J = 14.0, 6.8 Hz, 1H), 2.44 (t, J = 7.2 Hz, 2H), 1.77 (quin, J = 7.2 Hz, 2H), 1.45 (s, 9H), 1.09 (d, J = 6.8 Hz, 6H).

(4) To a solution of the hydrochloride salt of Compound **1-5** (100 mg, 242 μmol; synthesis described in patent WO2023078426) in methanol (20.0 mL) was added triethylamine to adjust the pH to 8. Subsequently, Compound **1-4** (53.0 mg, 218 μmol) and zinc chloride (132 mg, 969 μmol) were added. The reaction mixture was stirred at 80°C under nitrogen atmosphere for 12 hours. Sodium cyanoborohydride (121 mg, 1.94 mmol) was then added, and stirring was continued for 72 hours. The reaction was quenched with water (5.0 mL) and extracted with ethyl acetate (30.0 mL × 1). The combined organic phases were washed with water (75.0 mL × 2) and saturated brine (75.0 mL × 2), dried over anhydrous sodium sulfate, filtered, and concentrated under reduced pressure. The crude product was purified by silica gel column chromatography (dichloromethane/methanol = 1:0 to 10:1) to afford Compound **1-6.**

MS-ESI $[M+H]^+$, calculated 640, found 640.

$^1H$ NMR (400 MHz, $CDCl_3$) δ 7.98-8.14 (m, 1H), 7.27-7.34 (m, 2H), 7.11-7.20 (m, 1H), 7.00-7.10 (m, 1H), 3.58-3.97 (m, 5H), 3.39-3.57 (m, 3H), 3.05-3.33 (m, 4H), 2.57-2.91 (m, 5H), 2.07-2.42 (m, 2H), 1.73 (s, 4H), 1.43-1.47 (m, 9H), 1.23-1.30 (m, 2H), 1.11-1.20 (m, 6H), 1.02-1.08 (m, 3H), 0.81-1.01 (m, 6H).

(5) To a solution of Compound **1-6** (60.0 mg, 93.7 μmol) in dichloromethane (6.0 mL) was added hydrogen

chloride in dioxane (4 mol/L, 1.50 mL). The reaction mixture was stirred at 25°C under nitrogen atmosphere for 1 hour. The mixture was filtered and concentrated under reduced pressure to afford the crude hydrochloride salt of Compound **1-7.**

MS-ESI [M+H]$^+$, calculated 540, found 540.

$^1$H NMR (400 MHz, $CDCl_3$) δ 8.40-8.50 (m, 1H), 7.52-7.66 (m, 1H), 7.36-7.45 (m, 1H), 6.91-7.33 (m, 2H), 3.89-4.19 (m, 4H), 3.68-3.87 (m, 3H), 3.54-3.64 (m, 1H), 3.34-3.51 (m, 3H), 3.00-3.19 (m, 3H), 2.72-2.76 (m, 3H), 2.45-2.65 (m, 2H), 2.25-2.41 (m, 1H), 1.72-2.06 (m, 5H), 1.17-1.27 (m, 6H), 1.03-1.16 (m, 9H).

(6) To a solution of Compound **1-7** (35.0 mg, 64.8 μmol) in N,N-dimethylformamide (3.0 mL) at 0°C was added potassium carbonate (51.0 mg, 369 μmol). Subsequently, Compound **1-8** (16.7 mg, 120 μmol, 11.3 μL) was added. The reaction mixture was stirred at 50°C under nitrogen atmosphere for 5 hours. The mixture was filtered and concentrated under reduced pressure. The crude product was purified by preparative HPLC (Xtimate C18, 150 mm × 40 mm, 10 μm; mobile phase A: water with ammonium bicarbonate; mobile phase B: acetonitrile, 44%-84% over 25 minutes) to afford Compound **1.**

MS-ESI [M+H]$^+$, calculated 598, found 598.

$^1$H NMR (400 MHz, MeOD) δ 7.94-8.01 (m, 1H), 7.41-7.53 (m, 1H), 7.31 (td, J = 8.4, 2.4 Hz, 1H), 7.19 (dd, J = 8.4, 2.8 Hz, 1H), 7.04-7.12 (m, 1H), 5.43-5.58 (m, 1H), 3.68-3.85 (m, 3H), 3.46-3.60 (m, 5H), 3.23 (br dd, J = 14.0, 7.2 Hz, 1H), 3.08-3.19 (m, 1H), 2.92-3.03 (m, 1H), 2.73-2.85 (m, 2H), 2.49-2.64 (m, 3H), 2.39-2.48 (m, 2H), 2.28 (s, 3H), 2.07-2.23 (m, 2H), 1.80-2.01 (m, 2H), 1.50-1.69 (m, 2H), 1.37-1.48 (m, 2H), 1.20-1.32 (m, 2H), 1.11-1.19 (m, 5H), 1.05 (br t, J = 7.2 Hz, 1H), 0.94 (br t, J = 7.6 Hz, 9H).

## Examples 2 and 3

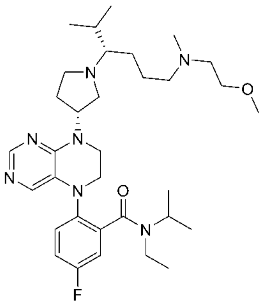

2

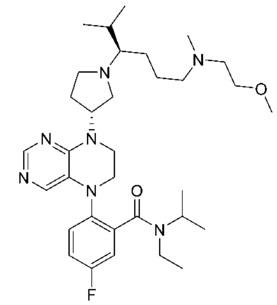

3

The synthetic route for Compounds **2** and **3** is illustrated below:

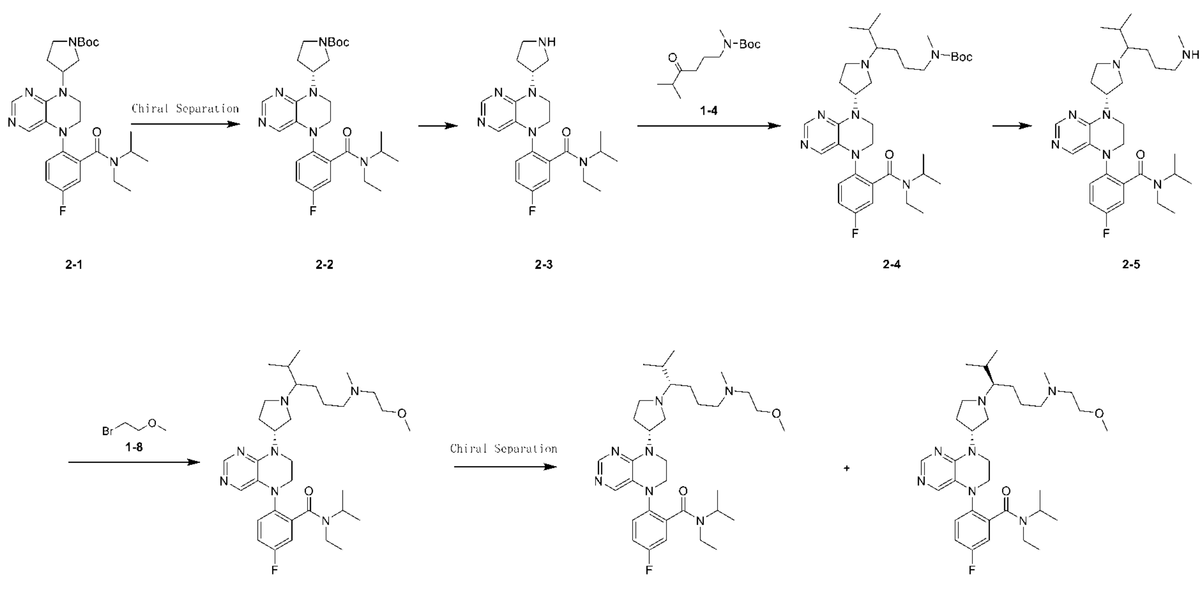


2-1 2-2 2-3 2-4 2-5

2-6 2 + 3

(1) Compound **2-1** (21.0 g; synthesis described in patent WO2023078426) was resolved by chiral supercritical fluid chromatography (SFC) to afford Compound **2-2.** Separation conditions: column type: Chiralpak AD; column specifications: 50 × 4.6 mm I.D., 3 μm; injection volume: 10 μL; mobile phase: A: carbon dioxide, B: isopropanol (0.05% diethylamine), B%: 5%-40% gradient elution over 1.5 minutes, 40% isocratic elution for 1 minute, 5% isocratic elution for 0.5 minutes; detection wavelength: 254 nm; column temperature: 35°C. Retention time: 1.185 minutes, ee value: 98.56%. MS-ESI $[M+H]^+$, calculated 513, found 513.

(2) To a solution of Compound **2-2** (10.0 g, 19.5 mmol) in dichloromethane (100 mL) was added hydrogen chloride in dioxane (30.0 mL). The reaction mixture was stirred at 25°C for 24 hours. The mixture was concentrated under reduced pressure to afford the crude hydrochloride salt of Compound **2-3.** MS-ESI $[M+H]^+$, calculated 413, found 413.

(3) To a solution of the hydrochloride salt of Compound **2-3** (4.30 g, 9.58 mmol) in methanol (80.0 mL) was added triethylamine to adjust the pH to 8. Subsequently, Compound **1-4** (2.80 g, 11.4 mmol) and zinc chloride (5.22 g, 38.3 mmol, 1.80 mL) were added. The reaction mixture was stirred at 80°C under nitrogen atmosphere for 2 hours. Sodium cyanoborohydride (2.41 g, 38.31 mmol) was then added, and stirred for 72 hours. The reaction was quenched with water (50.0 mL) and extracted with ethyl acetate (300 mL × 1). The combined organic phases were washed with water (150 mL × 2) and saturated brine (150 mL × 2), dried over anhydrous sodium sulfate, filtered, and concentrated under reduced pressure. The crude product was purified by silica gel column chromatography (dichloromethane/methanol = 1:0 to 10:1) to afford Compound **2-4.**

MS-ESI $[M+H]^+$, calculated 640, found 640.

(4) To a solution of Compound **2-4** (2.00 g, 3.13 mmol) in dichloromethane (20.0 mL) was added hydrogen chloride in dioxane (4 mol/L, 10.0 mL). The reaction mixture was stirred at 25°C under nitrogen atmosphere for 1 hour. The mixture was filtered and concentrated under reduced pressure to afford the crude hydrochloride salt of Compound 2-5.

MS-ESI $[M+H]^+$, calculated 540, found 540.

(5) To a solution of Compound **2-5** (2.40 g, 4.45 mmol) in N,N-dimethylformamide (10.0 mL) at 0°C was added potassium carbonate (3.07 g, 22.2 mmol). Subsequently, Compound **1-8** (1.82 g, 9.78 mmol) was added. The reaction mixture was stirred at 50°C under nitrogen atmosphere for 5 hours. The mixture was filtered and concentrated under reduced pressure. The crude product was purified by preparative HPLC (Xtimate C18, 150 mm × 40 mm, 10 μm; mobile phase A: water with ammonium bicarbonate; mobile phase B: acetonitrile, 44%-84% over 25 minutes) to afford Compound **2-6.**

MS-ESI $[M+H]^+$, calculated 598, found 598.

(6) Compound **2-6** (0.45 g) was resolved by chiral supercritical fluid chromatography (SFC) to afford Compounds **2** and **3.** Separation conditions: column type: Chiralpak IG; column specifications: 50 × 4.6 mm I.D., 3 μm; injection volume: 10 μL; mobile phase: A: carbon dioxide, B: ethanol (0.05% diethylamine), B%: 20%-40% gradient elution over 1.5 minutes, 40% isocratic elution for 1 minute, 20% isocratic elution for 0.5 minutes; detection wavelength: 254 nm; column temperature: 35°C.

Compound **2:** Retention time: 1.547 minutes, ee value: 99.58%.

MS-ESI $[M+H]^+$, calculated 598, found 598.

$^1$H NMR (400 MHz, MeOD) δ 8.14 (s, 1H), 7.48-7.57 (m, 1H), 7.35 (td, J = 8.4, 2.8 Hz, 1H), 7.12-7.25 (m, 2H), 3.77-4.01 (m, 5H), 3.75 (t, J = 4.8 Hz, 3H), 3.67 (br d, J = 13.2 Hz, 2H), 3.46-3.51 (m, 1H), 3.42 (s, 3H), 3.38 (br s, 2H), 3.25-3.29 (m, 2H), 3.08-3.21 (m, 1H), 2.93 (s, 3H), 2.40-2.85 (m, 2H), 2.17-2.38 (m, 2H), 1.91-2.10 (m, 2H), 1.76-1.90 (m, 3H), 1.18-1.38 (m, 3H), 1.17 (br s, 3H), 1.00-1.15 (m, 9H), 0.98 (br s, 1H).

Compound **3:** Retention time: 1.708 minutes, ee value: 98.44%.

MS-ESI $[M+H]^+$, calculated 598, found 598.

$^1$H NMR (400 MHz, MeOD) δ 7.99 (s, 1H), 7.48-7.57 (m, 1H), 7.30-7.35 (m, 1H), 7.10-7.22 (m, 2H), 3.62-3.89 (m, 3H), 3.59 (br t, J = 5.2 Hz, 2H), 3.40-3.56 (m, 2H), 3.36 (s, 3H), 3.10-3.28 (m, 2H), 2.92-3.02 (m, 1H), 2.88 (br s, 2H), 2.59-2.80 (m, 3H), 2.50 (br s, 3H), 2.27 (br d, J = 3.9 Hz, 2H), 1.97-2.12 (m, 3H), 1.85-1.95 (m, 1H), 1.57-1.76 (m, 2H), 1.42-1.57 (m, 2H), 1.28-1.35 (m, 1H), 1.10-1.27 (m, 6H), 1.06 (br t, J = 6.8 Hz, 1H), 0.85-1.02 (m, 8H).

## Examples 4 and 5

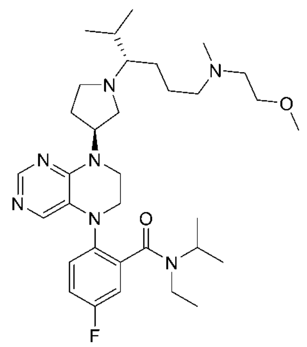

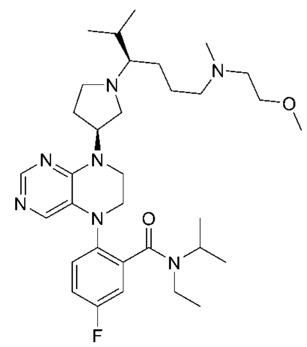

4 or 5

The synthetic route for Compounds **4** and **5** is illustrated below:

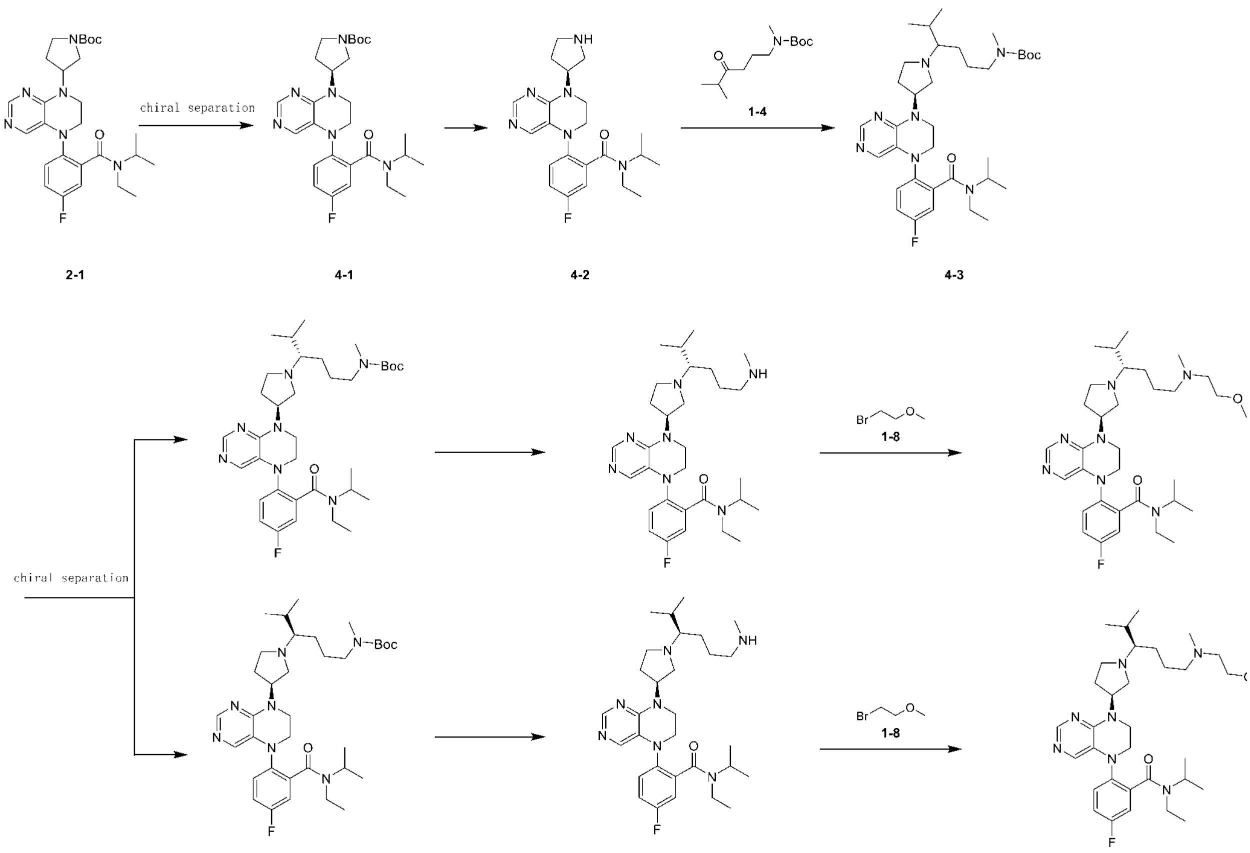


2-1 4-1 4-2 4-3

4-4 or 5-4 4-5 or 5-5 4 or 5

(1) Compound **2-1** (21.0 g; synthesis described in patent WO2023078426) was resolved by chiral supercritical fluid chromatography (SFC) to afford Compound **4-1.** Separation conditions: column type: Chiralpak AD; column specifications: 50 × 4.6 mm I.D., 3 μm; injection volume: 10 μL; mobile phase: A: carbon dioxide, B: isopropanol (0.05% diethylamine), B%: 5%-40% gradient elution over 1.5 minutes, 40% isocratic elution for 1 minute, 5% isocratic elution for 0.5 minutes; detection wavelength: 254 nm; column temperature: 35°C. Retention time: 1.411 minutes, ee value: 94.16%.

MS-ESI $[M+H]^+$, calculated 513, found 513.

(2) To a solution of Compound **4-1** (10.6 g, 20.6 mmol) in dichloromethane (100 mL) was added hydrogen chloride in dioxane (30.0 mL). The reaction mixture was stirred at 25°C for 24 hours. The mixture was concentrated under reduced pressure to afford the crude hydrochloride salt of Compound **4-2.**

MS-ESI $[M+H]^+$, calculated 413, found 413.

(3) To a solution of the hydrochloride salt of Compound **4-2** (10.0 g, 22.2 mmol) in methanol (80.0 mL) was added

triethylamine to adjust the pH to 8. Subsequently, Compound **1-4** (6.50 g, 26.7 mmol) and zinc chloride (12.1 g, 89.1 mmol) were added. The reaction mixture was stirred at 80°C under nitrogen atmosphere for 2 hours. Sodium cyanoborohydride (5.60 g, 89.1 mmol) was then added, and stirred for 72 hours. The reaction was quenched with water (50.0 mL) and extracted with ethyl acetate (300 mL × 1). The combined organic phases were washed with water (150 mL × 2) and saturated brine (150 mL × 2), dried over anhydrous sodium sulfate, filtered, and concentrated under reduced pressure. The crude product was purified by silica gel column chromatography (dichloromethane/methanol = 1:0 to 10:1) to afford Compound **4-3.**

MS-ESI $[M+H]^+$, calculated 640, found 640.

(4) Compound **4-3** (13.0 g) was resolved by chiral supercritical fluid chromatography (SFC) to afford Compounds **4-4** and **5-4.** Separation conditions: column type: Chiralpak AD-3; column specifications: 50 × 4.6 mm I.D., 3 μm; injection volume: 10 μL; mobile phase: A: carbon dioxide, B: isopropanol (0.05% diethylamine), B%: 5%-40% gradient elution over 1.5 minutes, 40% isocratic elution for 1 minute, 5% isocratic elution for 0.5 minutes; detection wavelength: 254 nm; column temperature: 35°C.

Compound **4-4:** Retention time: 1.034 minutes, ee value: 95.00%. MS-ESI $[M+H]^+$, calculated 640, found 640.

Compound **5-4:** Retention time: 1.105 minutes, ee value: 87.90%. MS-ESI $[M+H]^+$, calculated 640, found 640.

(5) To a solution of Compound **4-4** (4.50 g, 7.03 mmol) in dichloromethane (20.0 mL) was added hydrogen chloride in dioxane (4 mol/L, 10.0 mL). The reaction mixture was stirred at 25°C under nitrogen atmosphere for 1 hour. The mixture was filtered and concentrated under reduced pressure to afford the crude hydrochloride salt of Compound **4-5.**

MS-ESI $[M+H]^+$, calculated 540, found 540.

(6) To a solution of Compound **4-5** (3.00 g, 5.21 mmol) in N,N-dimethylformamide (35.0 mL) at 0°C was added potassium carbonate (3.60 g, 26.0 mmol). Subsequently, Compound 1-8 (1.94 g, 10.4 mmol) was added. The reaction mixture was stirred at 50°C under nitrogen atmosphere for 5 hours. The mixture was filtered and concentrated under reduced pressure. The crude product was purified by preparative HPLC (Xtimate C18, 200 mm × 40 mm, 10 μm; mobile phase A: water with formic acid; mobile phase B: acetonitrile, 0%-24% over 20 minutes) to afford Compound **4.**

MS-ESI $[M+H]^+$, calculated 598, found 598.

$^1H$ NMR (400 MHz, MeOD) δ 8.45 (br d, J = 9.2 Hz, 1H), 7.54-7.68 (m, 1H), 7.22-7.47 (m, 2H), 6.93-7.17 (m, 1H), 5.45-5.70 (m, 1H), 3.60-4.09 (m, 12H), 3.40-3.53 (m, 7H), 3.12-3.24 (m, 2H), 2.94 (d, J = 4.0 Hz, 3H), 2.47-2.59 (m, 2H), 2.24-2.40 (m, 1H), 1.98-2.15 (m, 1H), 1.78-1.96 (m, 3H), 1.07-1.27 (m, 15H).

(7) Using Compound **5-4** as the starting material, Compound **5** was obtained following the same experimental procedures as described in steps (5) and (6): MS-ESI $[M+H]^+$, calculated 598, found 598. $^1H$ NMR (400 MHz, MeOD) δ 8.45 (d, J = 14.4 Hz, 1H), 7.52-7.69 (m, 1H), 7.34-7.46 (m, 1H), 7.28 (dd, J = 8.0, 2.8 Hz, 1H), 6.85-7.23 (m, 1H), 5.24-5.76 (m, 1H), 3.92-4.25 (m, 4H), 3.55-3.92 (m, 7H), 3.33-3.55 (m, 8H), 3.09-3.28 (m, 2H), 2.90-2.98 (m, 3H), 2.47-2.66 (m, 2H), 2.28-2.44 (m, 1H), 1.78-2.13 (m, 4H), 1.06-1.31 (m, 15H).

**Test Example 1**

**Determination of Antiproliferative Activity of Compounds Against MV-4-11 Cells (CTG Method):**

1. Experimental Principle: MV-4-11 is a human leukemia cell line harboring MLL translocation and expressing the MLL fusion protein MLL-AF4. The compounds of the present invention inhibit the proliferation of MV-4-11 cells by disrupting the menin/MLL protein-protein interaction.
2. Experimental Materials: CellTiter-Glo was purchased from Promega (Cat. #G7571); IMDM medium was purchased from Gibco (Cat. #12440061); fetal bovine serum was purchased from Excell (Cat. #FND500); dimethyl sulfoxide (DMSO) was purchased from Sigma (Cat. #D2650); 384-well cell culture plates were purchased from Corning (Cat. #3756); automated cell counter was obtained from Life Technologies (Model: Countess II); microplate reader was obtained from PerkinElmer (Model: EnVision Multilabel Reader).
3. Experimental Methods: Cells in logarithmic growth phase were resuspended in growth medium (IMDM + 10% FBS) and diluted to the target density (50,000/mL). The cell suspension was seeded into 384-well plates at 50 μL per well and incubated overnight at 37°C in a 5% $CO_2$ incubator.

Test compounds were dissolved in DMSO to prepare 10 mM stock solutions. The stock solutions were first diluted to 2 mmol/L with DMSO, followed by 3-fold serial dilutions to generate 10 concentrations. From each concentration, 5.5 μL was diluted with 94.5 μL growth medium. The diluted solutions were then added to the 384-well plates containing seeded cells at 5 μL per well.

Cells treated with test compounds were incubated at 37°C in a 5% $CO_2$ incubator for 72 hours. The 384-well

plates were equilibrated at room temperature, and 15 μL of CellTiter-Glo reagent was added to each well. The plates were mixed on a vortex mixer for 2 minutes and incubated at room temperature for 60 minutes. Luminescence values were read using the EnVision Multilabel Reader, and $IC_{50}$ values were calculated using GraphPad Prism 5.0 software.

4. Experimental Data:

The specific test results are shown in Table 1:

**Table 1**

| Test Compound | MV-4-11 $IC_{50}$ (nM) |
|---|---|
| Example **1** | 61.42 |
| Example **3** | 22.89 |
| Comparative Compound **1** | 293.1 |
| Comparative Compound **2** | 800 |
| Comparative Compound **3** | 663.08 |
| Comparative Compound **4** | 289.85 |
| Comparative Compound **5** | 114.41 |
| Comparative Compound **6** | 138.35 |

Comparative Compound **1** is from Example **3** of patent WO2023078426, with the structure:

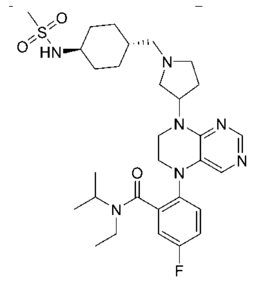

Comparative Compound **2** is from Example 4 of patent WO2023078426, with the structure:

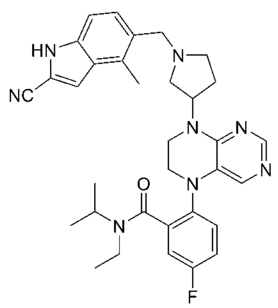

Comparative Compound **3** is from Example 28 of patent WO2023078426, with the structure:

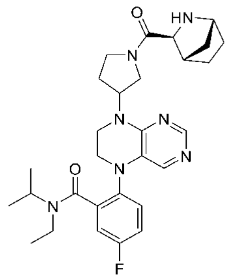

Comparative Compound **4** is from Example **29** of patent WO2023078426, with the structure:

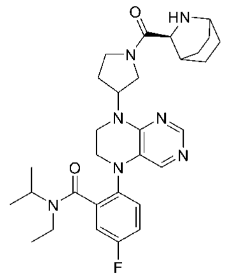

Comparative Compound **5** is from Example **39** of patent WO2023078426, with the structure:

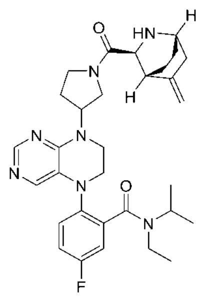

Comparative Compound **6** is from Example **40** of patent WO2023078426, with the structure:

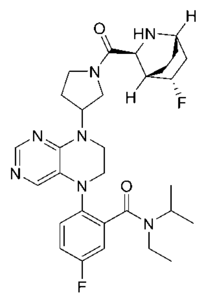

As demonstrated by the test data in Table 1, the compounds of Formula I according to the present invention exhibit superior inhibitory activity against the growth of human myeloid monocytic leukemia MV-4-11 cells compared to previously reported compounds, demonstrating potential for use in the preparation of medicaments for the treatment and prevention of leukemia.

**Test Example 2**

**Antiproliferative Activity of Compounds Against MV-4-11, Molm-13, OCI-AML-3, and HL-60 Cells:**

**[0176]** [0134]
1. Experimental Principle: MV-4-11, Molm-13, and OCI-AML-3 cells are human hematologic tumor cell lines harboring the

Menin-MLL fusion. HL-60 is a wild-type human hematologic tumor cell line without Menin-MLL fusion. The compounds of the present invention inhibit the proliferation of MV-4-11, Molm-13, and OCI-AML-3 cells through non-covalent binding to Menin, while exhibiting minimal inhibitory effect on HL-60 proliferation.

2. Experimental Materials: MV-4-11, Molm-13, OCI-AML-3, and HL-60 cells were all purchased from Cobioer; CellTiter-Glo® was purchased from Promega (Cat. #G7571); RPMI-1640 was purchased from Viva Cell (Cat. #C3010-0500); IMDM was purchased from Viva Cell (Cat. #C3040-0500); fetal bovine serum (FBS) was purchased from Biosera (Cat. #FB-1058/500); penicillin-streptomycin was purchased from BasalMedia (Cat. #S110JV); 0.25% Trypsin-EDTA was purchased from BasalMedia (Cat. #S310KJ); dimethyl sulfoxide (DMSO) was purchased from Sinopharm (Cat. #30072418); 96-well plates were purchased from Corning (Cat. #3610); incubator was obtained from PHCbi (Model: MCO-18AC); inverted microscope was obtained from Mingmei (Cat. #MI52-0); automated cell counter was obtained from BodBoge (Model: JSY-SC-031N); microplate reader was obtained from PerkinElmer (Model: Envision); data processing software: GraphPad Prism 8.0.

3. Experimental Methods: Four cell lines in logarithmic growth phase were resuspended in their respective growth media (MV-4-11: IMDM + 20% FBS; Molm-13: RPMI-1640 + 20% FBS; OCI-AML-3: RPMI-1640 + 20% FBS; HL-60: RPMI-1640 + 10% FBS) and diluted to target densities (12,500, 6,250, 12,500, and 12,500/mL, respectively). The cell suspensions were seeded into 96-well plates at 80 μL per well and incubated overnight at 37°C in a 5% $CO_2$ incubator. Culture medium served as the background control. Test compounds were dissolved in DMSO to prepare 10 mmol/L stock solutions. The stock solutions were first diluted to 2 mmol/L with DMSO, followed by 4-fold serial dilutions to generate 10 concentrations. From each concentration, 2 μL was diluted with 78 μL growth medium. The diluted solutions were then added to the 96-well plates containing seeded cells at 20 μL per well. Cells treated with test compounds were incubated at 37°C in a 5% $CO_2$ incubator for 7 days. The 96-well plates were equilibrated at room temperature, and 25 μL of CellTiter-Glo reagent was added to each well. After incubation at room temperature for 10 minutes, luminescence values were read using the EnVision microplate reader, and $IC_{50}$ values were calculated using GraphPad Prism 8.0 software.

4. Experimental Data:

The specific test results are shown in Table 2:

**Table 2**

| Test Compound | MV 4-11 $IC_{50}$(nM) | MOLM13 $IC_{50}$(nM) | OCI-AML3 $IC_{50}$(nM) | HL-60 $IC_{50}$(nM) |
|---|---|---|---|---|
| Example 3 | 10.13 | 26.70 | 103.47 | >10000 |
| SNDX-5613 | 14.45 | - | - | >10000 |
| KO539 | 14.38 | - | - | 2048.63 |

**[0177]** [0135] As demonstrated by the test data in Table 2, the compounds of Formula I according to the present invention exhibit superior inhibitory activity against the growth of leukemia cells while having minimal impact on normal cell lines.

Structure of SNDX-5613:

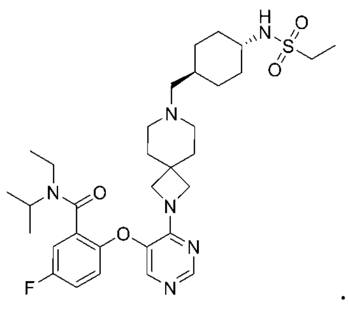

.

Structure of KO539:

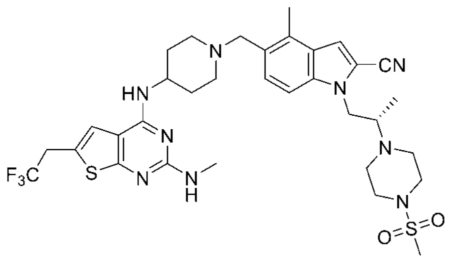
.

**Test Example 3**

**Inhibitory Effect of Compounds on hERG Ion Channel:**

1. Objective: To evaluate the inhibitory effect of test compounds on hERG current in HEK293 cells stably transfected with the hERG potassium channel using manual patch-clamp technique.
2. Experimental Materials: The test compound was Compound 3 prepared according to the examples of the present invention; the positive control compound SNDX-5613 was purchased from Chengdu Zhihui Zhongxin Biomedical Co., Ltd.; HEK293 cells were obtained from Procell (Cat. #CL-0001). Manual patch-clamp system amplifier was obtained from AXON (Model: Axon MultiClamp 700B); manual patch-clamp system digitizer was obtained from Axon (Model: Axon Digidata 1550B); motorized micromanipulator was obtained from Sutter Instruments (Model: MP-225); peristaltic pump was obtained from Longer Precision Pump Co., Ltd., Baoding, China (Model: BT100-2J); micropipette puller was obtained from NARISHIGE (Model: PC-10).
3. Experimental Methods:
Preparation of Electrophysiological Solutions: Extracellular solution: 140 mM NaCl, 3.5 mM KCl, 1 mM $MgCl_2 \cdot 6H_2O$, 2 mM $CaCl_2 \cdot 2H_2O$, 10 mM D-Glucose, 10 mM HEPES, 1.25 mM $NaH_2PO_4 \cdot 2H_2O$, pH adjusted to 7.4 with NaOH; osmolality adjusted to 290-310 mOsm; filtered and stored at 4°C.

Intracellular solution (mM): 20 mM KCl, 115 mM K-Aspartic, 1 mM $MgCl_2 \cdot 6H_2O$, 5 mM EGTA, 10 mM HEPES, 2 mM $Na_2$-ATP, pH adjusted to 7.2 with KOH; osmolality adjusted to 290-310 mOsm; filtered, aliquoted, and stored at -20°C.

Cell Preparation: HEK-293-hERG cells were subcultured to an appropriate state, washed with phosphate-buffered saline, dissociated using TrypLE solution, resuspended in culture medium, transferred to centrifuge tubes, centrifuged, and the supernatant was discarded. Cells were resuspended in extracellular solution for use and stored at 2-8°C. The cell suspension was plated onto coverslips and cultured in 24-well plates or 6 cm cell culture dishes. After 18 hours, coverslips were removed for patch-clamp recording. Prior to patch-clamp recording, the cell suspension was added to a recording dish to ensure adequate cell density with cells in a single, dispersed state.

Electrophysiological Experiments: Whole-cell patch-clamp technique was employed to record hERG currents. Cell suspension was added to a small recording dish and placed on an inverted microscope stage. After cells adhered, extracellular solution was perfused at a recommended flow rate of 1-2 mL/min. Glass micropipettes were fabricated using a micropipette puller in two steps, and when filled with intracellular solution, exhibited a resistance of 2-5 MΩ upon immersion in solution.

Following establishment of the whole-cell recording configuration, the holding potential was maintained at -80 mV. The voltage protocol consisted of depolarization from -80 mV to -50 mV for 0.5 s (serving as leak current detection), followed by a step to +30 mV for 2.5 s, then rapid repolarization to -50 mV for 4 s to elicit hERG tail currents. Data were acquired every 16 s to observe the effect of compounds on hERG tail currents. The 0.5 s step to -50 mV served as leak current detection. Solution exchange was performed using a peristaltic pump perfusion system. Once the recorded hERG current stabilized, compounds were administered via continuous extracellular perfusion from low to high concentrations (0.3, 1, 3, 10, and 30 μmol/L). Following administration of each compound concentration, cells were allowed to equilibrate for at least 5 minutes (or until current stabilization) before proceeding to the next concentration. Each concentration was tested on at least three cells with three independent replicates. All electrophysiological experiments were conducted at room temperature.

Data Acquisition and Analysis: Experimental data were acquired using Clampex software and analyzed using Clampfit software. First, the current following each compound concentration was normalized to the control current $\left(\frac{\text{Peak tail current（compound）}}{\text{Peak tail current（blank）}}\right)\times 100$ , then the inhibition rate for each compound concentration was calculated

$(1-\frac{\text{Peak tail current (compound)}}{\text{Peak tail current (blank)}})\times 100$ . The mean and standard error were calculated for each concentration, and the half-maximal inhibitory concentration for each compound was calculated using the following equation:

Y = Bottom + (Top-Bottom)/(1+10^((Log$IC_{50}$-X)×HillSlope))

The dose-response relationship was fitted using this nonlinear regression equation, where $IC_{50}$ represents the half-maximal inhibitory concentration. Curve fitting and $IC_{50}$ calculations were performed using GraphPad Prism software. If the maximum inhibition rate at all tested concentrations was less than 50%, the $IC_{50}$ value was not calculated.

Test Results: The $IC_{50}$ of Compound 3 was >30 μM, while the $IC_{50}$ of the positive control compound SNDX-5613 was 7.59 μM, indicating that Compound 3 exhibits minimal inhibitory effect on the hERG channel and demonstrates superior cardiac safety compared to SNDX-5613.

**Test Example 4**

**In Vivo Efficacy Study of MV-4-11 Xenograft Model**

1. Objective: To evaluate the in vivo efficacy of test compounds in a subcutaneous MV-4-11 tumor xenograft model in female NOD/SCID mice.
2. Experimental Materials: The test compound was Compound 3 prepared according to the examples of the present invention; the positive control compound SNDX-5613 was purchased from Chengdu Zhihui Zhongxin Biomedical Co., Ltd.; MV-4-11 cells were purchased from Nanjing Cobioer; NOD/SCID mice were purchased from Beijing Vital River Laboratory Animal Technology Co., Ltd.
3. Experimental Methods: Female NOD/SCID mice, 6-8 weeks of age, weighing 16-22 g, were used. MV-4-11 cells were cultured in IMDM medium supplemented with 20% fetal bovine serum. Cells in exponential growth phase were harvested, resuspended in phosphate-buffered saline, and prepared as a cell suspension at a density of $1 \times 10^7$ cells/0.1 mL. Cells were subcutaneously inoculated into the right dorsal neck region of each mouse ($1 \times 10^7$ cells/mouse) with 50% Matrigel. When the mean tumor volume reached approximately 50-100 $mm^3$, mice were randomized and treatment was initiated by oral gavage administration once daily. Tumor diameters were measured twice weekly using digital calipers. Tumor volume was calculated using the formula: $V = 0.5 \times a \times b^2$, where a and b represent the longest and shortest tumor diameters, respectively.

Experimental results are shown in Figure 1. The results demonstrate that Compound 3 of the present invention exhibits superior efficacy compared to the positive control SNDX-5613 in the MV-4-11 subcutaneous tumor xenograft model.

**Test Example 5**

**Pharmacokinetic Characterization of Compounds in Rats**

1. Objective: This study aimed to characterize the pharmacokinetic properties of selected compounds from the examples of the present invention in SD rats following single intravenous (IV) injection and oral (PO) administration using LC-MS/MS methodology.
2. Experimental Materials: Test compounds were prepared according to the examples of the present invention; male SD rats were obtained from the Laboratory Animal Department of Shanghai Institute of Planned Parenthood Research (License No. SCXK(Shanghai)2018-0006) or Shanghai Jihui Laboratory Animal Breeding Co., Ltd. (License No. SCXK(Shanghai)2022-0009).
3. Experimental Methods:
Preparation of 0.5% methylcellulose: 5 g of methylcellulose was weighed and dissolved in 1000 mL of purified water.
Preparation of 0.5% methylcellulose (pH = 5.03): 5 g of methylcellulose was weighed and dissolved in 1000 mL of purified water, and the pH was adjusted to 5.03 with 100 mM citric acid.

Preparation of oral formulations: The required amount of test compound was weighed and dissolved in 0.5% methylcellulose solution or 0.5% methylcellulose (pH = 5.03) to obtain a suspension or solution at 1 or 3 mg/mL.

Preparation of intravenous injection formulations: The required amount of test compound (EB229, EB300,

EB341, EB342) was weighed and dissolved in 0.5 mL dimethyl sulfoxide to prepare a 4 mg/mL solution. To 0.25 mL of this solution, 0.5 mL of polyethylene glycol 15-hydroxystearate (Solutol) and 4.25 mL of normal saline were added to obtain a 0.2 mg/mL solution. For EB834, 6 mg was weighed and dissolved in 0.1 mL dimethyl sulfoxide, followed by addition of 0.2 mL polyethylene glycol 15-hydroxystearate (Solutol) and 1.7 mL normal saline to obtain a 3 mg/mL solution.

Dosing: Male SD rats were fasted overnight prior to oral (PO) administration; fasting was not required for the single intravenous (IV) injection group. The specific study design is shown in Table 3. Food was provided 4 hours post-dosing.

**Table 3**

| Compound | Route | Vehicle | n | Dose (mg/kg) | Conc. (mg/mL) | Vol. (mL/kg) | Sampling Time Points (h) |
|---|---|---|---|---|---|---|---|
| Comparative Comp. 1 | IV | 5% DMSO/10% Solutol/85% Saline | 3 | 1 | 0.2 | 5 | 0.083, 0.5, 1, 2, 4, 8, 24 |
| | PO | 0.5% MC | 3 | 10 | 1 | 10 | 0.5, 1, 2, 4, 6, 8, 24 |
| Comparative Comp. 2 | IV | 5% DMSO/10% Solutol/85% Saline | 3 | 1 | 0.2 | 5 | 0.083, 0.25, 0.5, 1, 2, 4, 8, 24 |
| | PO | 0.5% MC | 3 | 10 | 1 | 10 | 0.25, 0.5, 1, 2, 4, 6, 8, 24 |
| Comparative Comp. 3 | IV | 5% DMSO/10% Solutol/85% Saline | 3 | 1 | 0.2 | 5 | 0.083, 0.25, 0.5, 1, 2, 4, 8, 24 |
| | PO | 0.5% MC | 3 | 10 | 1 | 10 | 0.25, 0.5, 1, 2, 4, 6, 8, 24 |
| Comparative Comp. 4 | IV | 5% DMSO/10% Solutol/85% Saline | 3 | 1 | 0.2 | 5 | 0.083, 0.25, 0.5, 1, 2, 4, 8, 24 |
| | PO | 0.5% MC | 3 | 10 | 1 | 10 | 0.25, 0.5, 1, 2, 4, 6, 8, 24 |
| Example 3 | IV | 5% DMSO/10% Solutol/85% Saline | 3 | 3 | 3 | 1 | 0.083, 0.25, 0.5, 1, 2, 4, 8, 24, 32, 48 |
| | PO | 0.5% MC (pH 5.03) | 3 | 30 | 3 | 10 | 0.25, 0.5, 1, 2, 4, 8, 24, 32, 48 |

Sample Collection: Blood was collected from the jugular vein or other suitable veins, with 0.2 mL collected at each time point. Samples were placed in tubes containing dipotassium ethylenediaminetetraacetate and kept on ice prior to centrifugation. Within 1 hour of blood collection, samples were centrifuged at 6800 × g for 6 minutes at 2-8°C and stored at -80°C. Blood sampling time points for intravenous and oral administration are shown in the table above. The specific test results are shown in Table 4.

**Table 4**

| Compound | IV | | | | | PO | | | |
|---|---|---|---|---|---|---|---|---|---|
| | $t_{1/2}$ h | Cmax ng/mL | $AUC_{(0-t)}$ h*ng/mL | Vss mL/kg | CL mL/min/kg | $t_{1/2}$ h | $C_{max}$ ng/mL | $AUC_{(0-t)}$ h*ng/mL | F % |
| Comparative Comp. 7 | 1.55 | 152 | 264 | 6622 | 62.1 | 2.29 | 27.0 | 119 | 3.7 |
| Comparative Comp. 8 | 2.06 | 271.45 | 85.72 | 9610 | 184.89 | 1.80 | 41.87 | 118.70 | 13.85 |
| Comparative Comp. 9 | 5.98 | 376.18 | 169.12 | 30640 | 92.41 | 5.12 | 101.15 | 228.28 | 13.50 |
| Comparative Comp. 10 | 0.82 | 16.24 | 16.87 | 59400 | 947.19 | 1.26 | 33.29 | 83.79 | 49.68 |

(continued)

| Compound | IV | | | | | PO | | | |
|---|---|---|---|---|---|---|---|---|---|
| | $t_{1/2}$ h | Cmax ng/mL | $AUC_{(0-t)}$ h*ng/mL | Vss mL/kg | CL mL/min/kg | $t_{1/2}$ h | $C_{max}$ ng/mL | $AUC_{(0-t)}$ h*ng/mL | F % |
| Example 3 | 52.9 | 193.33 | 494 | 202858 | 59.2 | 21.3 | 1135 | 8497 | 172 |

Comparative Compound 7 is from Example 9 of patent WO2023078426, with the structure:

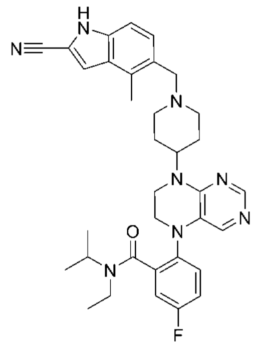

Comparative Compound 8 is from Example 11 of patent WO2023078426, with the structure:

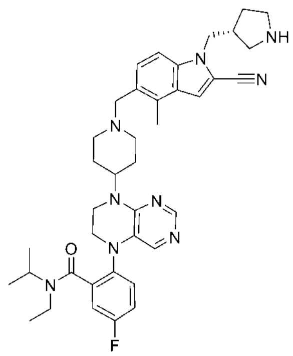

Comparative Compound 9 is from Example 12 of patent WO2023078426, with the structure:

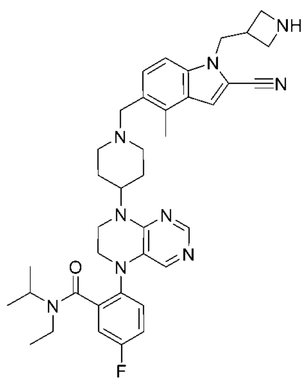

Comparative Compound 10 is from Example 13 of patent WO2023078426, with the structure:

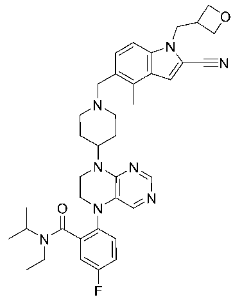

The data in Table 4 demonstrate that selected compounds from the examples of the present invention possess excellent pharmacokinetic properties in rats, superior to previously reported compounds.

**Test Example 6**

**Pharmacokinetic Study in Mice**

1. Objective: This study aimed to characterize the pharmacokinetic properties of selected compounds from the examples of the present invention in male ICR mice following single intravenous (IV) injection and oral (PO) administration using LC-MS/MS methodology.
2. Experimental Materials: Test compounds were prepared according to the examples of the present invention; male ICR mice were obtained from Shanghai Jihui Laboratory Animal Breeding Co., Ltd. (License No. SCXK(Shanghai) 2022-0009).
3. Experimental Methods:
Preparation of 0.5% methylcellulose (pH = 4.91): 5 g of methylcellulose was weighed and dissolved in 1000 mL of purified water, and the pH was adjusted to 4.91 with 100 mM citric acid.

Preparation of oral formulations: The required amount of test compound was weighed and dissolved in 0.5% methylcellulose (pH = 4.91) solution to obtain a suspension or solution at 3 mg/mL.
Preparation of intravenous injection formulations: The required amount of test compound was weighed and dissolved in 0.5 mL dimethyl sulfoxide to prepare a 12 mg/mL solution. To 0.25 mL of this solution, 0.5 mL of polyethylene glycol 15-hydroxystearate (Solutol) and 4.25 mL of normal saline were added to obtain a 0.6 mg/mL solution.
Dosing: Male ICR mice were fasted overnight prior to oral (PO) administration; fasting was not required for the single intravenous (IV) injection group. The dosing regimen and administration volumes are shown in Table 5. Food was provided 4 hours post-dosing.

**Table 5**

| Route | Number of Animals | Dose | Concentration | Volume |
|---|---|---|---|---|
| | | (mg/kg) | (mg/mL) | (mL/kg) |
| IV | 3 | 3 | 0.6 | 5 |
| PO | 3 | 30 | 3 | 10 |

Sample Collection: Blood was collected from the jugular vein or other suitable veins, with 0.03 mL collected at each time point. Samples were placed in tubes containing dipotassium ethylenediaminetetraacetate and kept on ice prior to centrifugation. Within 1 hour of blood collection, samples were centrifuged at 6800 × g for 6 minutes at 2-8°C and stored at -80°C. Blood sampling time points for intravenous administration were 0.083, 0.25, 0.5, 1, 2, 4, 8, 24, and 32 hours post-dosing. Blood sampling time points for oral administration were 0.25, 0.5, 1, 2, 4, 8, 24, and 32 hours post-dosing.
The specific test results are shown in Table 6.

**Table 6**

| Compound | IV | | | | | PO | | | |
|---|---|---|---|---|---|---|---|---|---|
| | $t_{1/2}$ h | Cmax ng/mL | $AUC_{(0-t)}$ h*ng/mL | Vss mL/kg | CL mL/min/kg | $t_{1/2}$ h | $C_{max}$ ng/mL | $AUC_{(0-t)}$ h*ng/mL | F % |
| Example **3** | 10.91 | 418.0 | 428.6 | 65108 | 104 | 6.49 | 293.0 | 1097.2 | 25.6 |

**Test Example 7**

**Inhibitory Activity of Compounds Against Wild-Type Menin Protein and Its Mutant Variants**

1. Objective: To evaluate the inhibitory activity of compounds against wild-type Menin protein (Menin-WT) and its mutant variants (Menin-M327V, Menin-M327I, Menin-T349M, Menin-S160T, Menin-S160C, Menin-G331R, and Menin-G331D).
2. Experimental Principle: The experiment employed fluorescence polarization (FP) to detect the binding of wild-type Menin protein and its mutant variants to MLL peptide. In the absence of inhibitors, binding of wild-type Menin protein and its mutant variants to MLL peptide generates a high FP signal; upon addition of inhibitors, the binding between Menin proteins and MLL peptide is disrupted, resulting in a low FP signal.
3. Experimental Materials: Final concentration of wild-type Menin protein and its mutant variants: 8 nM; final concentration of MLL peptide: 4 nM. Assay buffer: 50 mM Tris-HCl (pH 8.0), 120 mM NaCl, 0.005% CA-630, 2 mM DTT.
4. Experimental Procedure: A 384-well plate was used, and 10 μL of wild-type Menin protein or its mutant variant was added to each assay well (except wells A1-H1 and I24-P24), while 10 μL of assay buffer was added to wells A1-H1 and I24-P24 as the 100% inhibition control group. The plate was centrifuged at 1000 rpm for 60 seconds. Subsequently, 10 μL of MLL peptide was added to each assay well. The plate was centrifuged at 1000 rpm for 60 seconds and sealed with a sealing membrane. After incubation at 23°C for 60 minutes, the plate was read using a PerkinElmer Envision reader.

Data Analysis:

%inhibition = (sample data - 0% inhibition) / (100% inhibition - 0% inhibition) × 100

Curve fitting: XLFIT software was used for curve fitting, employing a four-parameter logistic model with percent inhibition versus log(compound concentration) as variables. The specific test results are shown in Table 7.

**Table 7**

| Menin | **WT** $IC_{50}$(nM) | **M327V** $IC_{50}$(nM) | **M327I** $IC_{50}$(nM) | **T349M** $IC_{50}$(nM) | **G331R** $IC_{50}$(nM) |
|---|---|---|---|---|---|
| Example 3 | 13.0 | 49.3 | 40.7 | 46.7 | 36.7 |
| SNDX-5613 | 17.6 | 1295 | 928 | 271 | 101 |
| KO-539 | 27.3 | >10000 | 6131 | 55.2 | 42.4 |

As demonstrated by the test data in Table 7, the compounds of Formula I according to the present invention exhibit excellent inhibitory activity against both wild-type Menin protein and various mutant subtypes. Notably, the compounds demonstrate superior inhibitory activity against the major mutant subtypes of Menin protein compared to SNDX-5613 and KO539。

**Applicant's Declaration**

The Applicant declares that the present invention illustrates the compounds, pharmaceutical compositions comprising the same, and applications thereof through the above examples, but the present invention is not limited to the above examples, which does not imply that the present invention must rely on the above examples for implementation. Those skilled in the art should understand that any modifications to the present invention, equivalent substitutions of raw

materials of the products of the present invention, additions of auxiliary components, and selection of specific modes all fall within the scope of protection and disclosure of the present invention.

All documents mentioned in the present invention are incorporated herein by reference, as if each document were individually incorporated by reference. Furthermore, it should be understood that after reading the above teachings of the present invention, those skilled in the art may make various changes or modifications to the present invention, and such equivalent forms also fall within the scope defined by the appended claims of this application.

## Claims

1. A compound, or a pharmaceutically acceptable salt, enantiomer, diastereomer, tautomer, cis-trans isomer, solvate, polymorph, deuterated derivative, or combination thereof, **characterized in that** the compound has the structure represented by Formula I:

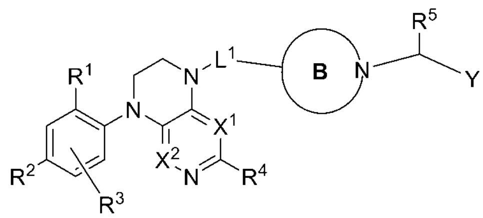

I ;

wherein,

$R^1$ is selected from the group consisting of: -C(O)($NR^aR^b$), phenyl, and 5-6 membered heteroaryl; wherein the phenyl or heteroaryl may be optionally substituted with $R^{1a}$;

$R^a$ and $R^b$ are each independently selected from the group consisting of: H, optionally substituted C1-C6 alkyl, optionally substituted 3-8 membered cycloalkyl, or optionally substituted 4-8 membered heterocyclyl; or Ra and Rb together with the nitrogen atom to which they are attached form an optionally substituted 4-8 membered heterocycle; wherein the heterocycle contains, in addition to the attached nitrogen atom, 0-2 heteroatoms selected from N, O, S, and P; wherein the substitution refers to replacement of one or more H in the group with R;

Each $R^{1a}$ is independently selected from the group consisting of: H, cyano, halogen, C1-C3 alkyl, halo C1-C3 alkyl, C2-C4 alkenyl, C2-C4 alkynyl, C1-C3 alkoxy, halo C1-C3 alkoxy, and C3-C5 cycloalkyl;

$R^2$ is selected from the group consisting of: H, halogen, methyl, and trifluoromethyl;

$R^3$ represents 0, 1, 2, or 3 groups each independently selected from the group consisting of: H, halogen, C1-C3 alkyl, and halo C1-C3 alkyl;

$R^4$ is selected from the group consisting of: H, optionally substituted C1-C6 alkyl, optionally substituted C1-C4 alkoxy, optionally substituted C1-C4 alkylamino, optionally substituted (C1-C4 alkyl)$_2$amino, halogen, $-NH_2$, $-NO_2$, -COOH, -CN, -OH, optionally substituted C1-C6 alkylsulfonyl, optionally substituted C1-C6 alkylsulfinyl, optionally substituted C1-C6 alkylthio, $-NHCOCR^{4a}=CH_2$, $-NHCOCHR^{4a}R^{4b}$, $-SO_2C(R^{4a})=CH_2$, $-NHSO_2CR^{4a}=CH_2$, or - $NHSO_2CHR^{4a}R^{4b}$; wherein each $R^{4a}$ is independently selected from the group consisting of: H, methyl, and fluorine; each $R^{4b}$ is independently a chlorine or bromine atom; wherein the substitution refers to replacement of one or more (e.g., 1, 2, or 3) H in the group with R;

$X^1$ and $X^2$ are each independently selected from N or $CR^X$; wherein $R^X$ is selected from the group consisting of: H, halogen, -CN, -OH, $-NH_2$, optionally substituted C1-C4 alkyl, optionally substituted C1-C4 alkoxy, optionally substituted C1-C4 alkylamino, and optionally substituted (C1-C4 alkyl)$_2$amino; wherein the substitution refers to replacement of one or more H in the group with R;

Ring B is a 4-12 membered saturated or partially saturated nitrogen-containing heterocycle; wherein the nitrogen-containing heterocycle may be optionally substituted with $R^B$, and the nitrogen-containing heterocycle contains at least one N atom and 0-2 heteroatoms selected from N, O, and S;

Each $R^B$ is independently selected from the group consisting of: deuterium, halogen, oxo, C1-C3 alkyl, halo C1-C3 alkyl, or cyano;

$L^1$ is selected from: absent (single bond), $-(CR^{La}R^{Lb})-$, $-(CR^{La}R^{Lb})_2-$ wherein $R^{La}$ and $R^{Lb}$ are each independently selected from the group consisting of: H, deuterium, optionally substituted C1-C4 alkyl, and halogen, or RLa and RLb together with the carbon atom to which they are attached form an optionally substituted 3-8 membered saturated or unsaturated carbocycle, or an optionally substituted 4-8 membered saturated or unsaturated

heterocycle; wherein the heterocycle contains 1-3 heteroatoms selected from N, O, S, and P;
$R^5$ is selected from the group consisting of: optionally substituted C1-C4 alkyl, optionally substituted 3-8 membered saturated or unsaturated carbocycle, optionally substituted 4-8 membered saturated or unsaturated heterocycle; wherein the heterocycle contains 1-3 heteroatoms selected from N, O, S, and P; wherein the substitution refers to substitution with one or more R;
Y is selected from: -C1-C6 alkylene-$NR^{Y1}R^{Y2}$, -3-6 membered cycloalkylene-$NR^{Y1}R^{Y2}$, optionally substituted 4-8 membered heterocyclyl; wherein the substitution refers to substitution with one or more R;
$R^{Y1}$ is selected from the group consisting of: H, C1-C4 alkyl; wherein the alkyl is optionally further substituted with one or more substituents selected from: halogen, hydroxyl, C1-C4 alkoxy, and cyano;
$R^{Y2}$ is -$L^2$-$R^{Y3}$; wherein $L^2$ is selected from: absent and C1-C6 alkylene; $R^{Y3}$ is selected from the group consisting of: hydrogen, C1-C6 alkyl, C1-C6 alkoxy, optionally substituted saturated or unsaturated 3-8 membered carbocycle, or optionally substituted saturated or unsaturated 4-12 membered heterocycle; wherein the substitution refers to substitution with one or more R;
alternatively $R^{Y1}$ and $R^{Y2}$ together with the nitrogen atom to which they are attached form an optionally substituted 4-10 membered nitrogen-containing heterocycle; the nitrogen-containing heterocycle contains at least one N atom and 0-2 heteroatoms selected from N, O, and S, wherein the substitution refers to replacement of one or more H in the group with R;
Each R is independently selected from the group consisting of: deuterium, halogen, -OH, oxo, mercapto, cyano, -$CD_3$, -C1-C6 alkyl, methylene, C2-C6 alkenyl, C2-C6 alkynyl, C3-C8 cycloalkyl, 6-10 membered aryl, 4-12 membered heterocyclyl, 5-10 membered heteroaryl, 6-10 membered aryl-C1-C6 alkylene-, 5-10 membered heteroaryl-C1-C6 alkylene-, C3-C8 cycloalkyl-C1-C6 alkylene-, 4-12 membered heterocyclyl-C1-C6 alkylene-, C1-C6 haloalkyl-, -OC1-C6 alkyl, -OC2-C6 alkenyl, C3-C8 cycloalkyl-O-, 4-12 membered heterocyclyl-O-, 6-10 membered aryl-O-, 5-10 membered heteroaryl-O-, -OC1-C6 alkylphenyl, -C1-C6 alkyl-OH, -C1-C6 alkyl-SH, -C1-C6 alkyl-O-C1-C6 alkyl, -OC1-C6 haloalkyl, -$NH_2$, -C1-C6 alkyl-$NH_2$, -N(C1-C6 alkyl)$_2$, -NH(C1-C6 alkyl), -N(C1-C6 alkyl)(C1-C6 alkylphenyl), -NH(C1-C6 alkylphenyl), - N(C1-C6 alkyl)(6-10 membered aryl), -NH(6-10 membered aryl), nitro, -C(O)-OH, -C(O)OC1-C6 alkyl, -$CONR^iR^{ii}$, -NHC(O)(C1-C6 alkyl), -NHC(O)(phenyl), -N(C1-C6 alkyl)C(O)(C1-C6 alkyl), -N(C1-C6 alkyl)C(O)(phenyl), -C(O)C1-C6 alkyl, 5-10 membered heteroaryl-C(O)-, - C(O)C1-C6 alkylphenyl, -C(O)C1-C6 haloalkyl, -OC(O)C1-C6 alkyl, -S(O)$_2$-C1-C6 alkyl, - S(O)-C1-C6 alkyl, -S(O)$_2$-phenyl, -S(O)$_2$-C1-C6 haloalkyl, -S(O)$_2$$NH_2$, -S(O)$_2$NH(C1-C6 alkyl), -S(O)$_2$NH(phenyl), -NHS(O)$_2$(C1-C6 alkyl), -NHS(O)$_2$(phenyl), and -NHS(O)$_2$(C1-C6 haloalkyl); wherein each hydrogen in the alkyl, alkenyl, alkynyl, cycloalkyl, phenyl, aryl, heterocyclyl, and heteroaryl is optionally further substituted with one or more substituents selected from: halogen, -OH, oxo (=O), -$NH_2$, C3-C8 cycloalkyl, 3-8 membered heterocyclyl, C1-C4 alkyl, C1-C4 haloalkyl-, -OC1-C4 alkyl, -C1-C4 alkyl-OH, -C1-C4 alkyl-O-C1-C4 alkyl, -OC1-C4 haloalkyl, cyano, nitro, -C(O)-OH, -C(O)OC1-C6 alkyl, -CON(C1-C6 alkyl)$_2$, - CONH(C1-C6 alkyl), -$CONH_2$, -NHC(O)(C1-C6 alkyl), -NH(C1-C6 alkyl)C(O)(C1-C6 alkyl), - $SO_2$(C1-C6 alkyl), -$SO_2$(phenyl), -$SO_2$(C1-C6 haloalkyl), -$SO_2NH_2$, -$SO_2$NH(C1-C6 alkyl), - $SO_2$NH(phenyl), -$NHSO_2$(C1-C6 alkyl), -$NHSO_2$(phenyl), and -$NHSO_2$(C1-C6 haloalkyl); $R^i$ and $R^{ii}$ are each independently H, deuterium, or C1-C6 alkyl.

2. The compound according to claim 1, **characterized in that**,

$R^1$ is selected from the group consisting of: -C(O)($NR^aR^b$) and 5-6 membered heteroaryl; wherein the heteroaryl is optionally substituted with $R^{1a}$;
$R^a$ and $R^b$ are each independently selected from the group consisting of: H, optionally substituted C1-C6 alkyl, optionally substituted 3-8 membered cycloalkyl;
Each $R^{1a}$ is independently selected from the group consisting of: H, cyano, halogen, C1-C3 alkyl, halo C1-C3 alkyl, C2-C4 alkenyl, C2-C4 alkynyl, C1-C3 alkoxy, and $C_3$-$C_5$ cycloalkyl.

3. The compound according to claim 1, **characterized in that** Ring B is selected from the groups shown below:

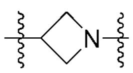 、 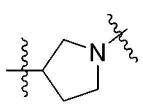 、 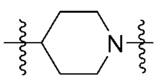 、 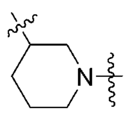 、 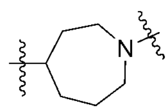 、 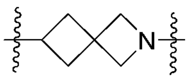 、

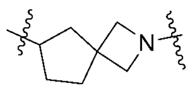、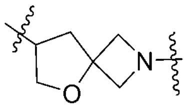、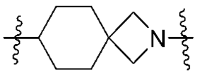、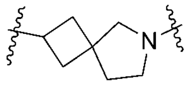、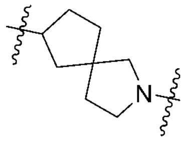、

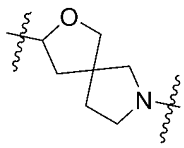、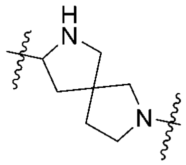、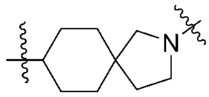、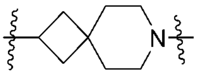、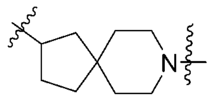、

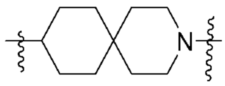、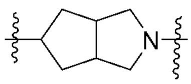、and 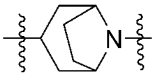;

wherein Ring B is optionally substituted with $R^B$; each $R^B$ is independently deuterium, halogen, oxo, C1-C3 alkyl, halo C1-C3 alkyl, or cyano.

**4.** The compound according to claim 1, **characterized in that** $X_1$ is N; $X_2$ is N or CH.

**5.** The compound according to claim 1, **characterized in that** Y is selected from: -C1-C6 alkylene-$NR^{Y1}R^{Y2}$, -3-6 membered cycloalkylene-$NR^{Y1}R^{Y2}$;

$R^{Y1}$ is selected from the group consisting of: H, C1-C4 alkyl; wherein the alkyl is optionally further substituted with one or more substituents selected from: halogen, hydroxyl, C1-C4 alkoxy, and cyano;
$R^{Y2}$ is $-L^2-R^{Y3}$; wherein $L^2$ is selected from: absent and C1-C6 alkylene; $R^{Y3}$ is selected from the group consisting of: hydrogen, C1-C6 alkyl, C1-C6 alkoxy, optionally substituted saturated or unsaturated 3-6 membered carbocycle, or optionally substituted saturated or unsaturated 4-6 membered heterocycle; wherein the substitution refers to substitution with one or more R;
or $R^{Y1}$ and $R^{Y2}$ together with the nitrogen atom to which they are attached form an optionally substituted 4-9 membered nitrogen-containing heterocycle; the nitrogen-containing heterocycle contains at least one N atom and 0-2 heteroatoms selected from N and O, wherein the substitution refers to replacement of one or more H in the group with R;
preferably, Y is selected from: -C1-C4 alkylene-$NR^{Y1}R^{Y2}$, -3-6 membered cycloalkylene-$NR^{Y1}R^{Y2}$.
$R^{Y1}$ is selected from the group consisting of: H, C1-C4 alkyl; wherein the alkyl is optionally further substituted with one or more substituents selected from: halogen, hydroxyl, C1-C4 alkoxy, and cyano;
$R^{Y2}$ is $-L^2-R^{Y3}$; wherein $L^2$ is selected from: absent and C1-C4 alkylene; $R^{Y3}$ is selected from the group consisting of: hydrogen, C1-C4 alkyl, C1-C4 alkoxy;
or $R^{Y1}$ and $R^{Y2}$ together with the nitrogen atom to which they are attached form an optionally substituted 4-10 membered nitrogen-containing heterocycle; the nitrogen-containing heterocycle contains at least one N atom and 0-2 heteroatoms selected from N and O, wherein the substitution refers to replacement of one or more H in the group with R, and R is as defined in claim 1.

**6.** The compound according to claim 1, **characterized in that**, $L^1$ is absent; $R^5$ is

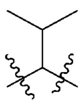;

Y is

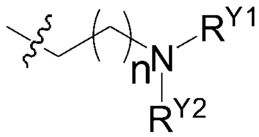 or 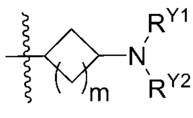 ;

wherein n is selected from: 0, 1, 2; m is selected from 1, 2;

wherein $R^{Y1}$ is selected from the group consisting of: H, C1-C4 alkyl; wherein the alkyl is optionally further substituted with one or more substituents selected from: halogen, hydroxyl, C1-C4 alkoxy, and cyano;

$R^{Y2}$ is $-L^2-R^{Y3}$; wherein $L^2$ is selected from: absent and C1-C4 alkylene; $R^{Y3}$ is selected from the group consisting of: hydrogen, C1-C4 alkyl, C1-C4 alkoxy;

or when $R^{Y1}$ and $R^{Y2}$ together with the nitrogen atom to which they are attached form an optionally substituted 4-10 membered nitrogen-containing heterocycle, the 4-10 membered nitrogen-containing heterocycle is selected from the group consisting of:

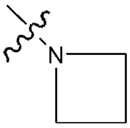 、 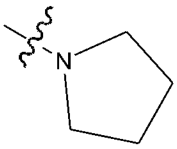 、 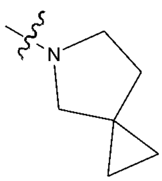 、 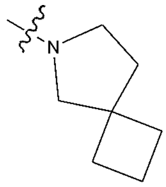 、 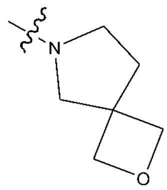 、 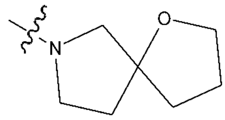 、

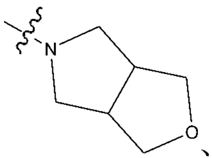 、 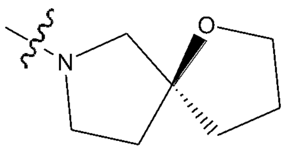 、 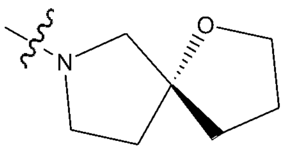 、 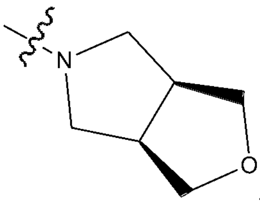 ;

wherein the substitution refers to replacement of one or more H in the group with R, and R is as defined in claim 1.

**7.** The compound according to claim 1, **characterized in that** the compound is selected from Table A:

Table A

| | | |
|---|---|---|
| 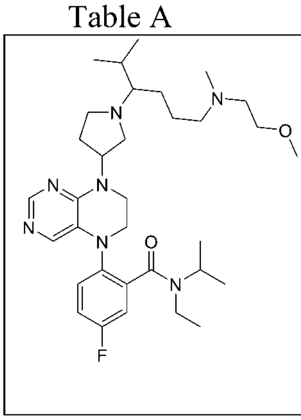 、 | 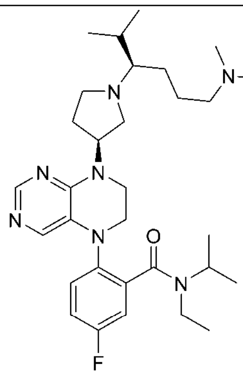 、 | 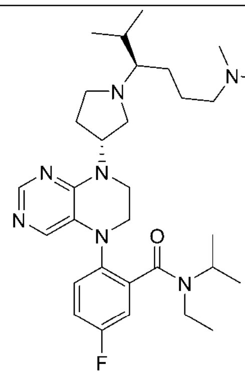 、 |

| | | |
|---|---|---|
| 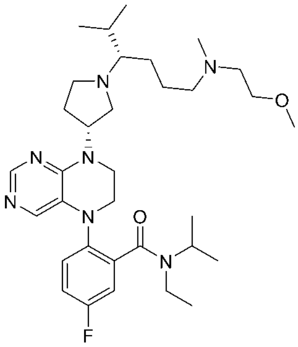 、 | 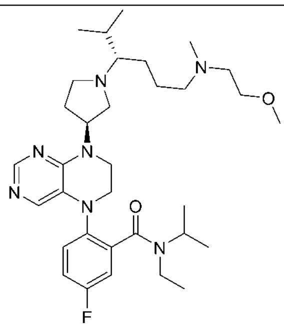 、 | 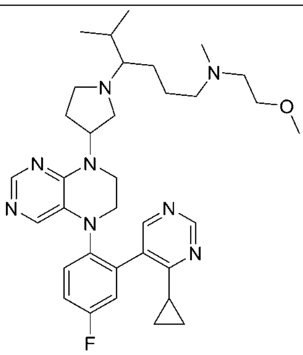 、 |
| 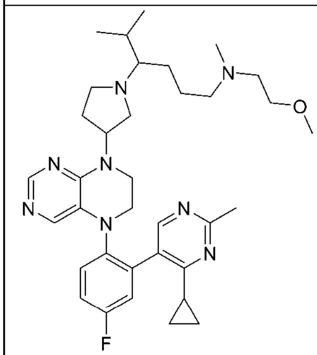 、 | 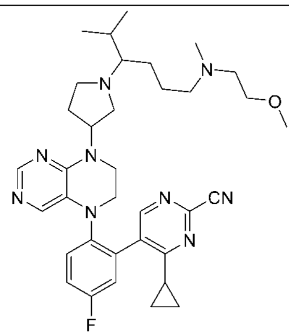 、 | 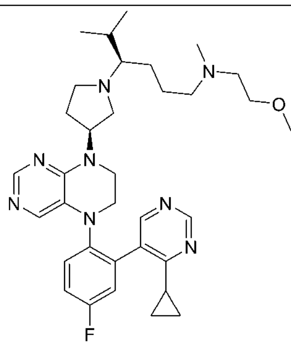 、 |
| 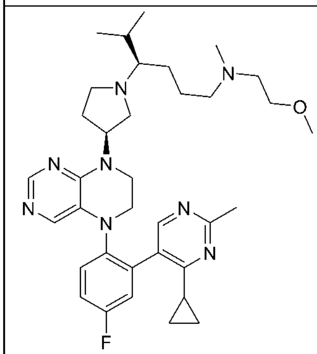 、 | 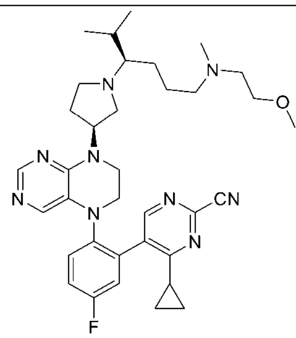 、 | 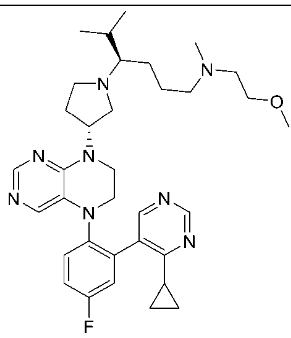 、 |
| 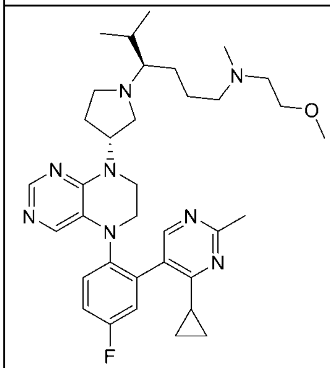 、 | 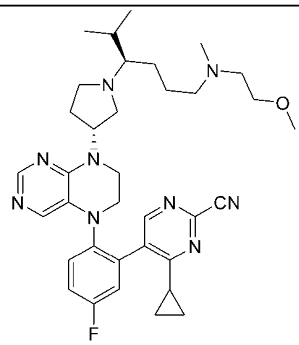 、 | 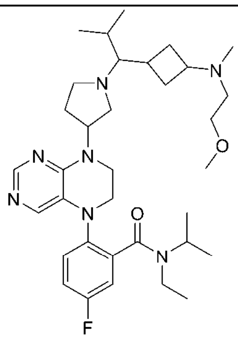 、 |
| 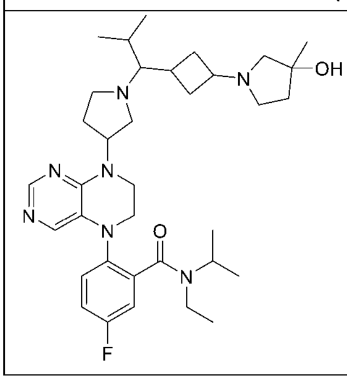 、 | 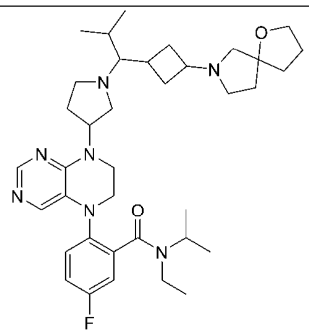 、 | 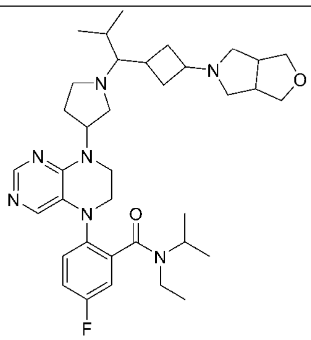 、 |

| | | |
|---|---|---|
| 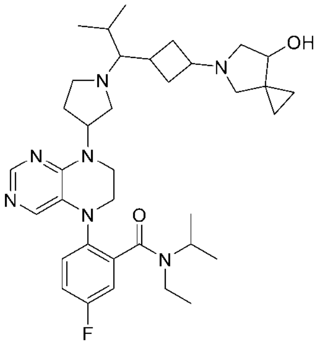 , | 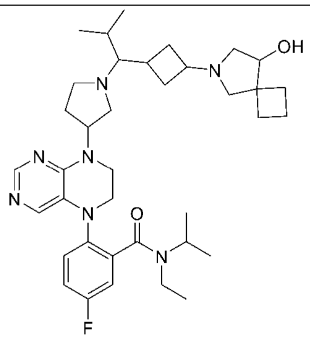 , | 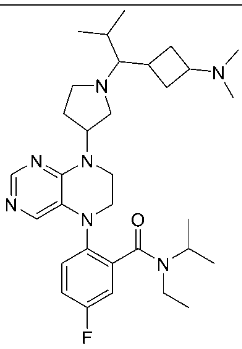 , |
| 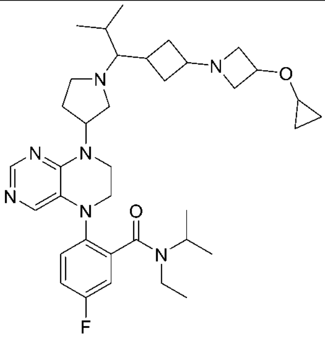 , | 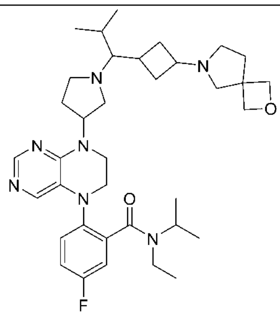 , | 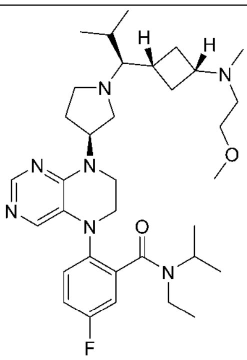 , |
| 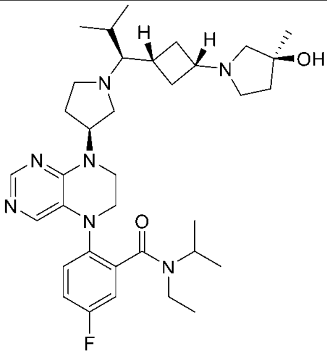 , | 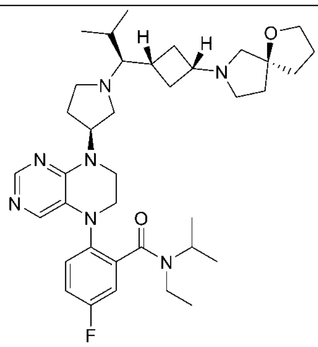 , | 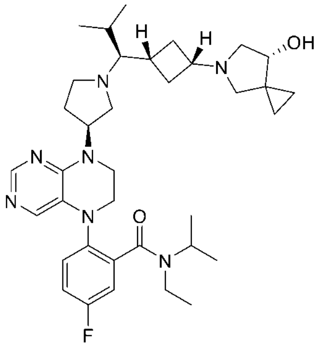 , |
| 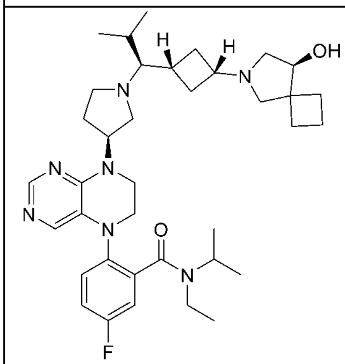 , | 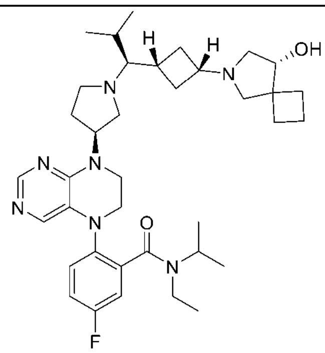 , | 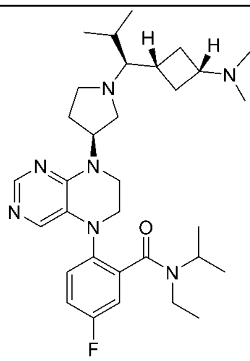 , |
| 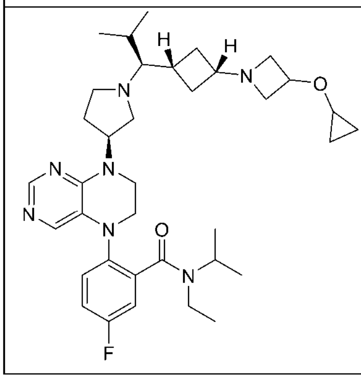 , | 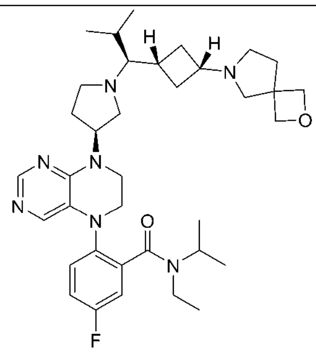 , | 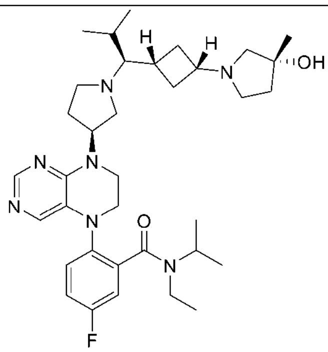 , |

| | | |
|---|---|---|
| 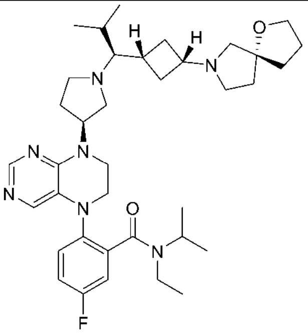 、 | 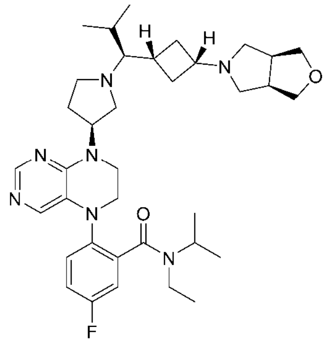 、 | 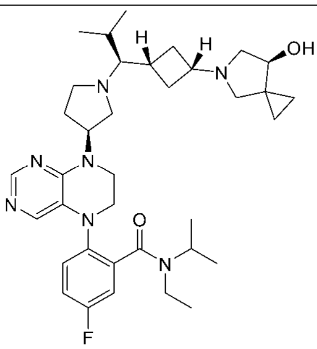 、 |
| 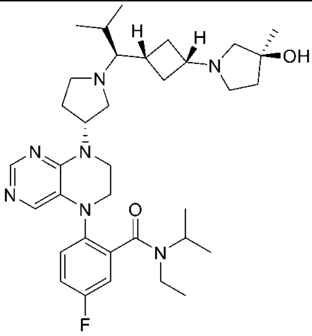 、 | 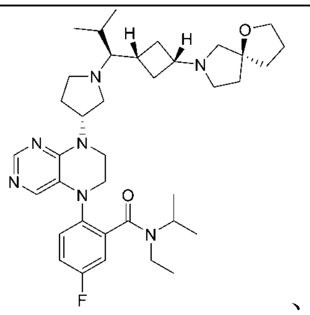 、 | 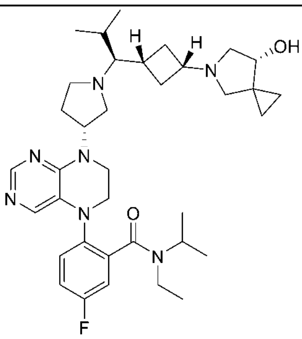 、 |
| 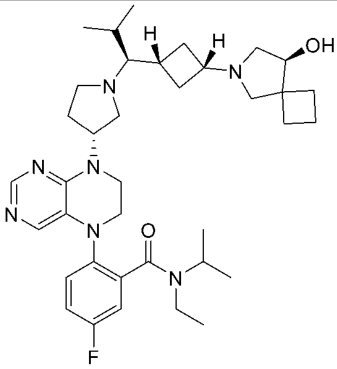 、 | 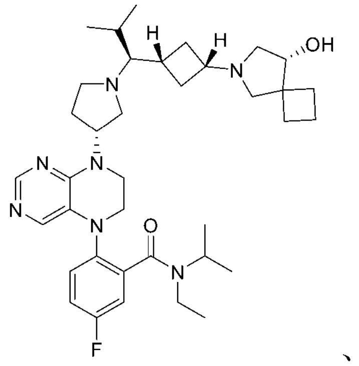 、 | 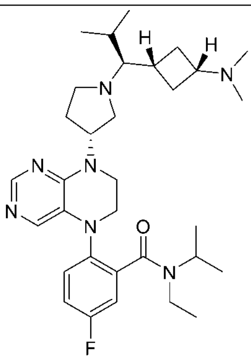 、 |
| 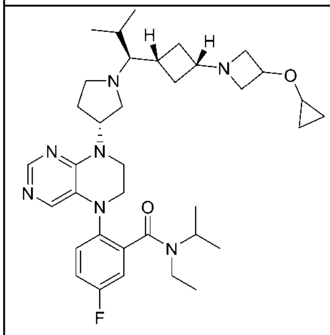 、 | 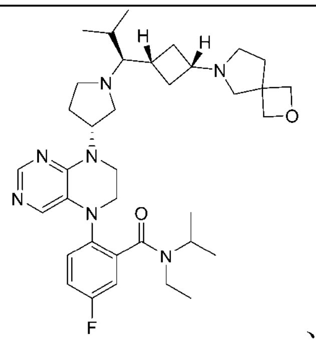 、 | 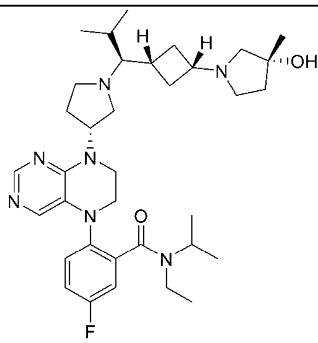 、 |
| 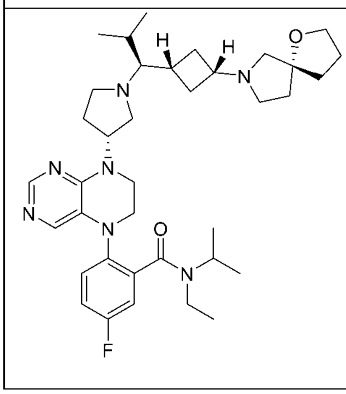 、 | 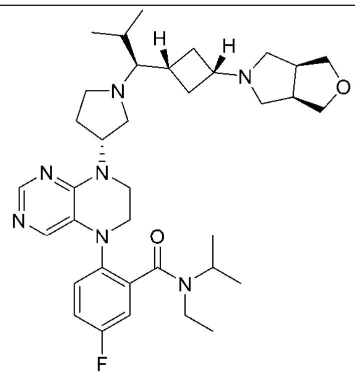 、 | 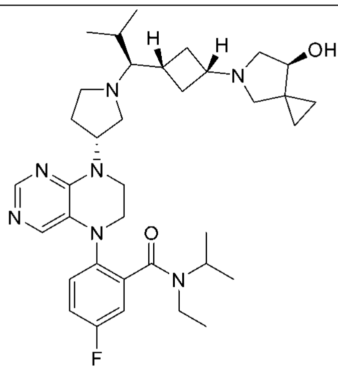 、 |

| | | |
|---|---|---|
| 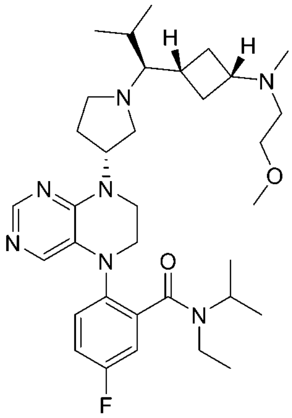 , | 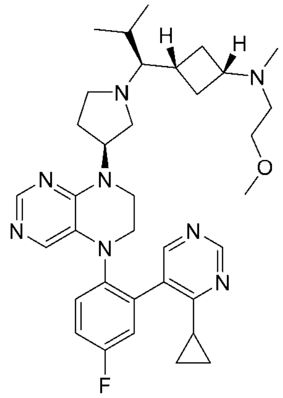 , | 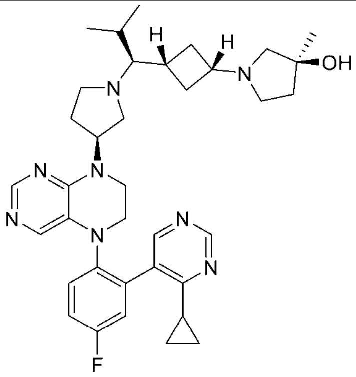 , |
| 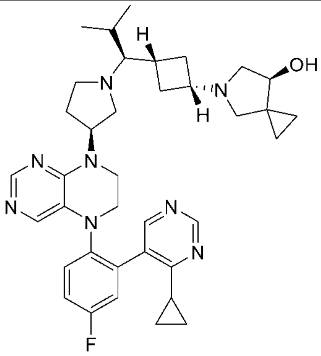 , | 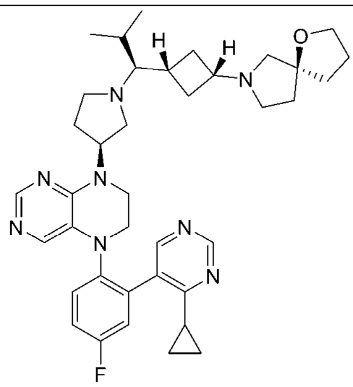 , | 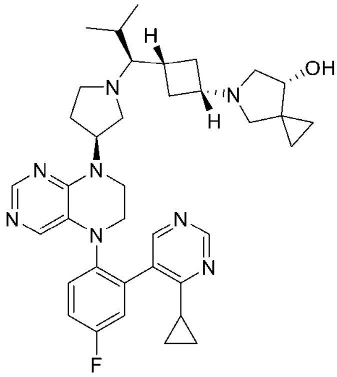 , |
| 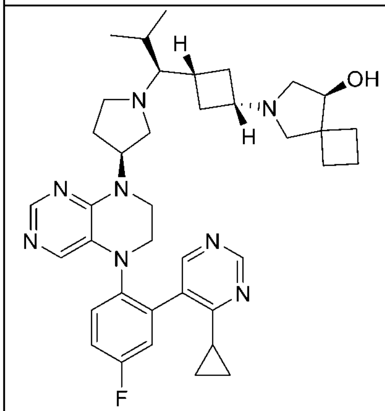 , | 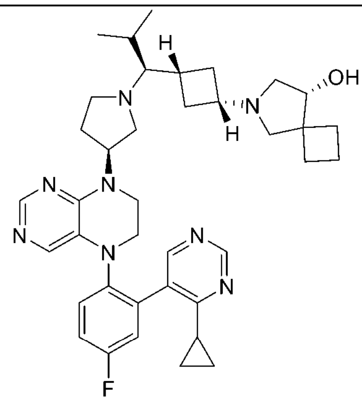 , | 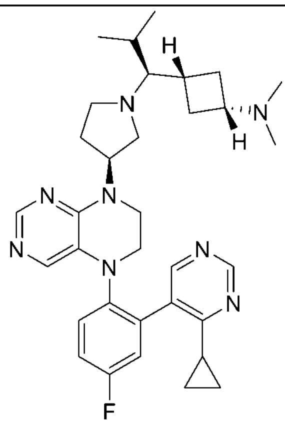 , |
| 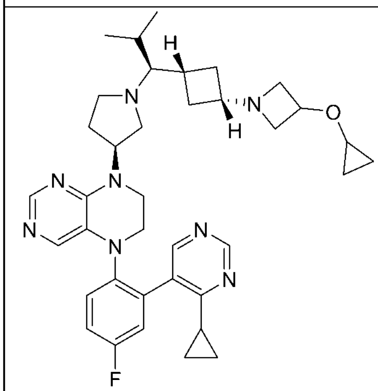 , | 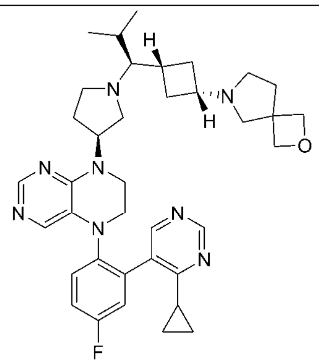 , | 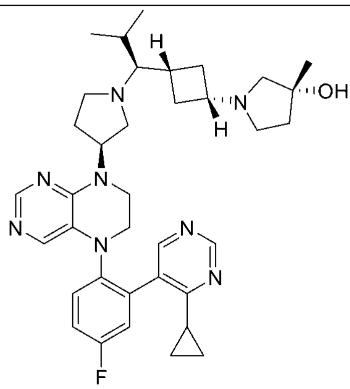 , |

| | | |
|---|---|---|
| 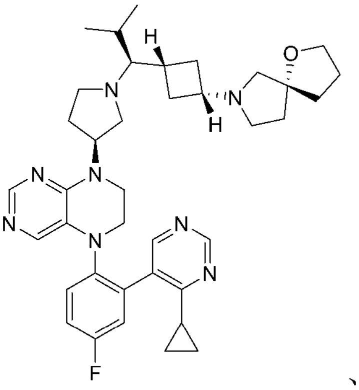 、 | 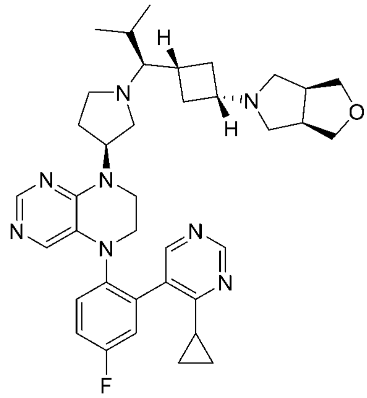 、 | 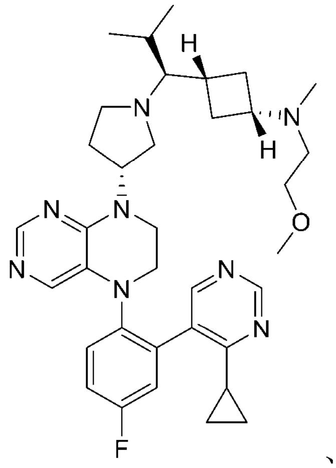 、 |
| 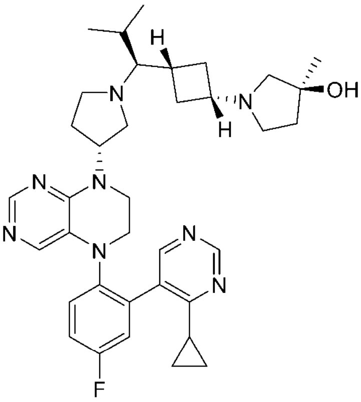 、 | 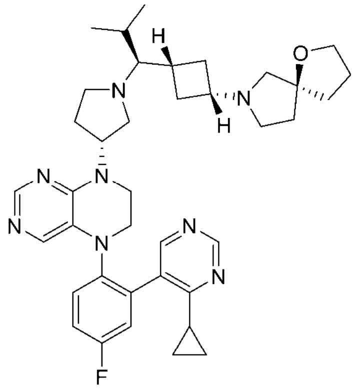 、 | 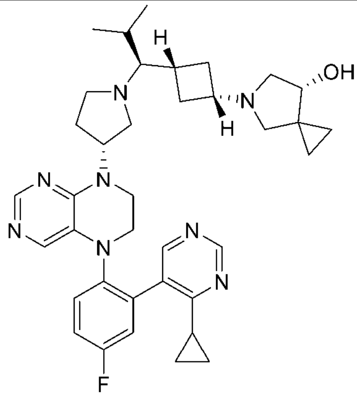 、 |
| 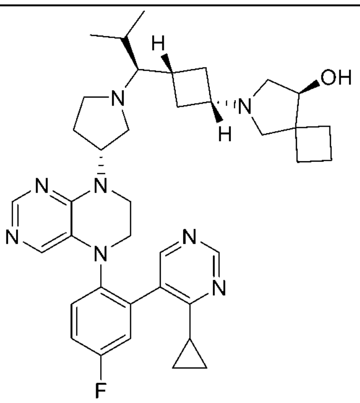 、 | 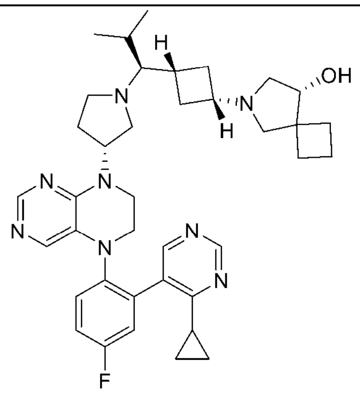 、 | 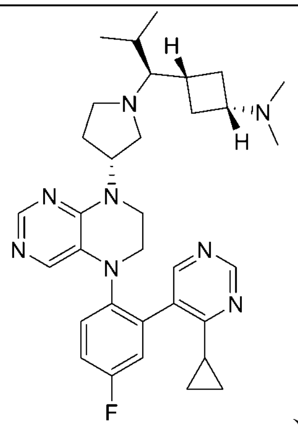 、 |
| 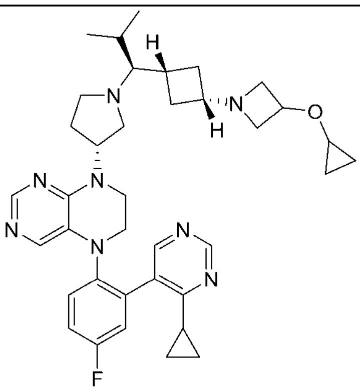 、 | 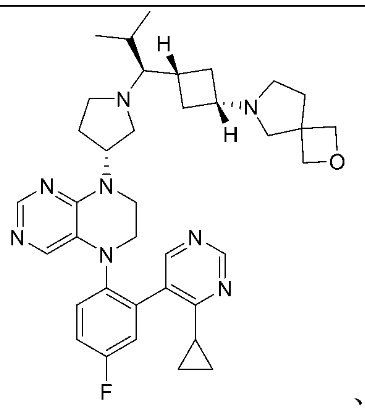 、 | 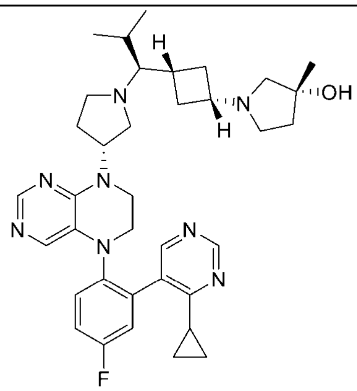 、 |

| | | |
|---|---|---|
| 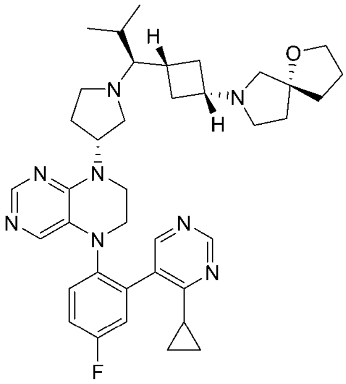 , | 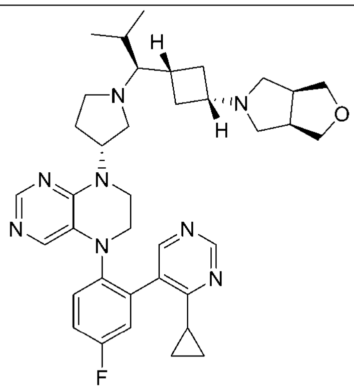 , | 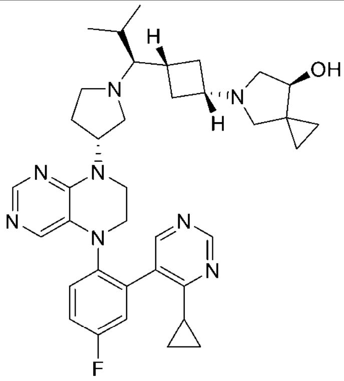 , |
| 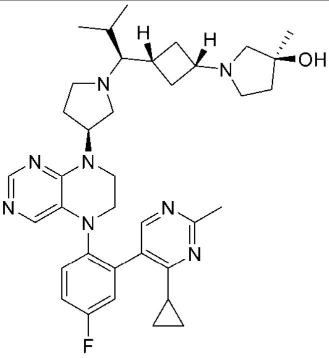 , | 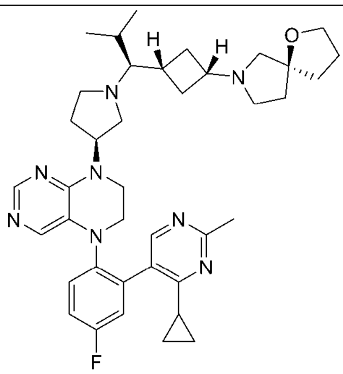 , | 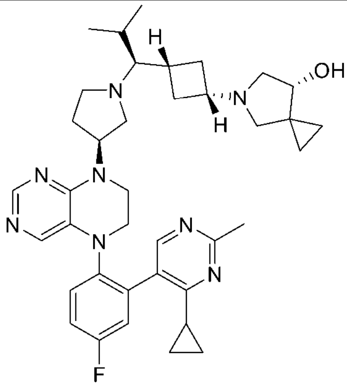 , |
| 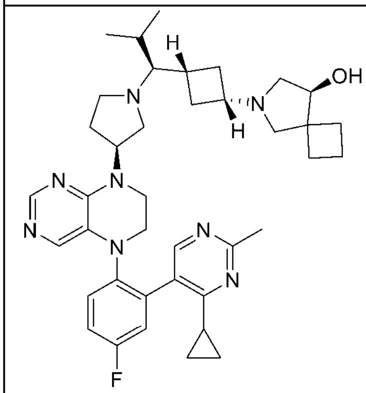 , | 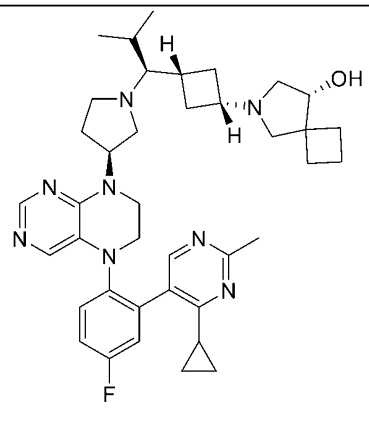 , | 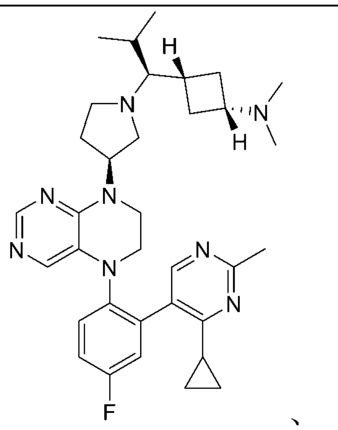 , |
| 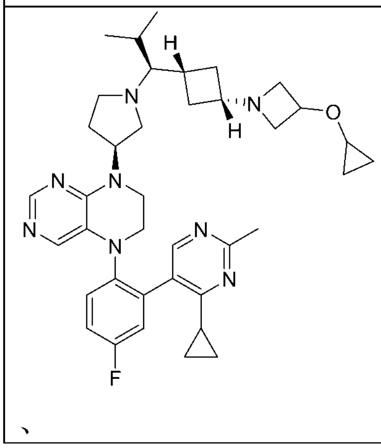 , | 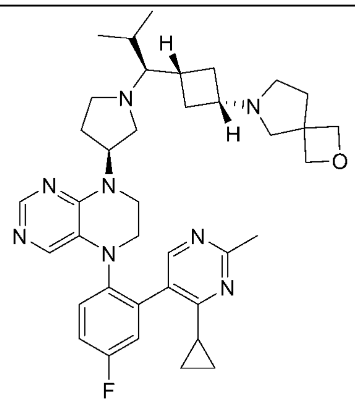 , | 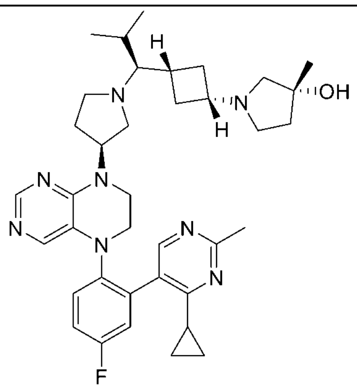 , |

| | | |
|---|---|---|
| 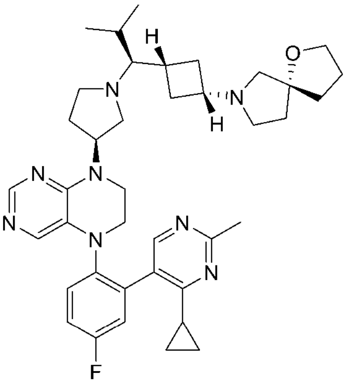 , | 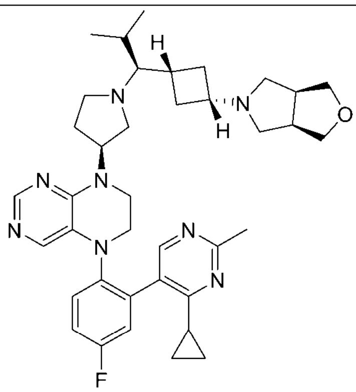 , | 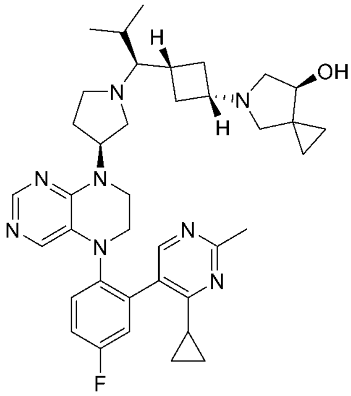 , |
| 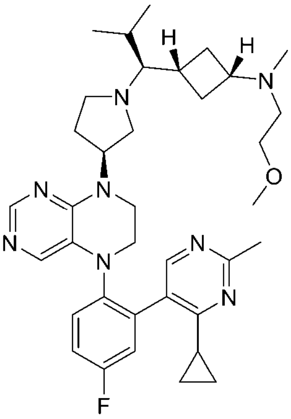 , | 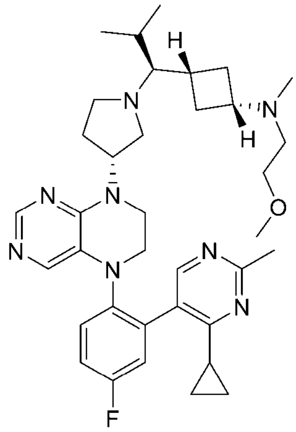 , | 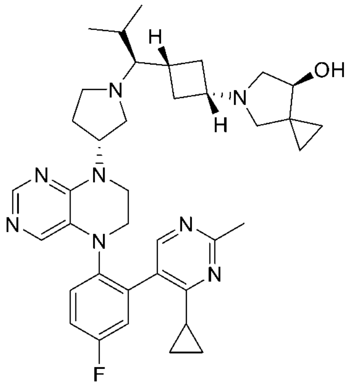 , |
| 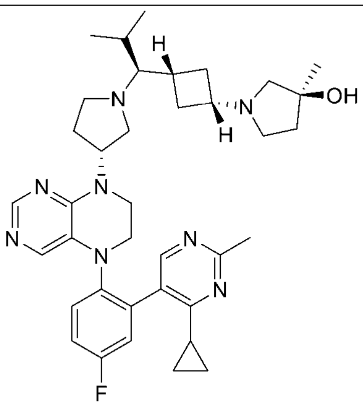 , | 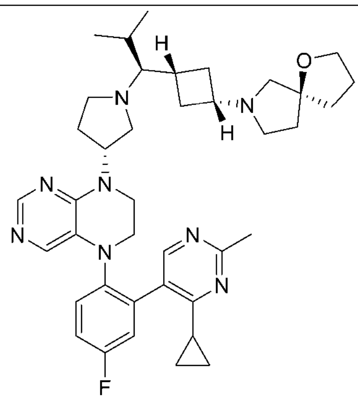 , | 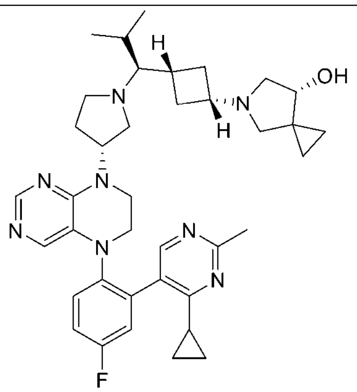 , |
| 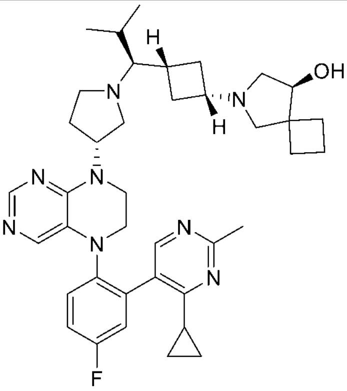 , | 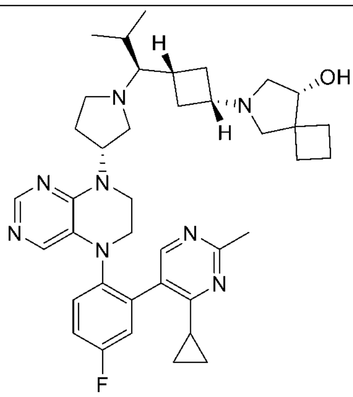 , | 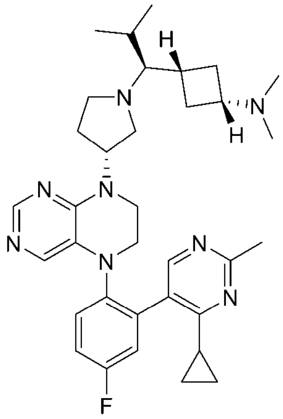 , |

| | | |
|---|---|---|
| 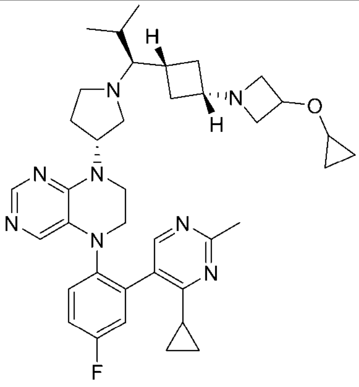 , | 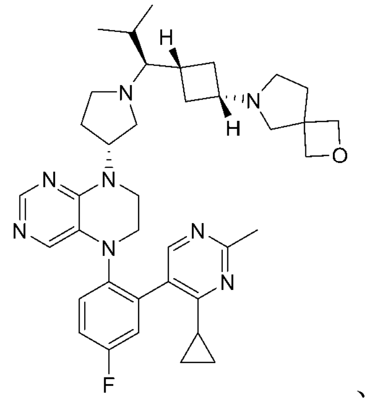 , | 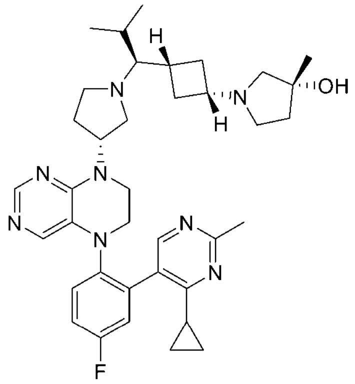 , |
| 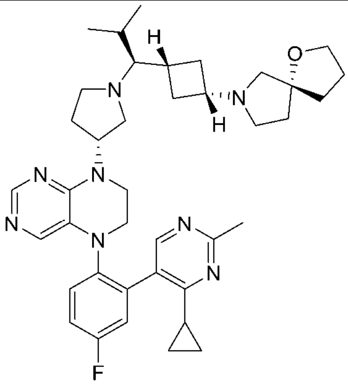 , | 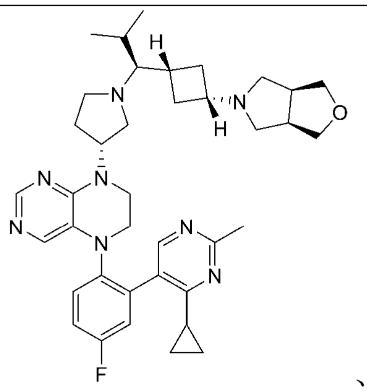 , | 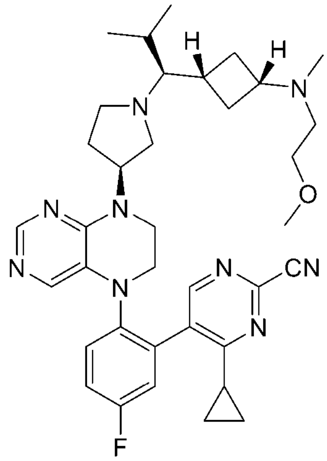 , |
| 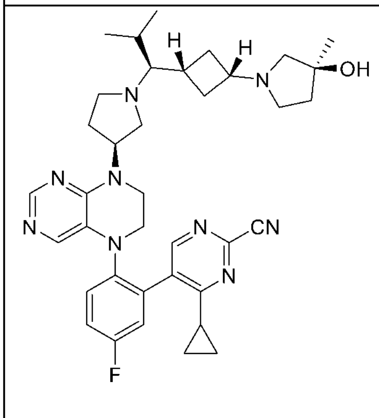 , | 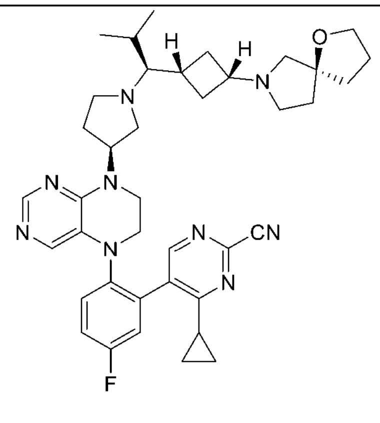 , | 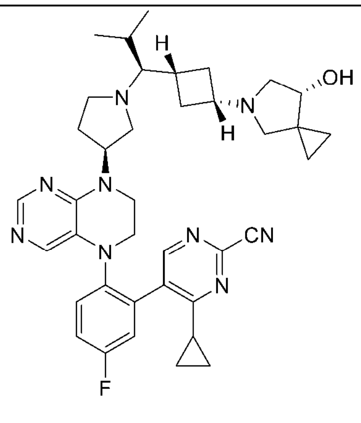 , |
| 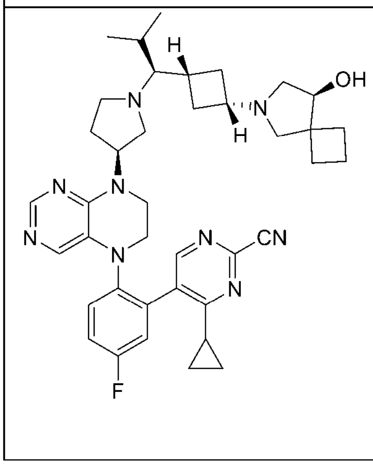 , | 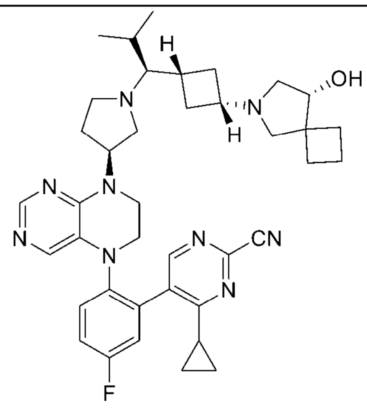 , | 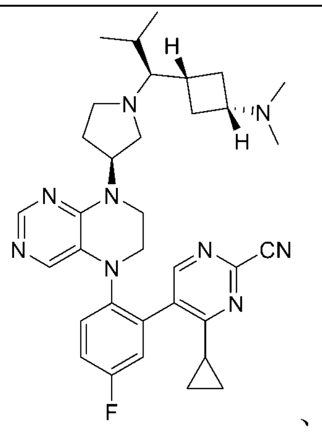 , |

| | | |
|---|---|---|
| 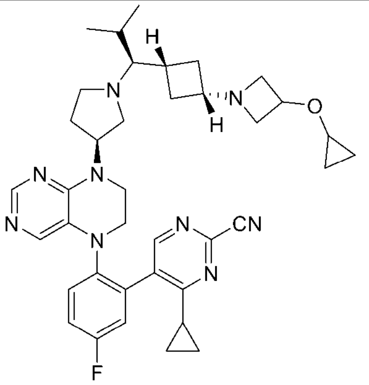 , | 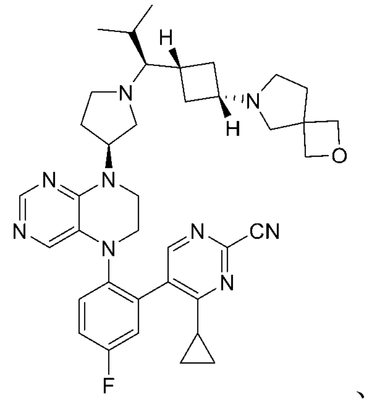 , | 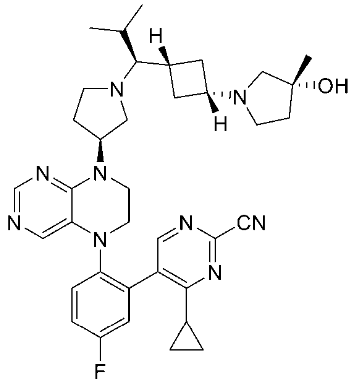 , |
| 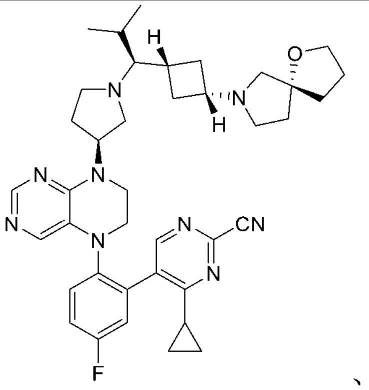 , | 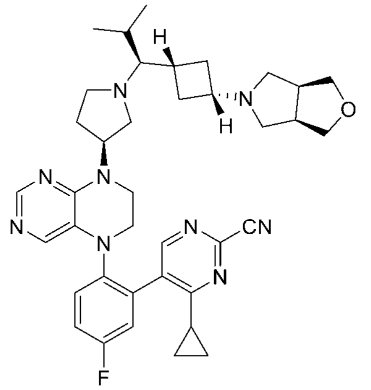 , | 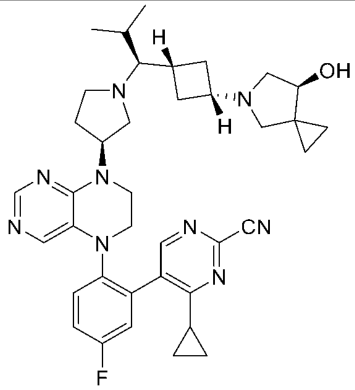 , |
| 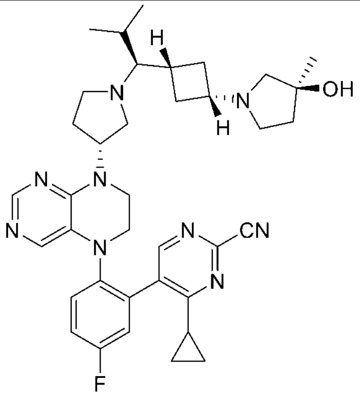 , | 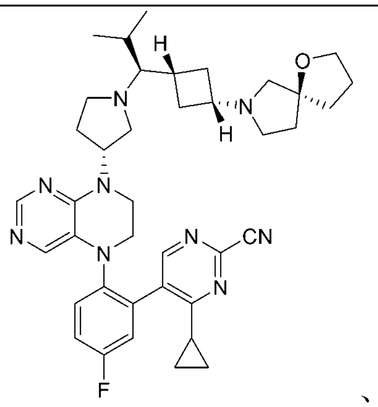 , | 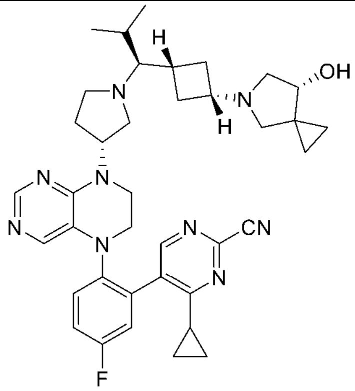 , |
| 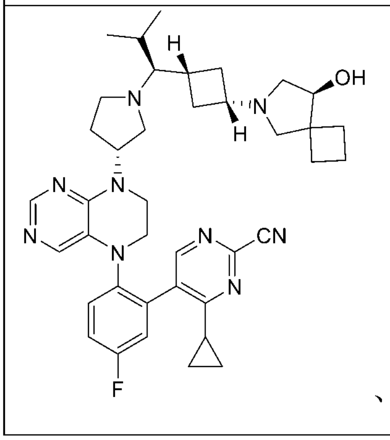 , | 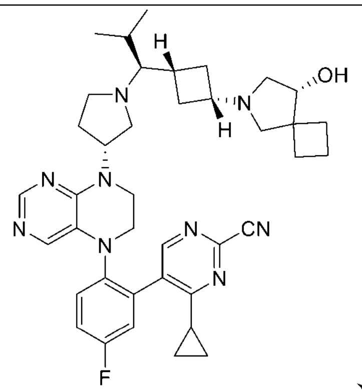 , | 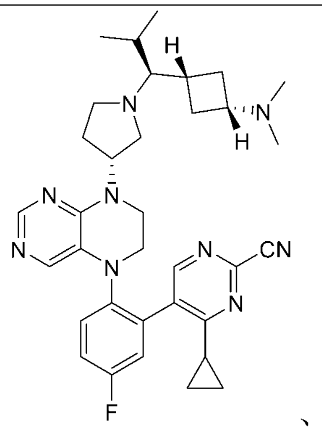 , |

| | | |
|---|---|---|
| 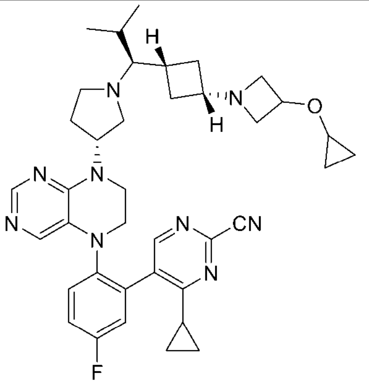 , | 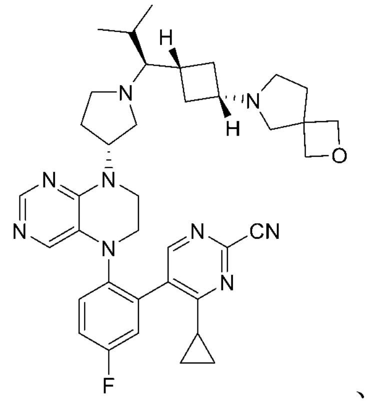 , | 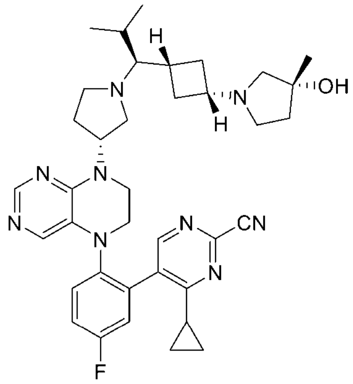 , |
| 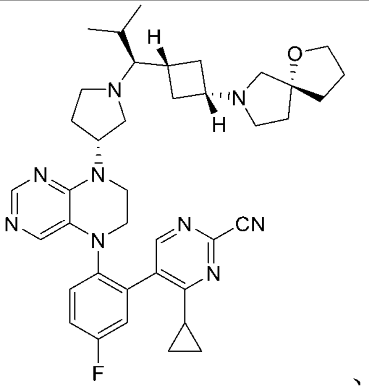 , | 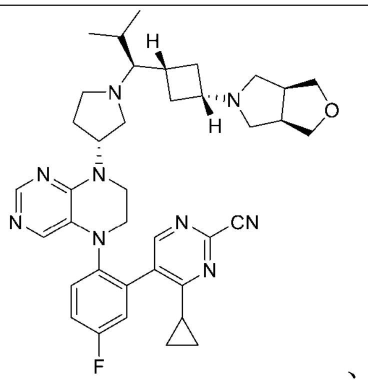 , | 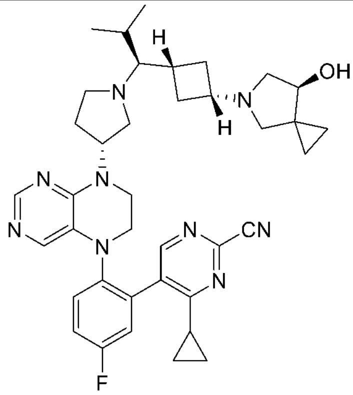 , |
| 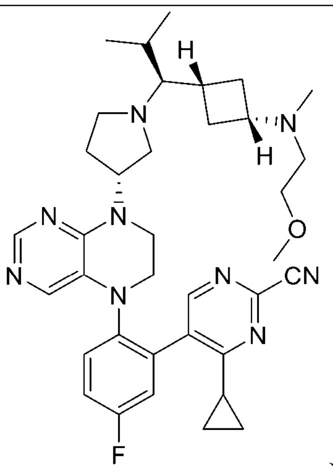 , | 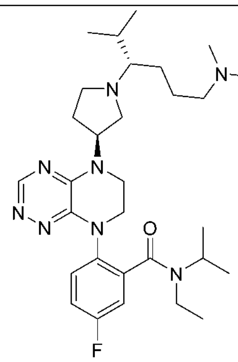 , | 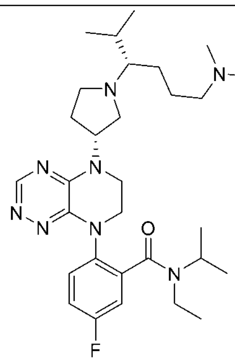 , |
| 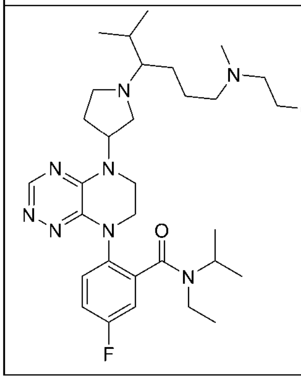 , | 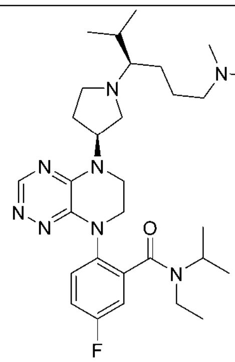 , | 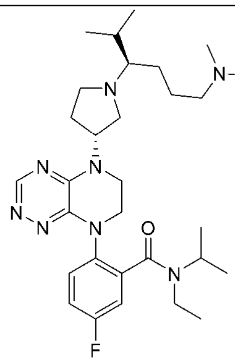 , |

| | | |
|---|---|---|
| 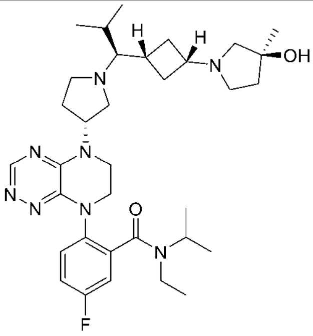 、 | 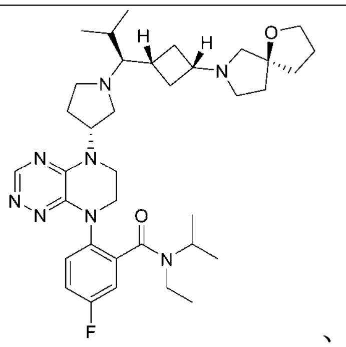 、 | 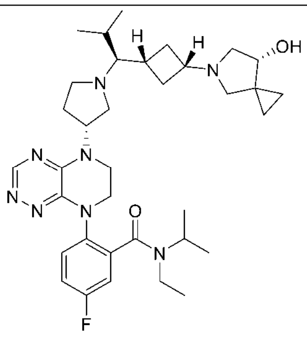 、 |
| 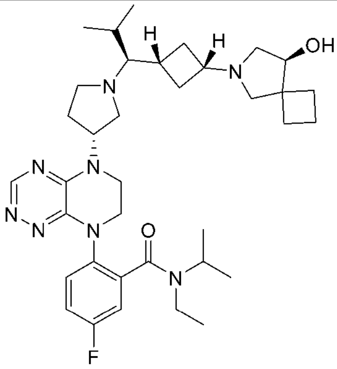 、 | 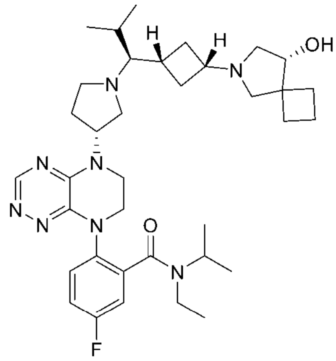 、 | 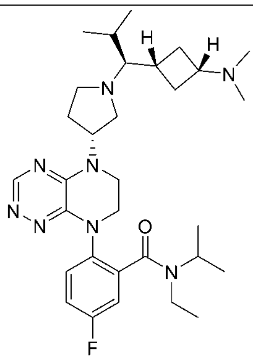 、 |
| 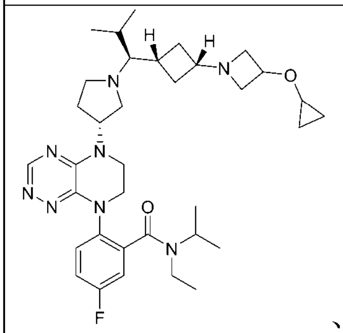 、 | 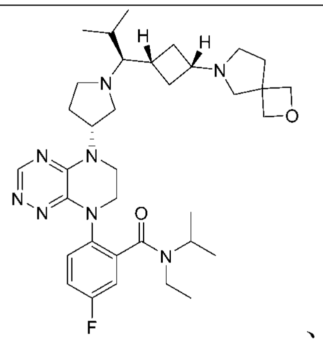 、 | 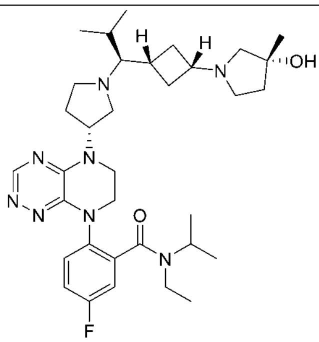 、 |
| 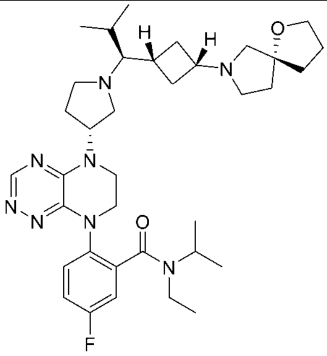 、 | 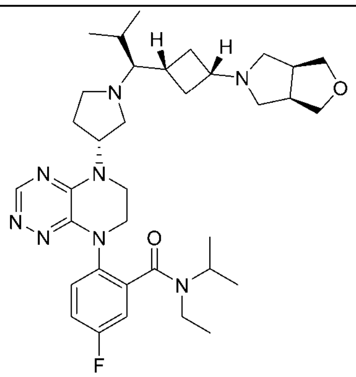 、 | 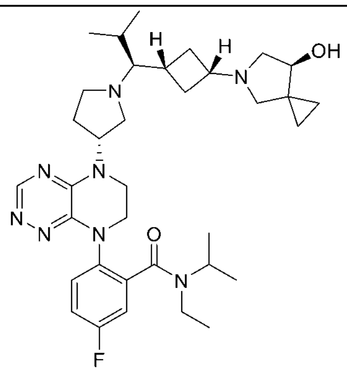 、 |

| | | |
|---|---|---|
| 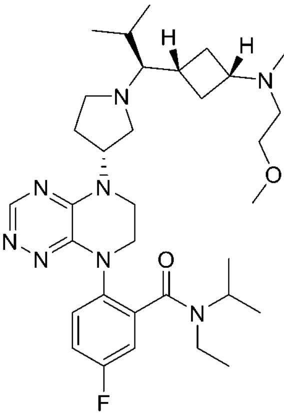 | | |

.

**8.** A pharmaceutical composition, **characterized in that** the pharmaceutical composition comprises one or more compounds according to any one of claims 1-7 in a safe and effective amount, and a pharmaceutically acceptable carrier.

**9.** Use of a compound according to any one of claims 1-7 or a pharmaceutical composition according to claim 8, **characterized in that** the use is selected from any one or more of the following (a)-(c):

(a) preparation of a medicament for preventing or treating diseases associated with MLL1, MLL2, MLL fusion proteins, and/or menin protein activity;
(b) preparation of an inhibitor for in vitro non-therapeutic inhibition of activity associated with MLL1, MLL2, MLL fusion proteins, and/or menin protein;
(c) preparation of an inhibitor for in vitro non-therapeutic inhibition of tumor cell proliferation.

**10.** The use according to claim 9, **characterized in that** the diseases associated with MLL1, MLL2, MLL fusion proteins, and/or menin protein activity are selected from the group consisting of: tumors, diabetes, and other diseases associated with MLL1, MLL2, MLL fusion proteins, and/or menin protein activity; wherein the tumors are selected from the group consisting of: leukemia, Ewing's sarcoma, breast cancer, prostate cancer, T-cell lymphoma, B-cell lymphoma, malignant rhabdoid tumor, synovial sarcoma, colorectal cancer, endometrial carcinoma, gastric cancer, hepatocellular carcinoma, renal cell carcinoma, lung cancer, melanoma, ovarian cancer, pancreatic cancer, glioblastoma, cholangiocarcinoma, nasopharyngeal carcinoma, cervical cancer, head and neck cancer, esophageal cancer, thyroid cancer, and bladder cancer; and the other diseases are selected from the group consisting of: autoimmune diseases and nonalcoholic steatohepatitis.

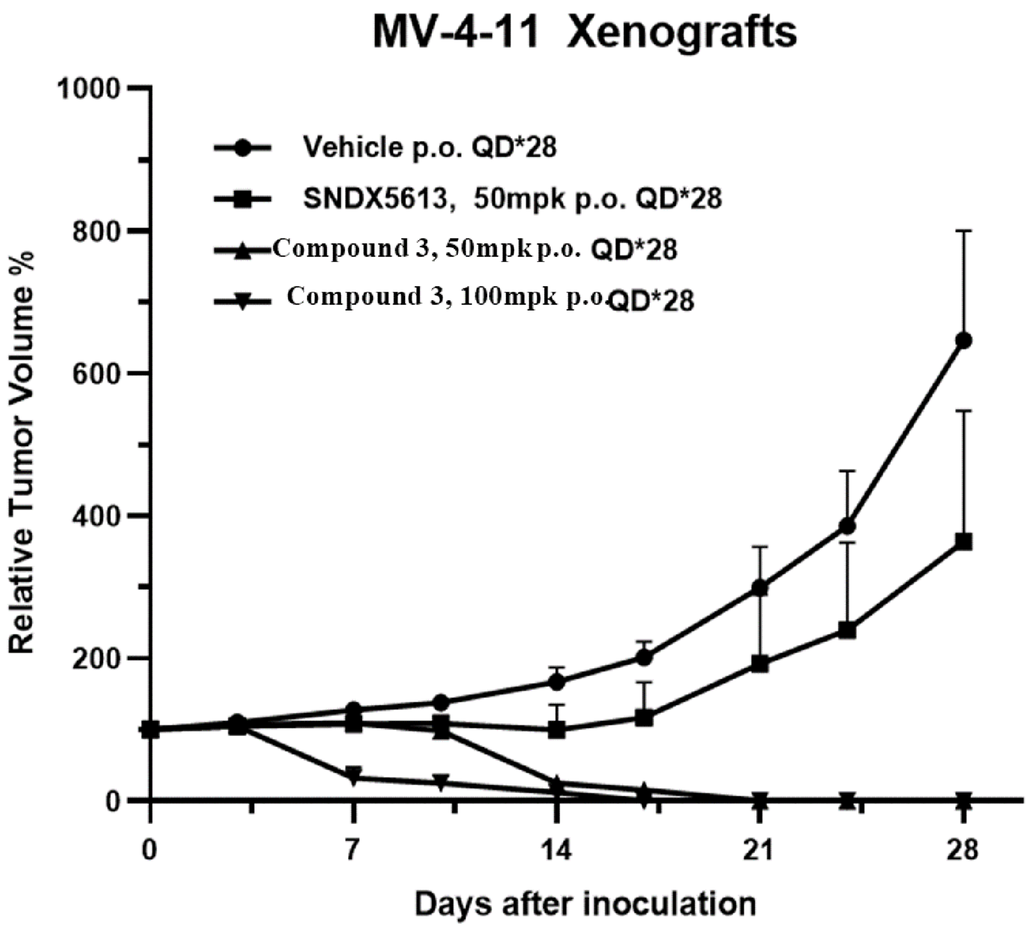
MV-4-11 Xenografts
Relative Tumor Volume %
1000
800
600
400
200
0
Vehicle p.o. QD*28
SNDX5613, 50mpk p.o. QD*28
Compound 3, 50mpk p.o. QD*28
Compound 3, 100mpk p.o. QD*28
0
7
14
21
28
Days after inoculation

Figure 1

## INTERNATIONAL SEARCH REPORT

International application No.
**PCT/CN2024/106034**

**A. CLASSIFICATION OF SUBJECT MATTER**

C07D471/04(2006.01)i; C07D 475/00(2006.01)i; A61K31/551(2006.01)i; A61K31/519(2006.01)i; A61K31/5377(2006.01)i; A61K45/06(2006.01)i; A61P35/00(2006.01)i; A61P3/10(2006.01)i; A61P35/02(2006.01)i; A61P37/06(2006.01)i; A61P1/16(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXTC, VCN, STN: 中山优理生物, 氮杂, 哌啶, 吡啶, 稠和, 抑制剂, 癌, 肿瘤, Inhibitor, piperidine, pyridine, cancer, tumor, menin, MLL, 结构检索

**C. DOCUMENTS CONSIDERED TO BE RELEVANT**

| Category* | Citation of document, with indication, where appropriate, of the relevant passages | Relevant to claim No. |
|---|---|---|
| PX | CN 116903609 A (SHANGHAI YOULI HUISHENG PHARMACEUTICAL CO., LTD.) 20 October 2023 (2023-10-20)<br>claims 1-10 | 1-10 |
| X | WO 2023078426 A1 (SHANGHAI YOULI HUISHENG PHARMACEUTICAL CO., LTD.) 11 May 2023 (2023-05-11)<br>description, pages 1-8, 18, and 32-74 | 1-10 |
| A | WO 2023239227 A1 (SAPIENSBIO INC.) 14 December 2023 (2023-12-14)<br>description, pages 1-5 | 1-10 |
| A | CN 101652365 A (ASTRAZENECA AB) 17 February 2010 (2010-02-17)<br>description, pages 1-4 | 1-10 |
| A | US 2014315911 A1 (OSI PHARMACEUTICALS, LLC.) 23 October 2014 (2014-10-23)<br>description, page 1 | 1-10 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 September 2024** | **24 September 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/106034**

| Patent document cited in search report | Publication date (day/month/year) | Patent family member(s) | Publication date (day/month/year) |
|---|---|---|---|
| CN 116903609 A | 20 October 2023 | None | |
| WO 2023078426 A1 | 11 May 2023 | TW 202319386 A | 16 May 2023 |
| WO 2023239227 A1 | 14 December 2023 | None | |
| CN 101652365 A | 17 February 2010 | EP 2109612 A2 | 21 October 2009 |
| | | EP 2109612 B1 | 10 November 2010 |
| | | DE 602008003404 D1 | 23 December 2010 |
| | | RU 2009132605 A | 10 March 2011 |
| | | ATE 487720 T1 | 15 November 2010 |
| | | CA 2675677 A1 | 07 August 2008 |
| | | JP 2010517989 A | 27 May 2010 |
| | | KR 20090104920 A | 06 October 2009 |
| | | US 2008194572 A1 | 14 August 2008 |
| | | US 7799781 B2 | 21 September 2010 |
| | | BRPI 0807868 A2 | 15 October 2013 |
| | | MX 2009008084 A | 12 October 2009 |
| | | AU 2008211729 A1 | 07 August 2008 |
| | | WO 2008093075 A2 | 07 August 2008 |
| | | WO 2008093075 A3 | 02 October 2008 |
| US 2014315911 A1 | 23 October 2014 | US 9351974 B2 | 31 May 2016 |
| | | WO 2013071217 A1 | 16 May 2013 |
| | | JP 2015501793 A | 19 January 2015 |
| | | EP 2776444 A1 | 17 September 2014 |
| | | EP 2776444 A4 | 22 July 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023078426 A


### Non-patent literature cited in the description


- **PERNER et al.** *Nature*, March 2023
- **CAREY** ; **SUNDBERG**. ADVANCED ORGANIC CHEMISTRY. Plenum Press, 2000, vol. A
- **B**. ADVANCED ORGANIC CHEMISTRY. Plenum Press, 2001
- Chiral Separations, Methods and Protocols. Methods in Molecular Biology. 2004, vol. 243
- **A.M. STALCUP**. Chiral Separations. *Annu. Rev. Anal. Chem.*, 2010, vol. 3, 341-63
- VOGEL'S ENCYCLOPEDIA OF PRACTICAL ORGANIC CHEMISTRY. Longman Scientific and Technical Ltd., 1991, 809-816
- **HELLER**. *Acc. Chem. Res.*, 1990, vol. 23, 128
- **GOODMAN** ; **GILMAN**. The Pharmacological Basis of Therapeutics. Pergamon
- Remington's, Pharmaceutical Sciences. Mack Publishing Co.
- **SAMBROOK et al.** Molecular Cloning: A Laboratory Manual. Cold Spring Harbor Laboratory Press, 1989